# EUROPEAN PATENT APPLICATION

(11) **EP 2 573 080 A1**
(43) Date of publication of application: **27.03.2013**
(21) Application number: 12198010.6
(22) Date of filing: 26.09.2008
(51) Int. Cl.: C07D 401/04, C07D 403/04, C07D 405/04, C07D 409/04, A61K 31/4035, A61P 21/00

(54) **Isoindoline compounds for the treatment of spinal muscular atrophy and other uses**

(30) Priority: 27.09.2007 US 975675 P
(62) Divisional of application: 08833236.6
(71) Applicant: The United States of America, as Represented by the Secretary, Department of Health and Human Services, Bethesda, Maryland 20892-7660 (US); Albany Molecular Research, INC., Albany, New York 12212 (US)
(72) Inventor: Heemskerk, Jill E., Bethesda, MD Maryland 20814 (US); McCall, John M., Boca Grande, FL Florida 33921 (US); Barnes, Keith D., Rexford, NY New York 12148 (US)
(74) Representative: Pfenning, Meinig & Partner GbR

(57) **Abstract**

Disclosed is a compound of Formula I 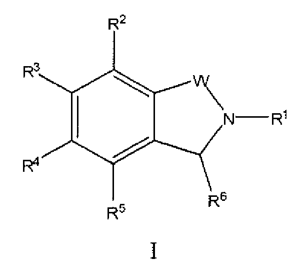 in which W and R¹-R⁶ are defined herein. Also disclosed is a method of treating spinal muscular atrophy, as well as methods of using such compounds to increase SMN expression, increase EAAT2 expression, or increase the expression of a nucleic acid that encodes a translational stop codon introduced directly or indirectly by mutation or frameshift.

## Description

### CROSS-REFERENCE TO RELATED APPLICATIONS

This patent application claims the benefit of U.S. Provisional Patent Application No. 60/975,675, filed September 27, 2007, which is incorporated by reference.

### BACKGROUND OF THE INVENTION

Spinal Muscular Atrophy (SMA) is a paralyzing and often fatal disease of infants and children. To date there is no effective treatment for SMA. This disease is caused by mutations that reduce the level of survival motor neuron protein (SMN), resulting in the loss of motor neurons in the central nervous system. Drugs that increase SMN expression are expected to be useful in the prevention and treatment of SMA. Prior studies in cultured cells have shown that indoprofen, a previously marketed non-steroidal anti-inflammatory drug (NSAID), increases the level of expression of SMN protein via an unknown mechanism (Lunn et al., Chem. & Biol., 11: 1489-1493(2004)). Indoprofen also was shown to increase survival of SMA model mouse fetuses when administered in utero. The mechanism of regulation of SMN expression is not thought to be related to indoprofen's NSAID activity since not all of the NSAIDs tested increase SMN expression. One possible mechanism of action is increased protein translation, as it has been shown that the level of the SMN protein can be improved by drugs that cause translational read-through of nonsense stop codons (Wolstencroft et al., Hum. Mol. Genet., 14: 1199-210 (2005)). However, indoprofen is not a useful drug for SMA because it is only weakly active in increasing SMN expression, does not enter the brain at sufficient levels, and has lethal side effects due to its cyclooxygenase (Cox) inhibitory activity.

Accordingly, there is a need for new compounds, compositions, and methods that can address these problems.

### BRIEF SUMMARY OF THE INVENTION

In one aspect, the invention provides a compound or pharmaceutically acceptable salt of Formula I: 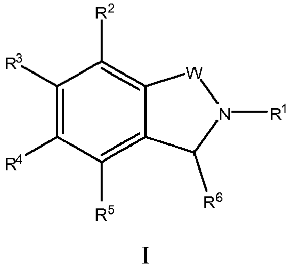 wherein W and R¹-R⁶ are as described herein. In another aspect, the invention provides a compound of Table 1 or pharmaceutically acceptable salt thereof. The invention also provides a pharmaceutical composition comprising at least one compound of Formula I or Table 1, or pharmaceutically acceptable salt thereof, and a pharmaceutically acceptable carrier.

In an additional aspect, the invention provides a method of increasing SMN expression in a cell comprising administering a compound of the invention to a cell comprising a nucleic acid encoding SMN2, whereby expression of SMN is increased. The invention further provides a method of treating spinal muscular atrophy (SMA) in a mammal comprising administering a therapeutically effective amount of at least one compound of the invention to the mammal, whereupon SMA is treated.

In yet another aspect, the invention provides a method of increasing the expression of excitatory amino acid transporter (EAAT2) in a cell comprising administering a compound of the invention to a cell comprising a nucleic acid that encodes EAAT2, whereby expression of EAAT2 is increased. The invention further provides a method of treating or preventing a neurological disorder (e.g., SMA, muscular dystrophy, multiple sclerosis, Parkinson's disease, Alzheimer's disease, amyotrophic lateral sclerosis (ALS), Huntington's disease, or epilepsy) in a mammal comprising administering a therapeutically effective amount of at least one compound of the invention to the mammal, whereupon the neurological disorder is treated or prevented. The treatment of the neurological disorder can be effected, for example, by increasing the expression of EAAT2.

In still another aspect, the invention provides a method of increasing in a cell the expression of a nucleic acid that encodes a translational stop codon, the method comprising administering a compound of the invention to a cell comprising a nucleic acid that encodes a translational stop codon, whereby expression of the nucleic acid is increased.

### BRIEF DESCRIPTION OF THE SEVERAL VIEWS OF THE DRAWING(S)

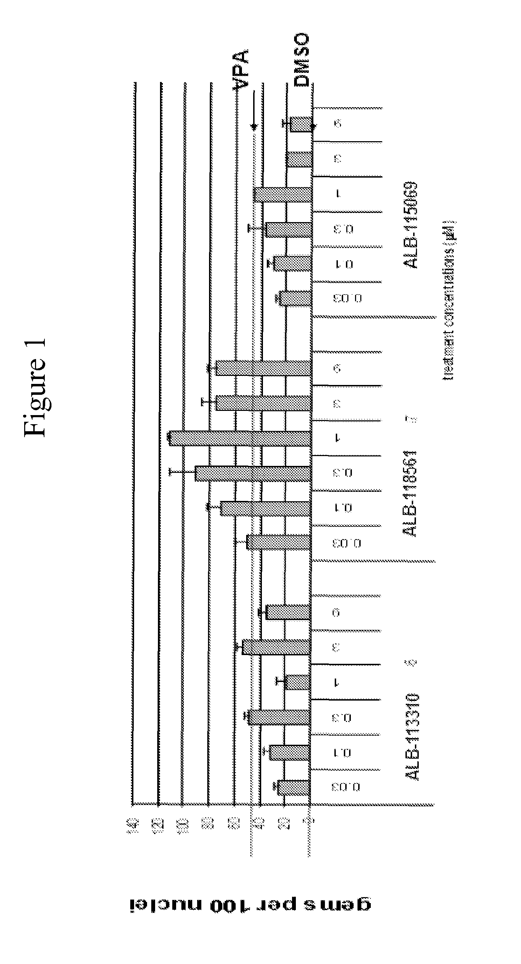

Figure 1 is a graph of SMN gems per 100 nuclei of fibroblasts treated with a compound of Formula I (ALB-118561) or other compounds. Compounds ALB-113310 and ALB-115069 are disclosed in WO 07/109211. SMN gem levels in cells treated with valproic acid (VPA) or dimethylsulfoxide (DMSO) in the absence of the other compounds are indicated on the graph.

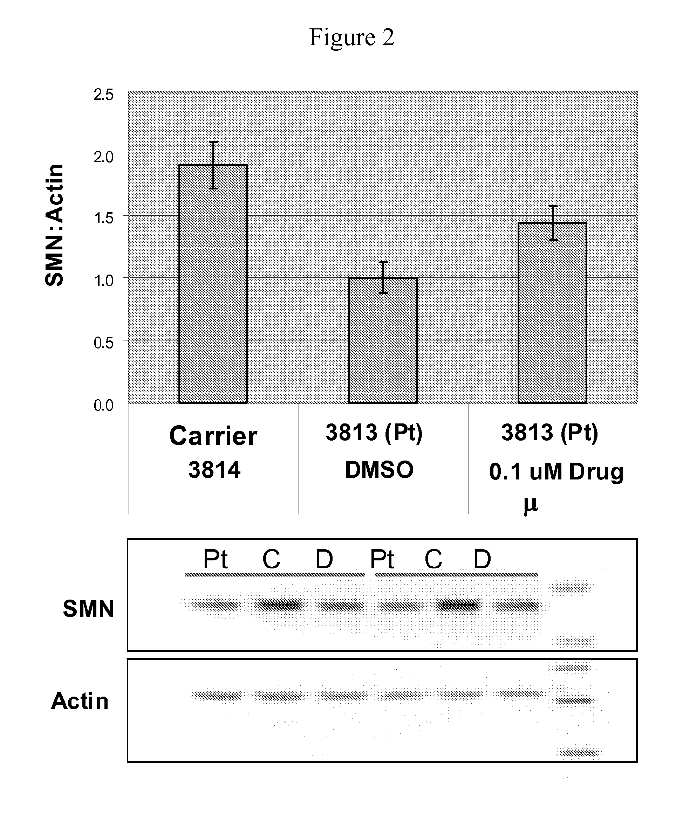

Figure 2 is a graph of the ratio of SMN to Actin in 3813 (Pt) fibroblasts treated with a compound of Formula I as compared to untreated carrier 3184 fibroblasts and 3813 (Pt) fibroblasts in DMSO only. Western blots of SMN and Actin also are shown in Figure 2.

### DETAILED DESCRIPTION OF THE INVENTION

The invention provides a compound or pharmaceutically acceptable salt of Formula I: 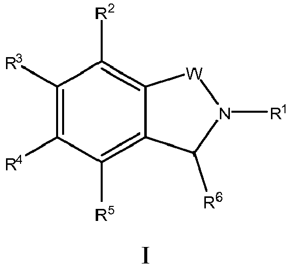 wherein
W is selected from the group consisting of C(O), C(S), and CH₂;
R¹ is aryl, cycloalkylaryl, heterocycloalkylaryl, heteroaryl, or heterocycloalkyl, each of which is optionally substituted with 1 to 3 substituents individually selected from the group consisting of C₁-C₈ alkyl, C₃-C₆ cycloalkyl, C₁-C₈ alkoxy, C₁-C₈ haloalkoxy, C₁-C₈ haloalkyl, halogen, -CN, -C(O)OR⁷, -C(O)NR⁷(R⁸), -NO₂, -SO₂NR⁷(R⁸), -NR⁹(SO₂)R¹⁰, -NR⁷C(O)NR⁸R⁹, amino, C₁-C₄ alkylamino, C₃-C₆ cycloalkylamino, aryl, heteroaryl, and heterocycloalkyl;
R², R³, R⁴, and R⁵ are independently selected from the group consisting of H, hydroxyl, C₁-C₈ alkyl, C₂-C₈ alkenyl, C₃-C₆ cycloalkyl, C₁-C₈ alkoxy, C₃-C₆ cycloalkyloxy, C₁-C₈ haloalkoxy, C₁-C₈ haloalkyl, halogen, alkylsulfonyl, -CN, -NO₂,-SO₂NR⁷(R⁸), ₋NR⁹(SO₂)R¹⁰, -NR⁷C(O)R⁸, -NR⁷C(O)NR⁸R⁹, amino, C₁-C₄ alkylamino, C₃-C₆ cycloalkylamino, aryl, heteroaryl, and heterocycloalkyl; or
R² and R³, R³ and R⁴ or R⁴ and R⁵ can be taken together with the carbon atoms to which they are attached to form a 5- or 6-membered cycloalkyl or heterocycloalkyl comprising 1 or 2 heteroatoms selected from the group consisting ofN, O, and S;
R⁶ is selected from the group consisting of H and C₁-C₈ alkyl;
R⁷, R⁸, and R⁹ are independently selected from the group consisting of H, C₁-C₈ alkyl, C₃-C₆ cycloalkyl, aryl, and heteroaryl; and
R¹⁰ is selected from the group consisting of C₁-C₈ alkyl, C₃-C₆ cycloalkyl, aryl, and heteroaryl;
wherein C₁-C₈ alkyl is optionally substituted with one or more substituents individually selected from the group consisting of -CN, C₁-C₈ alkoxy, C₁-C₈ haloalkoxy, C₁-C₈ haloalkyl, -C(O)OR⁶, -C(O)NR⁶(R⁷), amino, C₁-C₄ alkylamino, C₃-C₆ cycloalkylamino, and heterocycloalkyl, and
wherein aryl is optionally substituted with one or more substituents individually selected from the group consisting of halogen, -NO₂, -CN, C₁-C₈ alkyl, C₁-C₈ haloalkyl, and C₁-C₈ alkoxy.

As used herein, unless otherwise specified, the term "alkyl" means a saturated straight chain or branched non-cyclic hydrocarbon having an indicated number of carbon atoms (e.g., C₁-C₂₀, C₁-C₁₀, C₁-C₄, etc.). Representative saturated straight chain alkyls include methyl, ethyl, *n*-propyl, *n*-butyl, *n*-pentyl, *n*-hexyl, *n*-heptyl, *n*-octyl, *n*-nonyl and *n-*decyl; while representative saturated branched alkyls include isopropyl, *sec-*butyl, isobutyl, *tert*-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like. An alkyl group can be unsubstituted or substituted.

The term "cycloalkyl," as used herein, means a cyclic alkyl moiety containing from, for example, 3 to 6 carbon atoms, preferably from 5 to 6 carbon atoms. Examples of such moieties include cyclopropyl, cyclobutyl, cyclopentyl, cyclohexyl, and the like.

The term "heterocycloalkyl" means a cycloalkyl moiety having one or more heteroatoms selected from nitrogen, sulfur, and/or oxygen. Preferably, a heterocycloalkyl is a 5 or 6-membered monocyclic ring and contains one, two, or three heteroatoms selected from nitrogen, oxygen, and/or sulfur. The heterocycloalkyl can be attached to the parent structure through a carbon atom or through any heteroatom of the heterocycloalkyl that results in a stable structure. Examples of such heterocyclic rings are pyrrolinyl, pyranyl, piperidyl, tetrahydrofuranyl, tetrahydrothiopheneyl, and morpholinyl.

As used herein, unless otherwise specified, the term "alkoxy" means -O-(alkyl), wherein alkyl is defined above. The term "cycloalkyloxy" means -O-(cycloalkyl), wherein cycloalkyl is defined above. Furthermore, as used herein, the term "haloalkoxy" means an alkoxy substituted with one or more halogens, wherein alkoxy and halogen are defined as above.

As used herein, the term "alkylsulfonyl" means -SO₂-(alkyl), wherein alkyl is defined above. An example of an alkylsulfonyl is methylsulfonyl.

As used herein, unless otherwise specified, the term "alkylamino" means -NH(alkyl) or -N(alkyl)(alkyl), wherein alkyl is defined above. As used herein, unless otherwise specified, the term "cycloalkylamino" means -NH(cycloalkyl) or -N(cycloalkyl)(cycloalkyl), wherein cycloalkyl is defined above.

As used herein, unless otherwise specified, the term "alkenyl group" means a straight chain or branched non-cyclic hydrocarbon having an indicated number of carbon atoms (e.g., C₂-C₂₀, C₂-C₁₀, C₂-C₄, etc.) and including at least one carbon-carbon double bond. Representative straight chain and branched alkenyls include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl, 3-decenyl, and the like. Any unsaturated group (double bond) of an alkenyl can be unconjugated or conjugated to another unsaturated group. An alkenyl group can be unsubstituted or substituted.

As used herein, unless otherwise specified the term "alkynyl group" means a straight chain or branched non-cyclic hydrocarbon having an indicated number of carbon atoms (e.g., C₂-C₂₀, C₂-C₁₀, C₂-C₆, etc.), and including at least one carbon-carbon triple bond. Representative straight chain and branched alkynyls include -acetylenyl, -propynyl, -1-butynyl, -2-butynyl, -1-pentynyl, -2-pentynyl, -3-methyl-1-butynyl, -4-pentynyl, -1-hexynyl, -2-hexynyl, -5-hexynyl, -1-heptynyl, -2-heptynyl, -6-heptynyl, -1-octynyl, -2-octynyl, -7-octynyl, -1-nonynyl, -2-nonynyl, -8-nonynyl, -1-decynyl, -2-decynyl, -9-decynyl, and the like. Any unsaturated group (triple bond) of an alkynyl can be unconjugated or conjugated to another unsaturated group. An alkynyl group can be unsubstituted or substituted.

As used herein, unless otherwise specified, the term "halogen" or "halo" means fluoro, chloro, bromo, or iodo. Furthermore, unless otherwise specified, the term "haloalkyl" means an alkyl substituted with one or more halogens, wherein alkyl and halogen are defined as above.

The term "aryl" refers to an unsubstituted or substituted aromatic carbocyclic moiety, as commonly understood in the art, and includes monocyclic and polycyclic aromatics such as, for example, phenyl, biphenyl, naphthyl, anthracenyl, pyrenyl, and the like. An aryl moiety generally contains from, for example, 6 to 30 carbon atoms, preferably from 6 to 18 carbon atoms, more preferably from 6 to 14 carbon atoms and most preferably from 6 to 10 carbon atoms. It is understood that the term aryl includes carbocyclic moieties that are planar and comprise 4n+2 π electrons, according to Hückel's Rule, wherein n = 1, 2, or 3.

The terms "cycloalkylaryl" and "heterocycloalkylaryl" refer to an aryl, as defined herein, that is substituted with a cycloalkyl group or a heterocycloalkyl group, respectively, as defined herein, or as a fused ring, e.g., benzo. Examples of cycloalkylaryl groups include 5-, 6-, 7-, or 8-1,2,3,4-tetrahydronaphthalenyl and 4-, 5-, 6-, or 7-2,3-dihydro-1H-indenyl. Examples of heterocycloalkylaryl include 5-, 6-, 7-, or 8-1,2,3,4-tetrahydroquinolinyl, 5-, 6-, 7-, or 8-1,2,3,4-tetrahydroquinoxalinyl, and 5-, 6-, 7-, or 8-2,3-dihydrobenzo[b][1,4]dioxinyl.

The term "heteroaryl" refers to aromatic 4, 5, or 6 membered monocyclic groups, 9 or 10 membered bicyclic groups, and 11 to 14 membered tricyclic aryl groups having one or more heteroatoms (O, S, or N). Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The nitrogen and sulfur atoms may optionally be oxidized, and the nitrogen atoms may optionally be quaternized. Illustrative examples of heteroaryl groups are pyridinyl, pyridazinyl, pyrimidyl, pyrazinyl, triazinyl, pyrrolyl, pyrazolyl, imidazolyl, (1,2,3,)- and (1,2,4)-triazolyl, pyrazinyl, pyrimidinyl, tetrazolyl, furyl, thiophenyl, isothiazolyl, thiazolyl, isoxazolyl, oxadiazolyl, oxazolyl, pyrrolo[2,3-c]pyridinyl, pyrrolo[3,2-c]pyridinyl, pyrrolo[2,3-b]pyridinyl, pyrrolo[3,2-b]pyridinyl, pyrrolo[3,2-d]pyrimidinyl, and pyrrolo[2,3-d]pyrimidinyl.

As used herein, unless otherwise specified, the term "substituted" means a group substituted by one or more substituents, such as, alkyl, alkenyl, alkynyl, cycloalkyl, aroyl, halo, haloalkyl (e.g., trifluoromethyl), haloalkoxy (e.g., trifluoromethoxy), hydroxy, alkoxy, alkylthioether, cycloalkyloxy, heterocylooxy, oxo, alkanoyl, aryl, arylalkyl, alkylaryl, heteroaryl, heteroarylalkyl, alkylheteroaryl, heterocyclo, aryloxy, alkanoyloxy, amino, alkylamino, arylamino, arylalkylamino, cycloalkylamino, heterocycloamino, alkanoylamino, aroylamino, aralkanoylamino, substituted alkanoylamino, substituted arylamino, substituted aralkanoylamino, thiol, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfonamido (e.g., -SO₂NH₂), substituted sulfonamido, nitro, cyano, carboxy, carbamyl (e.g., -CONH₂), substituted carbamyl (e.g., -CONH-alkyl, -CONH-aryl, -CONH-arylalkyl, or instances where there are two substituents on the nitrogen selected from alkyl or arylalkyl), alkoxycarbonyl, aryl, substituted aryl, guanidino, substituted or unsubstituted heterocycloalkyl, and substituted or unsubstituted heteroaryl.

Whenever a range of the number of atoms in a structure is indicated (e.g., a C₁-C₈, C₁-C₆, C₁-C₄, or C₁-C₃ alkyl, haloalkyl, alkylamino, alkenyl, etc.), it is specifically contemplated that any sub-range or individual number of carbon atoms falling within the indicated range also can be used. Thus, for instance, the recitation of a range of 1-8 carbon atoms (e.g., C₁-C₈), 1-6 carbon atoms (e.g., C₁-C₆), 1-4 carbon atoms (e.g., C₁-C₄), 1-3 carbon atoms (e.g., C₁-C₃), or 2-8 carbon atoms (e.g., C₂-C₈) as used with respect to any chemical group (e.g., alkyl, haloalkyl, alkylamino, alkenyl, etc.) referenced herein encompasses and specifically describes 1, 2, 3, 4, 5, 6, 7, or 8 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 1-7 carbon atoms, 1-8 carbon atoms, 2-3 carbon atoms, 2-4 carbon atoms, 2-5 carbon atoms, 2-6 carbon atoms, 2-7 carbon atoms, 2-8 carbon atoms, 3-4 carbon atoms, 3-5 carbon atoms, 3-6 carbon atoms, 3-7 carbon atoms, 3-8 carbon atoms, 4-5 carbon atoms, 4-6 carbon atoms, 4-7 carbon atoms, 4-8 carbon atoms, 5-6 carbon atoms, 5-7 carbon atoms, 5-8 carbon atoms, 6-7 carbon atoms, or 6-8 carbon atoms, as appropriate).

The invention encompasses all compounds described by Formula I, and salts thereof, without limitation. However, for the purposes of further illustration, aspects and embodiments of the invention are discussed herein.

In one embodiment of the invention, R¹ is selected from the group consisting of:
a C₆₋₃₀ aryl,
a heteroaryl consisting of one or two oxygen or sulfur atoms and/or from one to four nitrogen atoms provided that the total number of heteroatoms in each ring is four or less and each ring has at least one carbon atom,
a heterocycloalkyl consisting of a 5- or 6-membered monocyclic ring that contains one, two, or three heteroatoms selected from nitrogen, oxygen, and/or sulfur,
a C₅₋₇ cycloalkyl-fused phenyl, and
a heterocycloalkyl-fused phenyl, wherein the heterocycloalkyl consists of a 5- or 6-membered monocyclic ring that contains one, two, or three heteroatoms selected from nitrogen, oxygen, and/or sulfur,
each of which is optionally substituted with 1 to 3 substituents individually selected from the group consisting of C₁-C₈ alkyl, C₃-C₆ cycloalkyl, C₁-C₈ alkoxy, C₁-C₈ haloalkoxy, C₁-C₈ haloalkyl, halogen, -CN, -C(O)OR⁷, -C(O)NR⁷(R⁸), -NO₂, -SO₂NR⁷(R⁸), -NR⁹(SO₂)R¹⁰, -NR⁷C(O)NR⁸R⁹, amino, C₁-C₄ alkylamino, C₃-C₆ cycloalkylamino, aryl, heteroaryl, and heterocycloalkyl.

Non-limiting examples of suitable R¹ groups include phenyl, biphenyl, naphthyl, anthracenyl, pyrenyl, 1H-indolyl, benzo[b]thiophenyl, benzofuranyl, 1H-benzo[d]imidazolyl, 2H-indazolyl, benzo[d]thiazolyl, benzo[d]oxazolyl, isoquinolinyl, quinolinyl, quinoxalinyl, 3,4-dihydro-2H-benzo[b][1,4]oxazinyl, 2H-benzo[b][1,4]oxazin-3(4H)-onyl, 1H-benzo[d]-imidazol-2(3H)-onyl, quinoline-2(1H)-onyl, 2,3-dihydro-1H-indenyl, pyridinyl, pyridazinyl, pyrimidyl, pyrazinyl, triazinyl, pyrrolyl, pyrazolyl, imidazolyl, (1,2,3,)- and (1,2,4)-triazolyl, pyrazinyl, pyrimidinyl, tetrazolyl, furyl, thiophenyl, isothiazolyl, thiazolyl, isoxazolyl, oxadiazolyl, oxazolyl, pyrrolo[2,3-c]pyridinyl, pyrrolo[3,2-c]pyridinyl, pyrrolo[2,3-b]pyridinyl, pyrrolo[3,2-b]pyridinyl, pyrrolo[3,2-d]pyrimidinyl, pyrrolo[2,3-d]pyrimidinyl, quinazolinyl, phthalazinyl, imidazo[1,2-a]pyridinyl, pyrrolinyl, pyranyl, piperidyl, tetrahydrofuranyl, tetrahydrothiopheneyl, morpholinyl, 1,2,3,4-tetrahydronaphthalenyl, 2,3-dihydro-1H-indenyl, 1,2,3,4-tetrahydroquinolinyl, 1,2,3,4-tetrahydroquinoxalinyl, and 2,3-dihydrobenzo[b] [1,4]dioxinyl.

More particularly, R¹ can be selected from the group consisting of
1H-indolyl (e.g., 4-, 5-, 6-, or 7-1H-indolyl),
benzo[b]thiophenyl (e.g., 4-, 5-, 6-, or 7-benzo[b]thiophenyl),
benzofuranyl (e.g., 4-, 5-, 6-, or 7-benzofuranyl),
1H-benzo[d]imidazolyl (e.g., 4-, 5-, 6-, or 7-1H-benzo[d]imidazolyl),
2H-indazolyl (e.g., 4-, 5-, 6-, or 7-2H-indazolyl),
benzo[d]thiazolyl (e.g., 4-, 5-, 6-, or 7-benzo[d]thiazolyl),
benzo[d]oxazolyl (e.g., 4-, 5-, 6-, or 7-benzo[d]oxazolyl),
isoquinolinyl (e.g., 5-, 6-, 7- or 8-isoquinolinyl),
quinolinyl (e.g., 5-, 6-, 7- or 8-quinolinyl),
quinoxalinyl (e.g., 5-, 6-, 7- or 8-quinoxalinyl),
3,4-dihydro-2H-benzo[b][1,4]oxazinyl (e.g., 5-, 6-, 7-, or 8-3,4-dihydro-2H-benzo[b][1,4]oxazinyl),
2H-benzo[b][1,4]oxazin-3(4H)-onyl (e.g., 5-, 6-, 7-, or 8-2H-benzo[b][1,4]oxazin-3(4H)-onyl),
1H-benzo[d]-imidazol-2(3H)-onyl (e.g., 4-, 5-, 6-, or 7-1H-benzo[d]-imidazol-2(3H)-onyl),
quinoline-2(1H)-onyl (e.g., 5-, 6-, 7- or 8-quinoline-2(1H)-onyl),
pyridinyl (e.g., 2-, 3- 4-, or 5-pyridinyl),
furyl (e.g., 2-, 3-, or 4-furyl),
thiophenyl (e.g., 2-, 3-, or 4-thiophenyl),
pyrrolo[2,3-c]pyridinyl (e.g., 4-, 5-, or 7-pyrrolo[2,3-c]pyridinyl),
pyrrolo[3,2-c]pyridinyl (e.g., 4-, 6-, or 7-pyrrolo[3,2-c]pyridinyl),
pyrrolo[2,3-b]pyridinyl (e.g., 4-, 5-, or 6-pyrrolo[2,3-b]pyridinyl),
pyrrolo[3,2-b]pyridinyl (e.g., 5-, 6-, or 7-pyrrolo[3,2-b]pyridinyl),
pyrrolo[3,2-d]pyrimidinyl (e.g., 2- or 4-pyrrolo[3,2-d]pyrimidinyl),
pyrrolo[2,3-d]pyrimidinyl (e.g., 2- or 4-pyrrolo[2,3-d]pyrimidinyl),
quinazolinyl (e.g., 5-, 6-, 7-, or 8-quinazolinyl),
phthalazinyl (e.g., 5-, 6-, 7-, or 8-phthalazinyl),
imidazo[1,2-a]pyridinyl (e.g., 5-, 6-, 7-, or 8-imidazo[1,2-a]pyridinyl),
1,2,3,4-tetrahydronaphthalenyl (e.g., 5-, 6-, 7-, or 8-1,2,3,4-tetrahydronaphthalenyl),
2,3-dihydro-1H-indenyl (e.g., 4-, 5-, 6-, or 7-2,3-dihydro-1H-indenyl),
1,2,3,4-tetrahydroquinolinyl (e.g., 5-, 6-, 7-, or 8-1,2,3,4-tetrahydroquinolinyl),
1,2,3,4-tetrahydroquinoxalinyl (e.g., 5-, 6-, 7-, or 8-1,2,3,4-tetrahydroquinoxalinyl), and
2,3-dihydrobenzo[b][1,4]dioxinyl (e.g., 5-, 6-, 7-, or 8-2,3-dihydrobenzo[b][1,4]dioxinyl). In an embodiment, R¹ is 1H-indolyl (e.g., 4-, 5-, 6-, or 7-1H-indolyl) or quinolinyl (e.g., 5-, 6-, 7- or 8-quinolinyl), particularly 5-indolyl, 6-indolyl, 5-quinolinyl, or 6-quinolinyl. In an especially preferred embodiment, R¹ can be selected from the group consisting of 1H-indolyl (e.g., 4-, 5-, 6-, or 7-1H-indolyl), benzo[b]thiophenyl (e.g., 4-, 5-, 6-, or 7-benzo[b]thiophenyl), benzofuranyl (e.g., 4-, 5-, 6-, or 7-benzofuranyl), benzo[d]thiazolyl (e.g., 4-, 5-, 6-, or 7-benzo[d]thiazolyl), and 2H-indazolyl (e.g., 4-, 5-, 6-, or 7-2H-indazolyl).

By way of further illustration, R¹ can be selected from the group consisting of 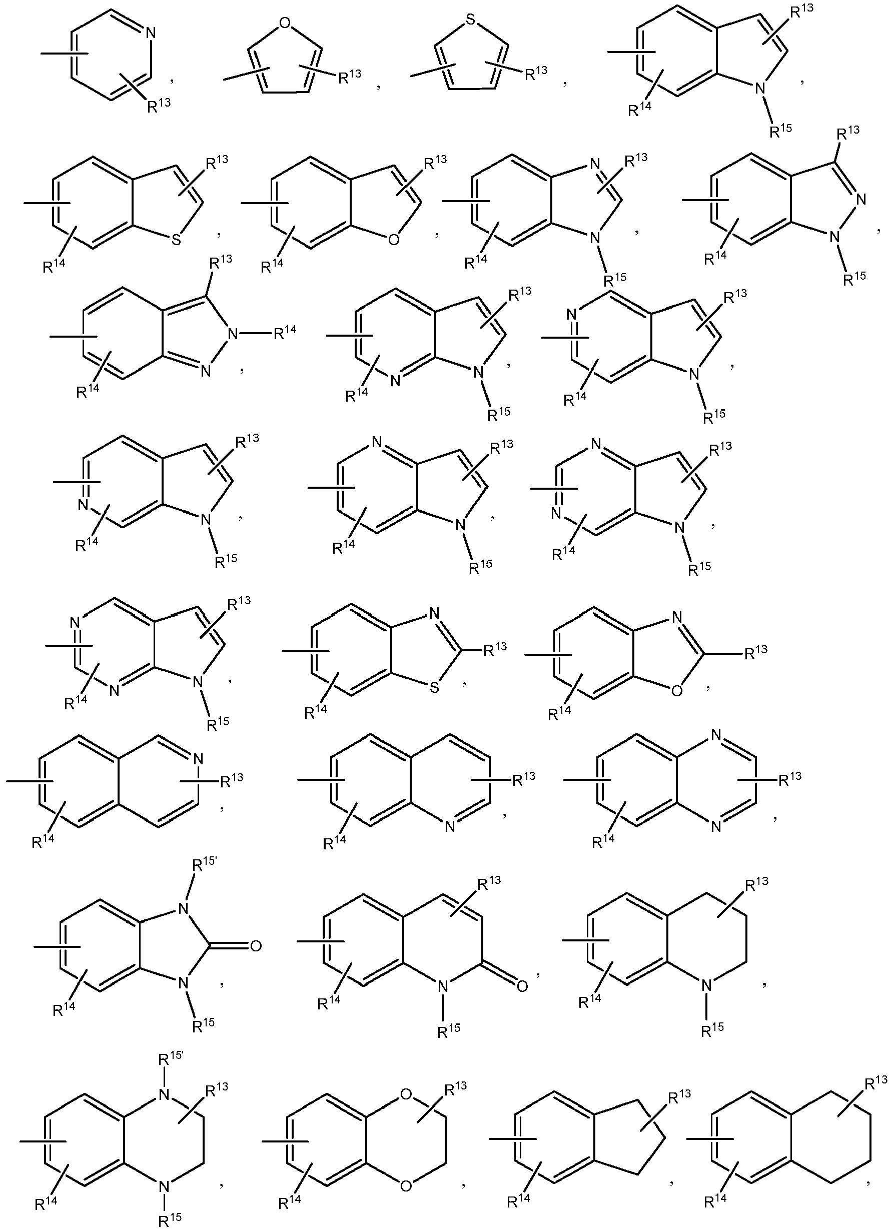 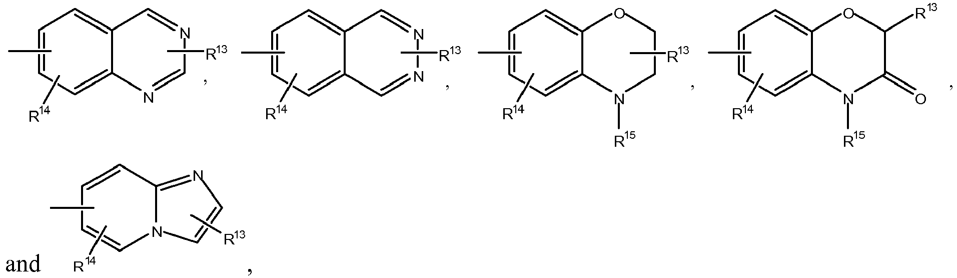 wherein
R¹³ and R¹⁴ are the same or different and each is selected from the group consisting of H, C₁-C₈ alkyl, C₃-C₆ cycloalkyl, C₁-C₈ alkoxy, C₁-C₈ haloalkoxy, C₁-C₈ haloalkyl, halogen, -CN, -C(O)OR⁷, -C(O)NR⁷(R⁸), -NO₂, -SO₂NR⁷(R⁸), -NR⁹(SO₂)R¹⁰, -NR⁷C(O)NR⁸R⁹, amino, C₁-C₄ alkylamino, C₃-C₆ cycloalkylamino, aryl, heteroaryl, and heterocycloalkyl; and
R¹⁵ and R^{15'} are the same or different and each is hydrogen or C₁-C₈ alkyl,
wherein C₁-C₈ alkyl is optionally substituted with one or more subsitutents individually selected from the group consisting of of -CN, C₁-C₈ alkoxy, C₁-C₈ haloalkoxy, C₁-C₈ haloalkyl, -C(O)OR⁶, -C(O)NR⁶(R⁷), amino, C₁-C₄ alkylamino, C₃-C₆ cycloalkylamino, and heterocycloalkyl.

In some embodiments, R¹ is selected from the group consisting of 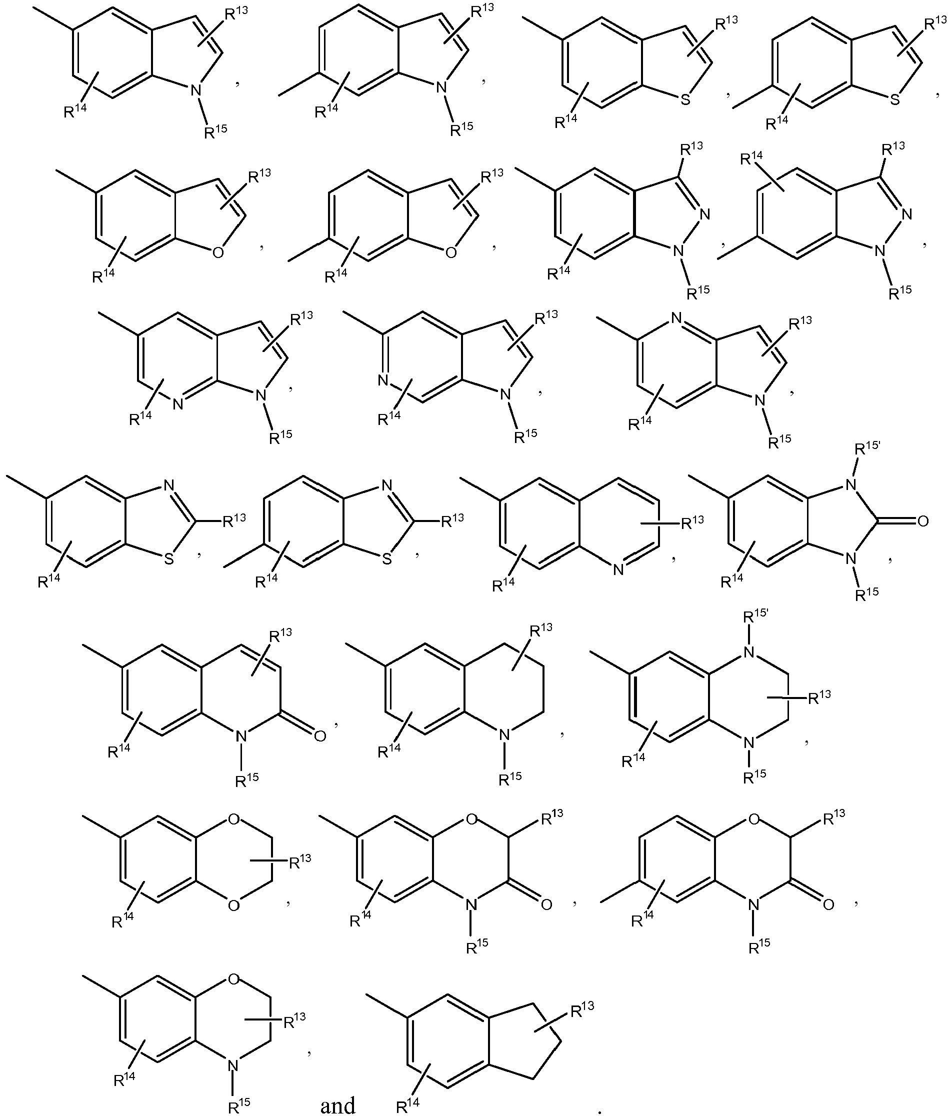 wherein R¹³, R¹⁴, R¹⁵, and R^{15'}are as previously defined.

In any of the foregoing examples, R¹³ can desirably be selected as H, halogen, -CN, C₁-C₈ alkyl, C₁-C₈ alkoxy, C₁-C₈ haloalkoxy, C₁-C₈ haloalkyl, amino, C₁-C₄ alkylamino, or phenyl, in which the C₁-C₈ alkyl is optionally substituted with one or more subsitutents individually selected from the group consisting of C₁-C₈ alkoxy, C₁-C₈ haloalkoxy, C₁-C₈ haloalkyl, -C(O)OR⁶, -C(O)NR⁶(R⁷), -CN, amino, C₁-C₄ alkylamino, C₃-C₆ cycloalkylamino, and heterocycloalkyl. In particular, R¹³ can be H or C₁-C₈ alkyl, in which the C₁-C₈ alkyl is optionally substituted with one or more subsitutents individually selected from the group consisting of C₁-C₈ alkoxy, C₁-C₈ haloalkoxy, C₁-C₈ haloalkyl, -C(O)OR⁶, -C(O)NR⁶(R⁷) -CN, amino, C₁-C₄ alkylamino, C₃-C₆ cycloalkylamino, and heterocycloalkyl. Preferably, R¹³ is C₁-C₈ alkyl that is optionally substituted with one or more subsitutents individually selected from the group consisting of C₁-C₈ alkoxy, C₁-C₈ haloalkoxy, C₁-C₈ haloalkyl, -C(O)OR⁶, -C(O)NR⁶(R⁷), -CN, amino, C₁-C₄ alkylamino, C₃-C₆ cycloalkylamino, and heterocycloalkyl. For example, R¹³ can be methyl, ethyl, propyl, isopropyl, -(CH₂)ₙCN, or -(CH₂)ₙNH₂, in which n is 1 to 8.

Similarly, in any of the foregoing examples, R¹⁴ preferably is hydrogen or C₁-C₈ alkyl (e.g., methyl, ethyl), and R¹⁵ and R^{15'} preferably are hydrogen or C₁-C₈ alkyl (e.g., methyl, ethyl, i-propyl).

In another embodiment, optionally in combination with the particular embodiments and aspects discussed with respect to R¹, W can be C(O) or CH₂.

In still another embodiment, R² is selected from the group consisting of H, hydroxyl, halogen, C₁-C₈ alkyl, C₁-C₈ alkoxy, C₁-C₈ haloalkoxy, nitro, amino, C₁-C₄ alkylamino, aryl, and heterocycloalkyl. Preferably, R² is selected from the group consisting of H, hydroxyl, nitro, amino, C₁-C₈ alkyl, C₁-C₈ alkoxy, C₁-C₈ haloalkoxy, -NR⁷C(O)R⁸, and phenyl. This embodiment optionally can be employed in combination with the particular embodiments and aspects discussed with respect to R¹ and W.

In yet another embodiment, R³ is H, hydroxyl, halogen, C₁-C₈ alkyl, C₂-C₈ alkenyl, C₁-C₈ alkoxy, C₃-C₆ cycloalkyloxy, C₁-C₈ haloalkoxy, alkylsulfonyl, -NO₂, amino, C₁-C₄ alkylamino, -NR⁷C(O)R⁸, heterocycloalkyl, or heteroaryl. In particular embodiments, R³ is H, hydroxyl, halogen (e.g., Br, Cl, I), -NO₂, C₁-C₈ alkyl (e.g., Me, Et,'Pr), C₂-C₈ alkenyl (e.g., 2-butenyl), -NR⁷C(O)R⁸ (e.g., -NHC(O)Me, -NHC(O)Et), C₁-C₈ alkoxy (e.g., OMe, OEt), C₃-C₆ cycloalkyloxy (e.g., cyclopentyloxy, cyclohexyloxy), C₁-C₈ haloalkoxy (e.g., -OCHF₂, -OCH₂CF₃, -OCBrF₂, or -OCF₂CHFCl), amino, -N(CH₃)₂, heterocycloalkyl (e.g., pyrrolidinyl, morpholinyl, piperazinyl), or heteroaryl (e.g., pyridinyl, pyrimidinyl). Preferably, R³ is H, halogen (e.g., Br, Cl, I), C₁-C₈ alkyl (e.g., Me, Et, ⁱPr), C₁-C₈ alkoxy (e.g., OMe, OEt), C₁-C₈ haloalkoxy (e.g., -OCHF₂, -OCH₂CF₃, -OCBrF₂, or -OCF₂CHFCl), amino, C₁-C₄ alkylamino (e.g., N(CH₃)₂), or heterocycloalkyl (e.g., pyrrolidinyl, morpholinyl, piperazinyl). These embodiments optionally can be employed in combination with the particular embodiments and aspects discussed with respect to R¹, W, and R².

According to a further embodiment of the invention, R⁴ is H, hydroxyl, halogen, C₁-C₈ alkyl, C₁-C₈ alkoxy, C₁-C₈ haloalkoxy, amino, C₁-C₄ alkylamino, or heterocycloalkyl. In preferred embodiments, R⁴ is H, C₁-C₈ alkoxy, or C₁-C₈ haloalkoxy (e.g., -OCHF₂, -OCH₂CF3, -OCBrF₂, or -OCF₂CHFCl). This embodiment optionally can be employed in combination with the particular embodiments and aspects discussed with respect to R¹, W, R², and R³_{.}

In an additional embodiement, R⁵ can be selected from H, hydroxyl, halogen, C₁-C₈ alkyl, C₁-C₈ alkoxy, C₁-C₈ haloalkoxy, amino, C₁-C₄ alkylamino, or -NR⁷C(O)R⁸. R⁵ preferably is H, C₁-C₈ alkyl, -NHC(O)R⁸, C₁-C₆ alkoxy, C₁-C₆ haloalkoxy, amino, and C₁-C₄ alkylamino. This embodiment optionally can be employed in combination with the particular embodiments and aspects discussed with respect to R¹, W, R², R³, and R⁴.

As an alternative to one or more of the foregoing embodiements, but optionally in combination with others as applicable, R² and R³, R³ and R⁴ or R⁴ and R⁵ can be taken together with the carbon atoms to which they are attached to form a 5- or 6-membered cycloalkyl or heterocycloalkyl comprising 1 or 2 heteroatoms selected from the group consisting of N, O, and S. For example, R³ and R⁴ can be taken together as -OCH₂CH₂O- to form a fused bicyclic ring with the benzene core of the compound of Formula I.

Specific examples of compounds of the invention are provided in Table 1. Accordingly, the invention encompasses each of the compounds provided in Table 1. In a preferred embodiment, the compound is selected from the group consisting of

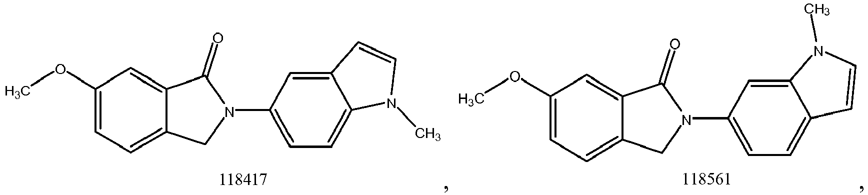

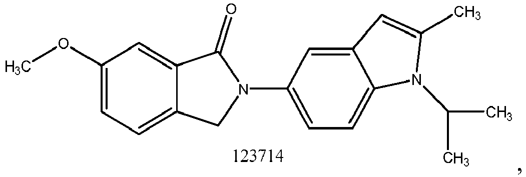

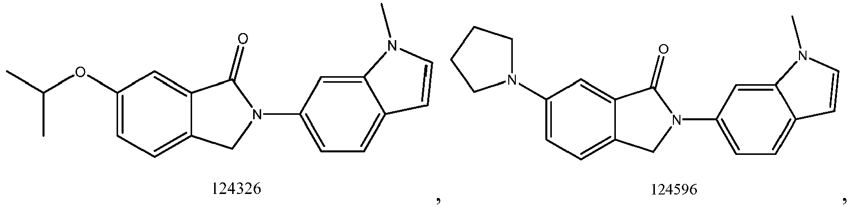

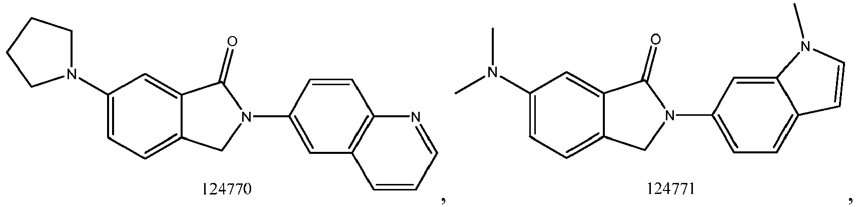

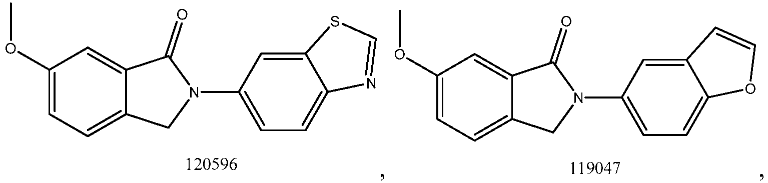

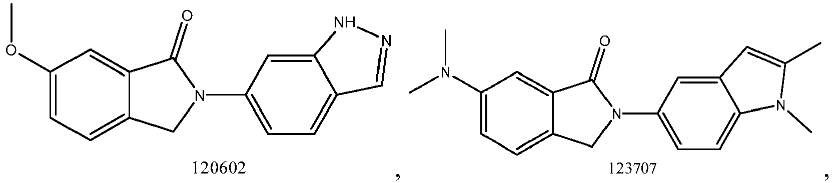

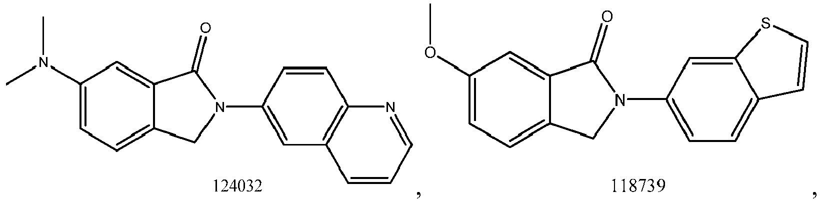

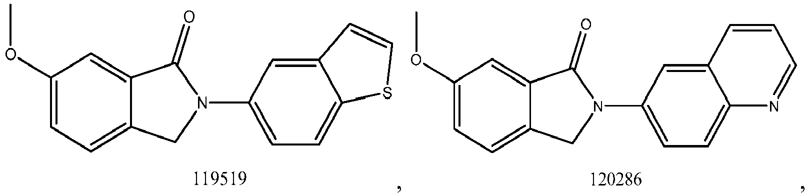

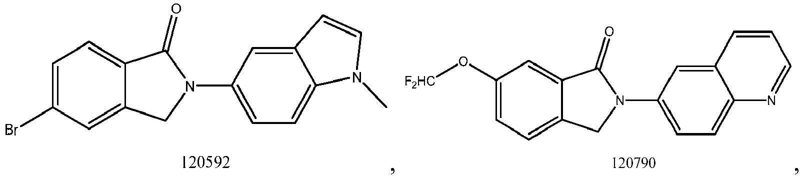

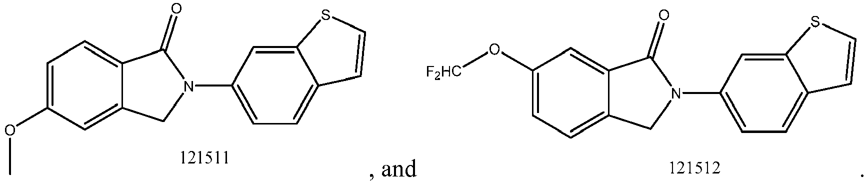

The pharmaceutically acceptable salts of the compounds of Formula I or Table 1 can be any pharmaceutically acceptable salt, prepared in any manner. Typically, such salts can be prepared from a compound using relatively nontoxic acids or bases, depending on the particular starting "parent" compound. Base addition salts of parent compounds with relatively acidic functionalities can be prepared by contacting the free acid form of such compounds with a sufficient amount of the desired base, either neat or in a suitable inert solvent. Examples of pharmaceutically acceptable base addition salts include sodium, potassium, calcium, ammonium, organic amino, magnesium salts, and the like. Acid addition salts of parent compounds having relatively basic functionalities can be obtained by contacting the free base form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al, Journal of Pharmaceutical Science, 66: 1-19 (1977)). Compounds of the invention may contain both basic and acidic functionalities, which allow the compounds to be converted into either base or acid addition salts. The neutral forms of the compounds can be regenerated by contacting the salt with a base or acid and isolating the parent compound in the conventional manner.

The compounds of the invention described herein can be prepared by any of several techniques. By way of a non-limiting example, the compounds can be prepared in accordance with Flow Diagrams 1, 2, or 3 or as set forth in the Examples.

With respect to Flow Diagram 1, compounds of Formula (a) are available from commercial sources or can be prepared using routine procedures. Formula (b) compounds can be prepared, as illustrated below in Flow Diagram 1, by reacting the ester (R = lower alkyl) of Formula (a), with a halogenating agent such as *N*-bromosuccinimide (NBS). The Formula (b) compound is then reacted with an amine of Formula (c) to provide the cyclized product of Formula I. Formula (c) compounds are available from commercial sources or can be prepared using routine procedures.

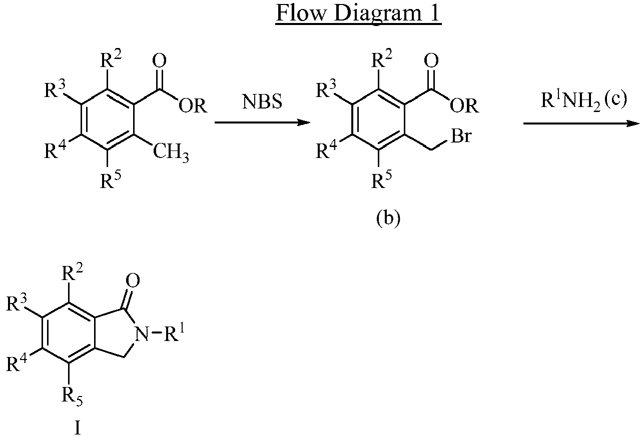

In some cases, the reaction of Formula (b) compounds with Formula (c) compounds affords the uncyclized intermediates of Formula (d), as illustrated in Flow Diagram 2. Hydrolysis of the lower esters of Formula (d) compounds to the acids of Formula (e), results in cyclization to compounds of Formula I. In some cases, the acids of Formula (e) do not cyclize under the reaction conditions to provide compounds of Formula I. In these instances, treatment of the Formula (e) compounds with a coupling agent (e.g., EDC (1-Ethyl-3-[3-dimethylaminopropyl]carbodiimide Hydrochloride)) affords the compound of Formula I.

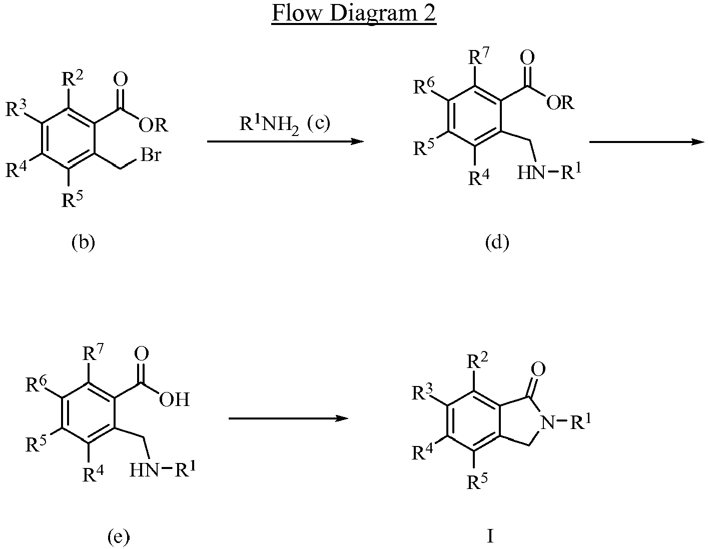

Another approach toward the preparation of Formula I compounds is illustrated in Flow Diagram 3. Specifically, reaction of Formula (f) compounds with Formula (g) compounds, wherein X is halogen and R¹ is a group as described herein in the presence of various cross-coupling catalysts provides compounds of Formula 1.

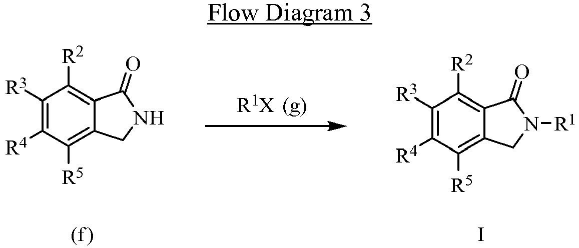

When compounds of Formula I are prepared according to Flow Diagram 1, 2, or 3 with a halogen (e.g. bromide, chloride, iodide) at R², R³, R⁴, or R⁵ (e.g., by utilization of a compound of Formula (a) containing a halogen at the desired position of the starting material), the compounds of Formula I can be further substituted by conventionally known techniques. For example, aryl, heteroaryl, alkyl, and amine derivatives can be prepared utilizing cross-coupling reactions, such as Suzuki, Stille, and Buchwald reactions.

When compounds of Formula I are prepared according to Flow Diagram 1 or 2 with a nitro at R², R³, R⁴, or R⁵ (e.g. by utilization of a compound of Formula (a) containing a nitro at the desired position of the starting material), the compounds of Formula I can be reduced to provide an amine, which can be further derivatized using conventional techniques to provide secondary amines, tertiary amines, amides, and sulfonamides.

When compounds of Formula I are prepared according to Flow Diagram 1 or 2 with a phenol group at R², R³, R⁴, or R⁵, the compounds of Formula I can be further derivatized by conventional procedures to provide ethers and esters.

The R¹ group of compounds of Formula I can be substituted with an R¹³ and/or R¹⁴ group, which can be further modified or derivatized using conventional techniques. When R¹³ and/or R¹⁴ is phenol, the compounds of Formula I can further derivatized by conventional procedures to provide ethers and esters. In certain cases, these phenol groups (e.g., 2-hydroxyquinoline) can be converted to a halogen, which in turn, can be converted to an ether, an amine, heteroaryl, aryl, or alkyl group by conventional techniques.

Any one or more of the compounds of the invention described herein can be formed as a pharmaceutical composition comprising at least one compound of the invention and a pharmaceutically acceptable carrier. The pharmaceutical composition can additionally comprise other pharmaceutically active agents or drugs. Examples of such other pharmaceutically active agents or drugs that can be suitable for use in combination with one or more compounds of the invention include drugs that enhance translational read-through (e.g., WO 2004/009558, gentamycin, or other aminoglycoside antibiotics), drugs that inhibit one or more histone deacetylase enzymes (e.g., valproate, phenylbutyrate, or hydroxyurea), or drugs that increase SMN expression via other mechanisms. The compounds of the invention also can be administered or formulated in combination with anticancer agents or other antibiotics. Suitable anticancer agents include, without limitation, alkylating agents; nitrogen mustards; folate antagonists; purine antagonists; pyrimidine antagoinists; spindle poisons; topoisomerase inhibitors; apoptosis inducing agents; angiogenesis inhibitors; podophyllotoxins; nitrosoureas; cisplatin; carboplatin; interferon; asparginase; tamoxifen; leuprolide; flutamide; megestrol ; mitomycin; bleomycin; doxorubicin; irinotecan; and taxol. Other antibiotics include macrolides (e.g., tobramycin), cephalosporins (e.g., cephalexin, cephradine, cefuroxime, cefprozil, cefaclor, cefixime, or cefadroxil), clarithromycin, erythromycin, penicillins (e.g., penicillin V), and quinolones (e. g., ofloxacin, ciprofloxacin, or norfloxacin).

The composition further comprises a pharmaceutically acceptable carrier. The carrier can be any of those conventionally used and is limited only by physico-chemical considerations, such as solubility and lack of reactivity with the active compound(s), and by the route of administration. It will be appreciated by one of skill in the art that, in addition to the following described pharmaceutical composition, the compounds of the present inventive methods can be formulated as inclusion complexes, such as cyclodextrin inclusion complexes, or liposomes.

The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular compound of the invention and other active agents or drugs used, as well as by the particular method used to administer the compound. Accordingly, there are a variety of suitable formulations of the pharmaceutical composition of the present inventive methods. The following formulations for oral, aerosol, parenteral, subcutaneous, intravenous, intramuscular, interperitoneal, rectal, and vaginal administration are exemplary and are in no way limiting. One skilled in the art will appreciate that these routes of administering the compound of the invention are known, and, although more than one route can be used to administer a particular compound, a particular route can provide a more immediate and more effective response than another route.

Injectable formulations are among those formulations that are preferred in accordance with the present invention. The requirements for effective pharmaceutical carriers for injectable compositions are well-known to those of ordinary skill in the art (See, e.g., Pharmaceutics and Pharmacy Practice, J.B. Lippincott Company, Philadelphia, PA, Banker and Chalmers, eds., pages 238-250 (1982), and ASHP Handbook on Injectable Drugs, Toissel, 4th ed., pages 622-630 (1986)).

Topical formulations are well-known to those of skill in the art. Such formulations are particularly suitable in the context of the present invention for application to the skin.

Formulations suitable for oral administration can consist of (a) liquid solutions, such as an effective amount of the inhibitor dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The compounds of the invention, alone or in combination with other suitable components, can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer. Such spray formulations also may be used to spray mucosa.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compounds of the invention can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts. Suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-β-aminopropionates, and 2-alkyl-imidazoline quaternary ammonium salts, and (e) mixtures thereof.

The parenteral formulations will typically contain from about 0.5% to about 25% by weight of the active ingredient in solution. Preservatives and buffers can be used. In order to minimize or eliminate irritation at the site of injection, such compositions may contain one or more nonionic surfactants having a hydrophile-lipophile balance (HLB) of from about 12 to about 17. The quantity of surfactant in such formulations will typically range from about 5% to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multidose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Additionally, the compounds of the invention, or compositions comprising such compounds, can be made into suppositories by mixing with a variety of bases, such as emulsifying bases or water-soluble bases. Formulations suitable for vaginal administration can be presented as pessaries, tampons, creams, gels, pastes, foams, or spray formulas containing, in addition to the active ingredient, such carriers as are known in the art to be appropriate.

One of ordinary skill in the art will readily appreciate that the compounds of the invention described herein can be modified in any number of ways to increase the therapeutic efficacy of the compound. For instance, the compound or inhibitor could be conjugated either directly or indirectly through a linker to a targeting moiety. The practice of conjugating compounds or inhibitors to targeting moieties is known in the art. The term "targeting moiety" as used herein, refers to any molecule or agent that specifically recognizes and binds to a cell-surface receptor, such that the targeting moiety directs the delivery of the compound or inhibitor to a population of cells on which surface the receptor is expressed. Targeting moieties include, but are not limited to, antibodies, or fragments thereof, peptides, hormones, growth factors, cytokines, and any other naturally- or non-naturally-existing ligands, which bind to cell surface receptors. The term "linker" as used herein, refers to any agent or molecule that bridges the compound or inhibitor to the targeting moiety. One of ordinary skill in the art recognizes that sites on the compounds or inhibitors, which are not necessary for the function of the compound or inhibitor, are ideal sites for attaching a linker and/or a targeting moiety, provided that the linker and/or targeting moiety, once attached to the compound or inhibitor, do(es) not interfere with the function of the compound or inhibitor.

Alternatively, the compounds of the invention described herein can be modified into a depot form, such that the manner in which the compound of the invention is released into the body to which it is administered is controlled with respect to time and location within the body (see, e.g., U.S. Patent 4,450,150). Depot forms of compounds of the invention can be, for example, an implantable composition comprising the compound and a porous material, such as a polymer, wherein the compound is encapsulated by or diffused throughout the porous material. The depot is then implanted into the desired location within the body and the compound is released from the implant at a predetermined rate by diffusing through the porous material.

In some contexts, the compounds of the invention can be advantageously administered via an implanted pump that allows intrathecal delivery. Such a delivery method is especially useful for delivery of drugs to the CNS when the drugs administered do not otherwise sufficiently penetrate the blood-brain barrier.

The compounds of the invention described herein can be administered to a cell *in vitro.* As used herein, the term *"in vitro"* means that the cell is not in a living organism. The compounds of the invention also can be administered to a cell *in vivo.* As used herein, the term *"in vivo"* means that the cell is a part of a living organism or is the living organism. Furthermore, the compounds of the invention can be administered to a host *in vivo* or *ex vivo.* The term *"ex vivo"* as used herein refers to the administration of a compound to a cell or a population of cells *in vitro,* followed by administration of the cell or population of cells to a host.

Furthermore, a compound of the invention can be administered alone, or in conjunction with an agent that enhances the efficacy of the compound of the invention. Such agents can include, for instance, any of the other active agents described herein with respect to the pharmaceutical composition, which agents can be administered in a composition separate from the composition comprising the compound of the invention.

The amount or dose of a compound of the invention should be sufficient to affect a therapeutic or prophylactic response in the host over a reasonable time frame. The appropriate dose will depend upon the nature and severity of the disease or affliction to be treated or prevented, as well as by other factors. For instance, the dose also will be determined by the existence, nature and extent of any adverse side effects that might accompany the administration of a particular compound. Ultimately, the attending physician will decide the dosage of the compound of the present invention with which to treat each individual patient, taking into consideration a variety of factors, such as age, body weight, general health, diet, sex, compound to be administered, route of administration, and the severity of the condition being treated. Typical doses might be, for example, 0.1 mg to 1 g daily, such as 5 mg to 500 mg daily.

The compounds of the invention can be administered to a cell, preferably to a cell of a host. Hosts include, for example, bacteria, yeast, fungi, plants, and mammals. Preferably, the host is a mammal. For purposes of the present invention, mammals include, but are not limited to, the order Rodentia, such as mice, and the order Logomorpha, such as rabbits. It is preferred that the mammals are from the order Carnivora, including Felines (cats) and Canines (dogs). It is more preferred that the mammals are from the order Artiodactyla, including Bovines (cows) and Swines (pigs) or of the order Perssodactyla, including Equines (horses). It is most preferred that the mammals are of the order Primates, Ceboids, or Simioids (monkeys) or of the order Anthropoids (humans and apes). An especially preferred mammal is the human. Furthermore, the host can be the unborn offspring of any of the forgoing hosts, especially mammals (e.g., humans), in which case any screening of the host or cells of the host, or administration of compounds to the host or cells of the host, can be performed in utero.

The compounds can be used for any purpose including, without limitation, the treatment, prevention, or diagnosis of a disease or condition, the screening of compounds that can be used to treat, prevent, or diagnose a disease or condition, or the research of the underlying mechanisms or causes of a disease or condition, which research can be used, for example, in the development of methods to treat, prevent, or diagnose the disease or condition. Without wishing to be bound by any particular theory, it is believed that the compounds of the invention are particularly useful with respect to diseases and conditions involving the under-expression of survival motor neuron protein (SMN), diseases and conditions that can be ameliorated by modulation of translational stop codons (e.g., UAA, UAG, UGA; both normal, naturally occurring and mutation introduced stop codons), and diseases and conditions associated with elevated glutamate levels in the central nervous system (CNS) and/or that could be ameliorated by increases in EAAT2 expression.

Preferred compounds of the invention can be used to increase SMN expression in a cell that comprises an SMN2 gene, particularly a cell that does not comprise an SMN1 gene, or that comprises a defective or deleted SMN1 gene. Thus, one aspect of the invention provides a method of increasing SMN expression in a cell comprising administering a compound of the invention to a cell comprising a nucleic acid that encodes SMN2, desirably a cell that comprises a defective or deleted SMN1 gene, whereby SMN expression is increased.

SMN1 and SMN2 refer to two different genes that each encode SMN protein, but that differ by a single base pair. The base pair change in SMN2 results in decreased expression of SMN due to an alternative splicing mechanism that results in an unstable protein that is missing exon 7. As a result, SMN2 transcripts typically are not properly expressed, and SMN protein is produced primarily by SMN1. Most patients that exhibit under-expression of SMN have a defective or mutant SMN1 gene, but an intact SMN2 gene. Without wishing to be bound by any particular theory, it is believed that the compounds of the invention increase the expression of SMN2 via a post-transcriptional mechanism, possibly by increasing translation or otherwise suppressing the effects of translational stop codons introduced by improper splicing of the SMN2 transcript.

The increase in expression of SMN after administration of a compound of the invention can be any increase as compared to the expression level of SMN in the cell in the absence of the compound of the invention. Typically, the cell will have a defective or mutant SMN1 protein and/or reduced levels of SMN protein expression in the absence of a compound of the invention. Methods for detecting and measuring increased SMN expression, particularly through expression of SMN2, are known in the art and described herein.

The cell to which the compound of the invention is administered preferably is in a host. Suitable hosts are as previously described herein. The host is desirably a mammal, especially a human. The method of this aspect of the invention is most suitable for use in conjunction with a host that is afflicted with a disease or at risk for developing a disease, such as a disease associated with the under-expression of SMN. Such diseases include, for example, spinal muscular atrophy (SMA). Preferably, one or more symptoms of the disease are prevented, reduced, or eliminated subsequent to administration of at least one compound of the invention, thereby effectively treating or preventing the disease to at least some degree. Accordingly, the invention provides a method of treating spinal muscular atrophy (SMA) in a mammal comprising administering a therapeutically effective amount of at least one compound of the invention to the mammal, whereupon SMA is treated.

In a related aspect, the invention provides a method of increasing in a cell the expression of a nucleic acid that encodes a translational stop codon. The stop codon can be a normally occurring stop codon or a stop codon introduced directly or indirectly by mutation or frameshift, particularly a nonsense stop codon. The method comprises administering a compound or pharmaceutically acceptable salt of the invention to a cell comprising a nucleic acid that encodes a translational stop codon, whereby expression of the nucleic acid is increased. Without wishing to be bound by any particular theory, it is believed that the compounds of the invention permit translational (ribosomal) read-through of stop codons, especially those introduced directly or indirectly by mutation or frameshift. According to a preferred aspect of the invention, the stop codon is one that is introduced directly or indirectly by a mutation or frameshift, and the compound of the invention permits translational read-through of the stop codon, preferably without interfering with the effect of normal stop codons (e.g., stop codons not introduced directly or indirectly by mutation or frameshift, which do not interfere with the production of a full-length protein). The compounds, thus, increase expression of the protein products of such nucleic acids. Compounds of the invention may also modulate gene expression through effects on naturally occurring stop codons.

As used herein, the term "translational stop codon introduced directly or indirectly by mutation or frameshift" means any stop codon that results in the premature or otherwise unusual termination of translation and consequential production of a truncated gene product or protein. Translational stop codons introduced directly or indirectly by mutation or frameshift include, for example, those that result in a gene product that has reduced stability or reduced activity (or no activity) as compared to the normal, full-length protein product, or results in the reduction or complete absence of a protein product. Translational stop codons introduced directly or indirectly by mutation or frameshift also include, for example, those that result in a mRNA that is a target of non-sense-mediated RNA decay. The translational stop codon can be present in the DNA or RNA of any type of cell, and can arise through any type of mutagenesis or frameshift event. For example, the nucleic acid that encodes the translational stop codon can be, without limitation, a defective SMN1 gene or a defective or normal SMN2 gene, or any transcript thereof. In particular, the alternative splicing event that occurs during SMN2 expression, which results in the deletion of SMN exon 7, also creates a nonsense stop codon. The nucleic acid comprising the stop codon can be endogenous to the cell, or a nucleic acid introduced into the cell, for example, by a virus. Without wishing to be bound by any particular theory, it is believed that a compound of the invention can increase expression of SMN by suppressing the effects of the nonsense stop codons, possibly by allowing translational read-through of the stop codon or by suppressing nonsense-mediated mRNA decay.

An appropriate screening assay can be employed to determine whether a cell or host comprises a nucleic acid that encodes a translational stop codon introduced directly or indirectly by mutation or frameshift. For instance, the DNA or RNA of a cell (e.g., a cell of a host or an organism that is pathogenic to the host) can be sequenced or subjected to Southern Blot, polymerase chain reaction (PCR), use of the Short Tandem Repeat (STR), or polymorphic length restriction fragments (RFLP) analysis to determine if a nonsense mutation is present. Alternatively, it can be determined if a cell (e.g., a cell of a host) expresses altered levels of a protein encoded by the nucleic acid using western blot or other immunoassays. Other methods of determining whether a nucleic acid that encodes a premature translational stop codon are available.

The method of this aspect of the invention is most suitable for use in conjunction with a host that is afflicted with, or at risk for developing, a disease, associated with a translational stop codon introduced by directly or indirectly mutation or frameshift. The types of diseases associated with translational stop codons introduced by mutation or frameshift, the symptoms of which can be ameliorated by the suppression of premature translation termination and/or nonsense-mediated mRNA decay, include, but are not limited to, genetic diseases, autoimmune diseases, blood diseases, collagen diseases, diabetes, neurodegenerative diseases, proliferative diseases, cardiovascular diseases, pulmonary diseases, inflammatory diseases, central nervous system diseases, infectious diseases (e.g., bacterial and viral), cancers (including tumors and other cancers), especially cancers associated with p53 mutations.

Specific examples of genetic diseases include, without limitation, SMA, amyloidosis, hemophilia, Alzheimer's disease, Tay Sachs disease, Niemann Pick disease, atherosclerosis, giantism, dwarfism, hypothyroidism, hyperthyroidism, aging, obesity, Parkinson's disease, Huntington's disease, cystic fibrosis, muscular dystrophy (e.g., Duchenne muscular dystrophy), heart disease, kidney stones, Rett syndrome, ataxia-telangiecstasia, familial hypercholesterolemia, retinitis pigmentosa, and Marfan syndrome.

Specific examples of inflammatory and autoimmune diseases include, without limitation, arthritis, rheumatoid arthritis, osteoarthritis, and graft versus host disease.

Specific examples of blood diseases include, without limitation, hemophilia, Von Willebrand disease, thalassemia (e.g., *β*-thalassemia), and kidney stones.

Specific examples of collagen diseases include, without limitation, osteogenesis imperfect, and cirrhosis.

Specific examples of central nervous system diseases include, without limitation, multiple sclerosis, muscular dystrophy (e.g., Duchenne muscular dystrophy), Alzheimer's disease, Huntington's disease, Tay Sachs disease, late infantile neuronal ceroidlipofuscinosis (LINCL), Leber's hereditary optic neuropathy, and Parkinson's disease.

Specific examples of infectious diseases include, without limitation, Human Immunodeficiency Virus infection (HIV/AIDS), viral hepatitis (e.g., hepatitis A, B, C, B with D, E, F, and/or G), Human Herpes virus (HHV) infection (including herpes zoster), Human Papilloma Virus (HPV) infection, severe acute respiratory syndrome (SARS), herpes (e.g., HSV-1, HSV-2), and Pseduomonas aeruginosa infection. Any type of HIV can be treated, but preferably an HIV-1 and/or HIV-2 infection is treated. The method also encompasses infection by an HIV group (e.g., groups M, N, and/or O), and subtype (e.g., clades A, B, C, D, E, EA, F, and/or G).

A compound of the invention can be combined with other well-known HIV therapies and prophylactic vaccines already in use. The combination of the compound of the invention can generate an additive or a synergistic effect with current treatments. The compound of the invention can be combined with other HIV and AIDS therapies and vaccines, such as highly active antiretroviral therapy (HAART), which comprises a combination of protease inhibitors and reverse transcriptase inhibitors, azidothymidine (AZT), structured treatment interruptions of HAART, cytokine immune enhancement therapy (e.g., interleukin (IL)-2, IL-12, CD40L + IL-12, IL-7, HIV protease inhibitors (e.g., ritonavir, indinavir, and nelfinavir, etc.), and interferons (IFNs), cell replacement therapy, recombinant viral vector vaccines, DNA vaccines, inactivated virus preparations, immunosuppressive agents, such as Cyclosporin A, and cyanovirin therapy (see, e.g., U.S. Patent No. 6,015,876 and International Patent Application Publication No. WO 03/072594). Such therapies can be administered in the manner already in use for the known treatment providing a therapeutic or prophylactic effect (see, e.g., Silvestri et al. Immune Intervention in AIDS. In: Immunology of Infectious Disease, H.E. Kauffman, A. Sher, and R. Ahmed eds., ASM Press, Washington DC (2002)).

A compound of the invention can be used in combination with one or more other antiviral agents, such as VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals), abacavir, aciclovir, acyclovir, adefovir, amantadine, amprenavir, arbidol atazanavir, atripla, brivudine, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, ganciclovir, ibacitabine, imunovir, idoxuridine, imiquimod, indinavir, inosine, lamivudine, lopinavir, loviride, MK-0518, maraviroc, moroxydine, nelfinavir, nevirapine, nexavir, oseltamivir, penciclovir, peramivir, pleconaril, podophyllotoxin, ribavirin, rimantadine, ritonavir, saquinavir, stavudine, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine. The combination of the compound of the invention and one or more antiviral agents can generate an additive or a synergistic effect with current treatments.

Specific examples of cancers include cancer of the head and neck, eye, skin, mouth, throat, esophagus, chest, bone, lung, colon, sigmoid, rectum, stomach, prostate, breast, ovaries, kidney, liver, pancreas, brain, intestine, heart or adrenals. More particularly, cancers include solid tumor, sarcoma, carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendothelio sarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, Kaposi's sarcoma, pinealoma, hemangioblastoma, acoustic neuroma, oligodendroglioma, menangioma, melanoma, neuroblastoma, retinoblastoma, a blood-born tumor, acute lymphoblastic leukemia, acute lymphoblastic B-cell leukemia, acute lymphoblastic T-cell leukemia, acute myeloblastic leukemia, acute promyelocytic leukemia, acute monoblastic leukemia, acute erythroleukemic leukemia, acute megakaryoblastic leukemia, acute myelomonocytic leukemia, acutenonlymphocyctic leukemia, acute undifferentiated leukemia, chronic myelocytic leukemia, chronic lymphocytic leukemia, hairy cell leukemia, or multiple myeloma. See, e.g., Harrison's Principles of Internal Medicine, Eugene Braunwald et al., eds., pp. 491-762 (15th ed. 2001).

Diseases, such as cancers, associated with p53 mutations that result in a translational stop codon include, but are not limited to, the diseases and mutations described in Masuda et al., Tokai J Exp Clin Med., 25 (2): 69-77 (2000); Oh et al., Mol Cells, 10 (3): 275-80 (2000); Li et al., Lab Invest., 80 (4): 493-9 (2000); Yang et al., Zhonghua Zhong Liu Za Zhi, 21 (2): 114-8 (1999); Finkelstein et al., Mol Diagn., 3 (1): 37-41 (1998); Kajiyama et al., Dis Esophagus., 11 (4): 279-83 (1998); Kawamura et al., Leuk Res., 23 (2): 115-26 (1999); Radig et al., Hum Pathol., 29 (11): 1310-6 (1998); Schuyer et al., Int J Cancer, 76 (3): 299-303 (1998); Wang-Gohrke et al., Oncol Rep., 5 (1): 65-8 (1998); Fulop et al., J Reprod Med., 43 (2): 119-27 (1998); Ninomiya et al., J Dermatol Sci., 14 (3): 173-8 (1997); Hsieh et al., Cancer Lett., 100 (1- 2): 107-13 (1996); Rall et al., Pancreas., 12 (1): 0-7 (1996); Fukutomi et al., NipponRinsho, 53 (11): 2764-8 (1995); Frebourg et al., Am JHum Genet., 56 (3): 608-15 (1995); Dove et al., Cancer Surv., 25: 335-55 (1995); Adamson et al., Br J Haematol., 89 (1): 61-6 (1995); Grayson et al., Am J Pediatr Hematol Oncol., 16 (4): 341-7 (1994); Lepelley etal., Leukemia, 8 (8): 1342-9 (1994); McIntyre et al., J Clin Oncol., 12 (5): 925-30 (1994); Horio et al., Oncogene, 9 (4): 1231-5 (1994); Nakamura et al., Jpn J Cancer Res., 83 (12): 1293-8 (1992); Davidoff et al., Oncogene, 7 (1): 127-33 (1992); and Ishioka etal., Biochem Biophys Res Commun., 177 (3): 901-6 (1991). Each of these references is incorporated herein by reference.

Preferably, one or more symptoms of the disease are prevented, reduced, or eliminated subsequent to administration of the compound of the invention, thereby effectively treating or preventing the disease to at least some degree. Accordingly, the method of the invention can be used to treat or prevent such a disease.

In yet another related aspect, the invention provides a method of increasing the expression of excitatory amino acid transporter (EAAT2) in a cell comprising administering a compound of the invention to a cell comprising a nucleic acid that encodes EAAT2, whereby expression of EAAT2 is increased. Certain diseases (e.g., multiple sclerosis, muscular dystrophy, Parkinson's disease, Alzheimer's disease, amyotrophic lateral sclerosis (ALS, also known as Lou Gehrig's disease), and epilepsy) and other conditions, such as stroke or other trauma to the CNS, are associated with elevated glutamate levels in the CNS that can be toxic, leading to brain damage or even death. Cellular uptake of glutamate via the EAAT2 transporter is responsible, at least in part, for maintaining appropriately low glutamate levels in the CNS. Without wishing to be bound by any particular theory, it is believed that the compounds of the invention can activate or enhance EAAT2 expression, thereby advantageously lowering glutamate levels in the CNS. Methods of detecting and measuring increased EAAT2 expression are known in the art and described, for instance, in Rothstein et al., Nature, 43 (3): 73-7 (2005).

The cell to which the compound of the invention is administered preferably is in a host. Suitable hosts are as previously described herein. The host is desirably a mammal, especially a human. The method of this aspect of the invention is most suitable for use in conjunction with a host that is afflicted with a disease or condition, or at risk for developing a disease or condition, associated with decreased expression of EAAT2 or elevated glutamate levels in the CNS. Such diseases and conditions include, for example, SMA, stroke, multiple sclerosis, Parkinson's disease, Alzheimer's disease, Huntington's disease, amyotrophic lateral sclerosis (ALS), and epilepsy. Preferably, one or more symptoms of the disease or condition are prevented, reduced, or eliminated subsequent to administration of the compound of the invention, thereby effectively treating or preventing the disease or condition to at least some degree. Accordingly, the invention provides a method of treating or preventing a neurological disorder selected from the group consisting of SMA, stroke, multiple sclerosis, Parkinson's disease, Alzheimer's disease, Huntington's disease, amyotrophic lateral sclerosis (ALS), and epilepsy in a mammal comprising administering a therapeutically effective amount of at least one compound of the invention to the mammal, whereupon the neurological disorder is treated or prevented. The treatment of the neurological disorder can be effected, for example, by increasing the expression of EAAT2.

In addition to the foregoing characteristics, preferred compounds of the invention are able to penetrate the blood-brain barrier so as to accumulate in therapeutically effective amounts, and do not have significant cyclooxygenase (Cox) inhibitory activity (e.g., have less than toxic levels of Cox inhibitory activity).

The following examples further illustrate the invention but, of course, should not be construed as in any way limiting its scope.

### EXAMPLE 1

This example illustrates a preparation of 6-methoxy-2-(1-methyl-1H-indol-5-yl)isoindolin-1-one in an embodiment of the invention.

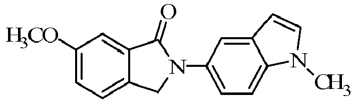

### Step A: Synthesis of ethyl 2-methyl-5-nitrobenzoate as an intermediate

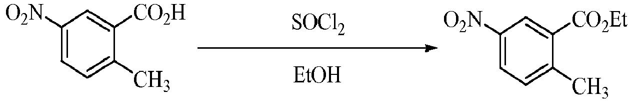

A mixture of 2-methyl-5-nitrobenzoic acid (10.0 g, 55.2 mmol) in ethanol (200 ml) was cooled to 0 °C with an ice bath, and thionyl chloride (16.0 mL, 220 mmol) was added dropwise over 10 min. After this time, the mixture was warmed to room temperature for 1 h, then transferred to an oil bath and heated to reflux overnight. The mixture was then cooled to room temperature and concentrated under reduced pressure. Purification by flash chromatography (silica, 9:1 hexanes/ethyl acetate) afforded ethyl 2-methyl-5-nitrobenzoate (12.4 g, quant.) as a pale yellow oil: ¹H NMR (300 MHz, CDCl₃) δ 8.76 (d, *J*= 2.5 Hz, 1H), 8.24 (dd, *J* = 8.5, 2.5 Hz, 1H), 7.43 (d, *J* = 8.4 Hz, 1H), 4.42 (q, *J* = 7.2 Hz, 2H), 2.72 (s, 3H), 1.44 (t, *J*= 7.1 Hz, 3H).

### Step B: Synthesis of ethyl 5-amino-2-methylbenzoate as an intermediate

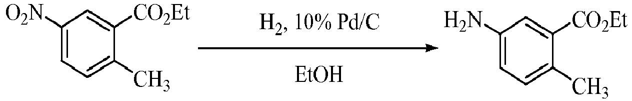

A mixture of ethyl 2-methyl-5-nitrobenzoate (11.5 g, 54 mmol) and 10% Pd/C (1.2 g) in ethanol (200 mL) was shaken under an atmosphere of hydrogen at 40 psi for 18 h. After this time, the mixture was filtered through diatomaceous earth and concentrated under reduced pressure to provide ethyl 5-amino-2-methylbenzoate (9.77 g, 98%) as an off-white solid: ¹H NMR (300 MHz, CDCl₃) δ 7.25 (d, *J*= 2.6 Hz, 1H), 7.02 (d, *J*= 8.1 Hz, 1H), 6.74 (d, *J*= 8.1 Hz, 1H), 4.33 (q, *J*= 7.2 Hz, 2H), 2.46 (s, 3H), 1.38 (t, *J*= 7.1 Hz, 3H).

### Step C: Preparation of ethyl 5-hydroxy-2-methylbenzoate as an intermediate

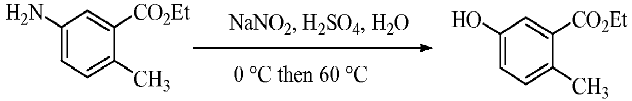

A mixture of ethyl 5-amino-2-methylbenzoate (14.4 g, 80.3 mmol) in aqueous 5% sulfuric acid (300 mL) was cooled with an ice-bath for 15 min. Sodium nitrite (6.09 g, 88.3 mmol) was then added dropwise in water (50 mL), and the resulting mixture stirred for at 0 °C for 30 min. After this time, the resulting mixture was heated at 60 °C for 18 h. The reaction mixture was then cooled to room temperature and diluted with ethyl acetate (150 mL). The organic layer was separated, dried over sodium sulfate, filtered, and concentrated under reduced pressure. Purification by flash chromatography (silica, gradient 0-40%, hexanes/ethyl acetate) afforded 12.48 g (86%) of ethyl 5-hydroxy-2-methylbenzoate as red-brown solid: ¹H NMR (300 MHz, CDCl₃) δ 7.44 (d, *J*= 2.8 Hz, 1H), 7.10 (d, *J*= 8.3 Hz, 1H), 6.91 (dd, *J* = 8.3, 2.8 Hz, 1H), 5.44 (s, 1H), 4.35 (q, *J* = 7.1 Hz, 2H), 2.50 (s, 3H), 1.38 (t, *J*= 7.1 Hz, 3H).

### Step D: Synthesis of ethyl 5-methoxy-2-methylbenzoate as an intermediate

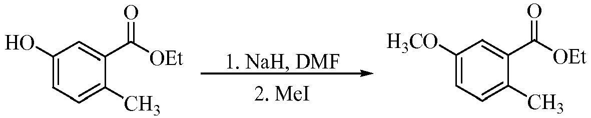

A mixture of sodium hydride (60% in mineral oil, 1.50 g, 37.4 mmol) in *N,N-*dimethylformamide (50 mL) was stirred for 10 min under a nitrogen atmosphere prior to dropwise addition of ethyl 5-hydroxy-2-methylbenzoate (5.18 g, 28.7 mmol) as a solution in *N,N*-dimethylformamide (100 mL). After this time, the mixture was allowed to stir for 3 h at room temperature before iodomethane (2.69 mL, 43.1 mmol) was added and the mixture stirred overnight at room temperature. After this time, the mixture was poured into water (200 mL) and extracted with ethyl acetate (200 mL). The organics were then washed with 5% aqueous lithium chloride (200 mL), water (200 mL) and brine (100 mL), dried over magnesium sulfate and concentrated under reduced pressure. Purification by flash chromatography (silica, 1:19 ethyl acetate/hexanes) afforded ethyl 5-methoxy-2-methylbenzoate (4.18 g, 75%) as a clear oil: ¹H NMR (300 MHz, CDCl₃) δ 7.44 (d, *J*= 2.7 Hz, 1H), 7.14 (d, *J* = 8.4 Hz, 1H), 6.95 (dd, *J* = 2.7, 3.0, 8.4, 8.7 Hz, 1H), 4.35 (q, *J* = 6.9, 7.2 Hz, 2H), 3.82 (s, 3H), 2.51 (s, 3H), 1.39 (t, *J*= 7.2 Hz, 3H).

### Step E: Synthesis of ethyl 2-(bromomethyl)-5-methoxybenzoate as an intermediate

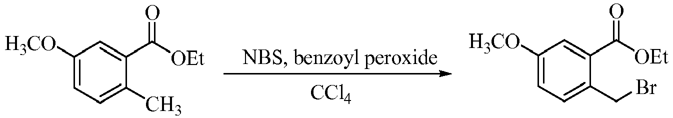

A mixture of ethyl 5-methoxy-2-methylbenzoate (4.18 g, 21.5 mmol) in carbon tetrachloride (120 mL) was heated to 78 °C in an oil bath prior to portionwise addition of a mixture of *N*-bromosuccinimide (4.21 g, 23.6 mmol) and benzoyl peroxide (0.52 g, 2.15 mmol) over 4 h. The mixture was then stirred overnight at 78 °C. After this time, the reaction mixture was cooled to room temperature and the solids removed by filtration. The filtrate was then concentrated under reduced pressure and purification by flash chromatography (silica, 1:49 ethyl acetate/hexanes) afforded ethyl 2-(bromomethyl)-5-methoxybenzoate (2.97 g, 51%) as clear oil: ¹H NMR (500 MHz, CDCl₃) δ 7.48 (d, *J*= 2.5 Hz, 1H), 7.36 (d, *J*= 8.5 Hz, 1H), 7.00 (dd, *J* = 2.5, 8.5 Hz, 1H), 4.92 (s, 2H), 4.41 (q, *J* = 7.0, 7.5 Hz, 2H), 3.84(s, 3H), 1.42 (t, *J* = 7.0, 7.5 Hz, 3H).

### Step F: Synthesis of 1-methyl-5-nitro-1H-indole as an intermediate

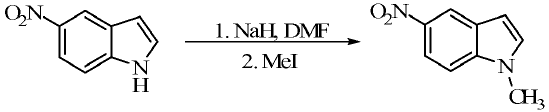

To a mixture of 5-nitro-1H-indole (5.0 g, 30.8 mmol) in *N,N*-dimethylformamide (100.0 mL) was added sodium hydride (60% in mineral oil, 1.35 g, 33.9 mmol), and the mixture stirred for 1 h at room temperature. After this time, iodomethane (2.11 mL, 33.9 mmol) was added, and the mixture was stirred at room temperature overnight. After this time, saturated aqueous ammonium chloride (100 mL) was added and the organics extracted with ethyl acetate (150 mL). The organics were then washed with 5% aqueous lithium chloride (200 mL), water (200 mL) and brine (1000 mL), dried over magnesium sulfate and concentrated under reduced pressure to afford 1-methyl-5-nitro-1H-indole (5.42 g, 100%) as an orange/brown solid: ¹H NMR (500 MHz, CDCl₃) δ 8.59 (d, *J*= 2.0 Hz, 1H), 8.13 (dd, *J*= 2.0, 9.0 Hz, 1H), 7.34 (d, *J* = 9.0 Hz, 1H), 7.20 (d, *J* = 3.0 Hz, 1H), 6.67 (dd, *J* = 0.5, 1.0, 3.0, 3.5 Hz, 1H), 3.86 (s, 3H).

### Step G: Synthesis of 1-methyl-1H-indol-5-amine as an intermediate

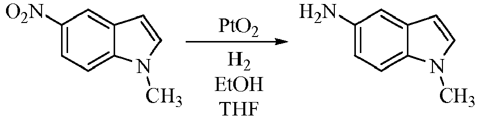

A mixture of 1-methyl-5-nitro-1H-indole (2.00 g, 11.3 mmol) and platinum (IV) oxide (0.20 g, 10% by weight) in a mixture of ethanol (20 mL) and tetrahydrofuran (20 mL) was shaken under an atmosphere of hydrogen at 40 psi for 4 h. After this time, the mixture was filtered through diatomaceous earth and concentrated under reduced pressure. Purification by flash chromatography (silica, 1:3, ethyl acetate/hexanes) afforded 1-methyl-1H-indol-5-amine (1.28g, 77%) as a red oil: ¹H NMR (500 MHz, CDCl₃) δ 7.12 (d, *J*= 8.5 Hz, 1H), 6.95 (d, *J* = 3.0 Hz, 1H), 6.92 (d, *J* = 2.0 Hz, 1H), 6.69 (dd, *J* = 2.0, 8.5 Hz, 1H), 6.28 (d, *J*= 3.0 Hz, 1H), 3.72 (s, 3H), 3.47 (bs, 2H).

### Step H: Synthesis of 6-methoxy-2-(1-methyl-1H-indol-5-yl)isoindolin-1-one

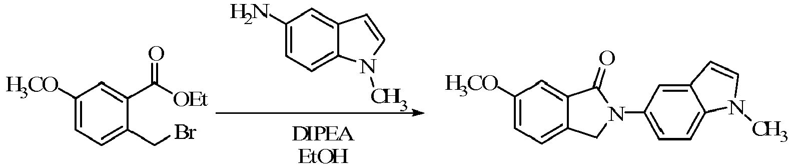

A mixture of 1-methyl-1H-indol-5-amine (0.161 g, 1.10 mmol), *N,N-*diisopropylethyl amine (0.191 mL, 1.10 mmol) in ethanol (30 mL) was stirred for 10 min in a sealed tube at room temperature. After this time, ethyl 2-(bromomethyl)-5-methoxybenzoate (0.200 g, 0.73 mmol) was added portionwise over 3 h and the mixture was then heated to 100 °C overnight. The mixture was then cooled to room temperature, LiOH•H₂O was added as a solution in water (2 mL), and the mixture stirred an additional night at room temperature. The mixture was then concentrated under reduced pressure and purification by flash chromatography (silica, 1:5 ethyl acetate/hexanes) afforded 6-methoxy-2-(1-methyl-1H-indol-5-yl)isoindolin-1-one (0.068 g, 32%) as a brown/red solid: mp 193-197 °C; ¹H NMR (500 MHz, CDCl₃) δ 7.89 (d, *J*= 2.0 Hz, 1H), 7.71 (dd, *J*= 2.0, 2.5, 8.5, 9.0 Hz, 1H), 7.43 (d, *J*= 2.5 Hz, 1H), 7.40 (d, *J*= 8.0 Hz, 1H), 7.36 (d, *J*= 8.5 Hz, 1H), 7.14 (dd, *J*= 2.5, 8.0 Hz, 1H), 7.08 (d, *J*= 3.0 Hz, 1H), 6.50 (d, *J*= 3.0 Hz, 1H), 4.85 (s, 2H), 3.90 (s, 3H), 3.81 (s, 3H); APCI MS *m*/*z* 293 [M + H]⁺.

### EXAMPLE 2

This example illustrates a preparation of 6-(difluoromethoxy)-2-(1-methyl-1*H-*indol-5-yl)isoindolin-1-one in an embodiment of the invention.

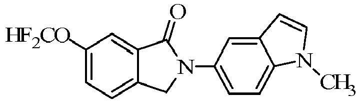

### Step A: Preparation of ethyl 5-(difluoromethoxy)-2-methylbenzoate as an intermediate

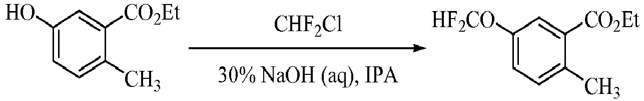

A mixture of ethyl 5-hydroxy-2-methylbenzoate (3.29 g, 18.2 mmol) in aqueous 30% aqueous sodium hydroxide (20 mL) and isopropyl alcohol (20 mL) was charged with 20 psi of chlorodifluoromethane in a high pressure flask, and stirred at room temperature for 10 min. The resulting mixture was heated at 50 °C for 0.5 h. After this time, the reaction mixture was cooled to room temperature and diluted with dichloromethane (50 mL) and water (200 mL). The organic layer was separated, dried over sodium sulfate, filtered, and concentrated under reduced pressure. Purification by flash chromatography (silica, gradient 0-7%, hexanes/ethyl acetate) afforded 2.50 g (59%) of ethyl 5-(difluoromethoxy)-2-methylbenzoate as a colorless oil: ¹H NMR (500 MHz, CDCl₃) δ 7.67 (d, J = 2.5 Hz, 1H), 7.26-7.22 (m, 1H), 7.18-7.16 (m, 1H), 6.50 (t, J = 73.6 Hz, 1H), 4.37 (q, J = 7.0 Hz, 2H), 2.57 (s, 3H), 1.40 (t, J = 7.0 Hz, 3H); ESI MS m/z 231 [M + H]+.

### Step B: Synthesis of ethyl 2-(bromomethyl)-5-(difluoromethoxy)benzoatebenzoate as an intermediate

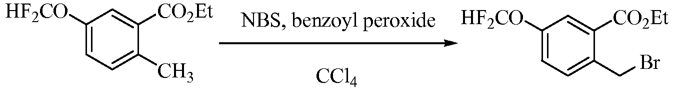

A mixture of ethyl 5-(difluoromethoxy)-2-methylbenzoate (4.25 g, 18.5 mmol), *N*-bromosuccinimide (3.45 g, 19.4 mmol) and benzoyl peroxide (0.45 g, 1.85 mmol) in carbon tetrachloride (40 ml) was heated to 70 °C in an oil bath for 3.5 h. The mixture was cooled to room temperature, and the crude mixture was triturated with 10% ethyl acetate in hexanes. The solids were removed by filtration. The filtrate was then concentrated under reduced pressure and purification by flash chromatography (silica, gradient 1-6%, ethyl acetate/hexanes) afforded ethyl 2-(bromomethyl)-5-(difluoromethoxy)benzoatebenzoate (4.56 g, 80%) as a clear oil: ¹H NMR (300 MHz, CDCl₃) 7.71 (d, *J*= 2.7 Hz, 1H), 7.47 (d, *J* = 8.5 Hz, 1H), 7.26-7.24 (m, 1H), 6.47 (t, *J* = 73.1 Hz, 1H), 4.93 (s, 2H), 4.42 (q, *J* = 7.1 Hz, 2H), 1.42 (t, *J* = 7.1 Hz, 3H).

### Step C: Preparation of 6-(difluoromethoxy)-2-(1-methyl-1H-indol-5-yl)isoindolin-1-one

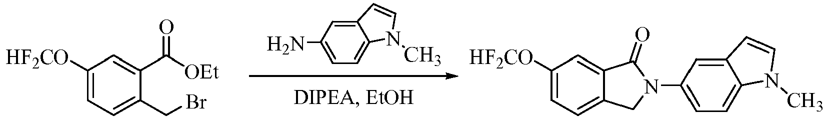

A mixture of ethyl 2-(bromomethyl)-5-(difluoromethoxy)benzoate (0.250 g, 0.80 mmol), 1-methyl-1*H-*indol-5-amine (0.142 g, 0.97 mmol) and *N,N-*diisopropylethylamine (0.170 mL, 0.97 mmol) in ethanol (10 mL) was heated in a sealed tube at 110 °C overnight. The mixture was cooled to room temperature, and then cooled for 30 min. in an ice-bath. The resulting solid was collected by filtration to afford 6-(difluoromethoxy)-2-(1-methyl-1*H-*indol-5-yl)isoindolin-1-one (0.055 g, 21%) as an off-white solid: mp 180-182 °C; ¹H NMR (500 MHz, CDCl₃) δ 7.89 (d, *J*= 2.0 Hz, 1H), 7.70-7.68 (m, 2H), 7.51 (d, *J*= 8.2 Hz, 1H), 7.38-7.34 (m, 2H), 7.10 (d, J= 3.0 Hz, 1H), 6.60 (t, *J*= 73.3 Hz, 1H), 6.51 (d, *J*= 3.0 Hz, 1H), 4.90 (s, 2H), 3.82 (s, 3H); ESI MS *m*/*z* 329 [M + H]⁺.

### EXAMPLE 3

This example illustrates a preparation of 6-(bromodifluoromethoxy)-2-(1-methyl-1*H*-indol-5-yl)isoindolin1-one in an embodiment of the invention.

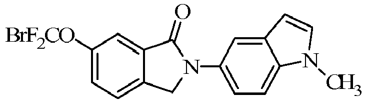

### Step A: Synthesis of ethyl 5-(bromodifluoromethoxy)-2-methylbenzoate as an intermediate

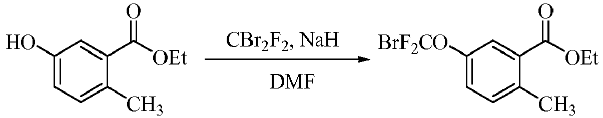

A solution of ethyl 5-hydroxy-2-methylbenzoate (0.500 g, 2.77 mmol) in *N,N-*dimethylformamide (5 mL) was added to an ice-cold stirred solution of sodium hydride (0.144 g, 3.60 mmol, 60% dispersion in mineral oil) in *N,N-*dimethylformamide (5 mL). The mixture was stirred for 5 min and dibromodifluoromethane (1.50 mL, 16.6 mmol) was added. The resulting mixture was allowed to warm to room temperature and stirred for 18 h. After this time, the mixture was poured onto ice-water and extracted with ethyl acetate. The resulting organic layer was dried over sodium sulfate, filtered, and concentrated under reduced pressure. Purification by flash chromatography (silica, gradient 1-5%, ethyl acetate/hexanes) afforded ethyl 5-(bromodifluoromethoxy)-2-methylbenzoate (0.094 g, 10%) as a clear oil: ¹H NMR (300 MHz, CDCl₃) δ 7.79 (s, 1H), 7.28-7.26 (m, 2H), 4.41-4.34 (m, 2H), 2.61 (s, 3H), 1.43-1.37 (m, 3H); ESI MS *m*/*z* 310 [M + H]⁺.

### Step B: Synthesis of ethyl 5-(bromodifluoromethoxy)-2-(bromomethyl)benzoate as an intermediate

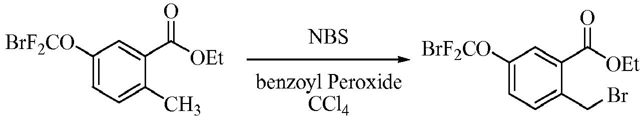

A mixture of ethyl 5-(bromodifluoromethoxy)-2-methylbenzoate (0.518 g, 1.70 mmol), *N*-bromosuccinimide (0.325 g, 1.83 mmol) and benzoyl peroxide (0.040 g, 0.17 mmol) in carbon tetrachloride (20 ml) was heated to 70 °C in an oil bath for 18 h. After this time, the mixture was cooled to room temperature and the solids removed by filtration. The filtrate was then concentrated under reduced pressure and purified by flash chromatography (silica, gradient 1-2%, ethyl acetate/hexanes) to afford ethyl 5-(bromodifluoromethoxy)-2-(bromomethyl)benzoate (0.690 g, 99%) as a yellow oil: ¹H NMR (300 MHz, CDCl₃) δ 7.84-7.83 (m, 1H), 7.54-7.51 (m, 1H), 7.40-7.36 (m, 1H) 4.95 (s, 2H), 4.47-4.34 (m, 2H), 1.46-1.38 (m, 3H).

### Step C: Preparation of 6-(bromodifluoromethoxy)-2-(1-methyl-1H-indol-5-yl)isoindolin1-one

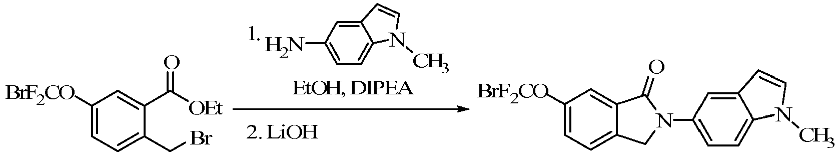

A mixture of ethyl 5-(bromodifluoromethoxy)-2-(bromomethyl)benzoate (0.500 g, 1.20 mmol), 1-methyl-1*H-*indol-5-amine (0.205 g, 1.40 mmol) *and N,N-*diisopropylethylamine (0.247 mL, 1.40 mmol) in ethanol (20 mL) was heated in a sealed tube at 110 °C overnight. The mixture was cooled to room temperature and lithium hydroxide (0.151 g, 3.60 mmol) in water (2 mL) was added. The resulting mixture was stirred for 3 h and then concentrated under reduced pressure. Purification by flash chromatography (silica, gradient 1-2%, methanol/methylene chloride) afforded 6-(bromodifluoromethoxy)-2-(1-methyl-1*H-*indol-5-yl)isoindolinl-one (0.106 g, 20%) as an off-white solid: mp 185-186 °C; ¹H NMR (300 MHz, CDCl₃) δ 7.88 (d, *J*= 2.0 Hz, 1H), 7.84 (br s, 1H), 7.69 (dd, *J*= 2.1, 8.8 Hz, 1H), 7.57 (d, *J*= 8.3 Hz, 1H),7.46-7.43 (m, 1H), 7.37 (d, *J*= 8.8 Hz, 1H), 7.10 (d, *J*= 3.1 Hz, 1H), 6.52-6.51 (m, 1H), 4.92 (s, 2H), 3.82 (s, 3H); ESI MS *m*/*z* 407 [M + H]⁺.

### EXAMPLE 4

This example illustrates a preparation of 6-(2-chloro-1,1,2-trifluoroethoxy)-2-(1-methyl-1*H-*indol-5-yl)isoindolin-1-one in an embodiment of the invention.

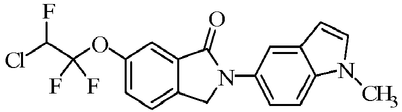

### Step A: Synthesis of ethyl 5-(2-chloro-1,1,2-trifluoroethoxy)-2-methylbenzoate as an intermediate

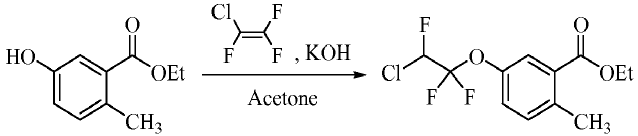

A mixture of ethyl 5-hydroxy-2-methylbenzoate (0.500 g, 2.77 mmol) and potassium hydroxide (0.077 g, 1.38 mmol) in acetone (5 mL) was stirred for 5 min in a pressure vessel prior to charging it with chlorotrifluoroethylene (20 psi). The resulting mixture was stirred at room temperature for 2 h. After this time, the mixture was poured onto ice-water and extracted with ethyl acetate. The resulting organic layer was dried over sodium sulfate, filtered, and concentrated under reduced pressure. Purification by flash chromatography (silica, gradient 1-10%, ethyl acetate/hexanes) afforded ethyl 5-(2-chloro-1,1,2-trifluoroethoxy)-2-methylbenzoate (0.595 g, 72%) as a clear oil: ¹H NMR (500 MHz, CDCl₃) δ 7.75 (s, 1H), 7.26-7.25 (m, 2H), 6.32-6.21 (m, 1H), 4.37 (q, *J*= 7.2 Hz, 2H), 2.59 (s, 3H), 1.40 (t, *J*= 7.1 Hz, 3H); ESI MS *m*/*z* 297 [M + H]⁺.

### Step B: Synthesis of ethyl 2-(bromomethyl)-5-(2-chloro-1,1,2-trifluoroethoxy)benzoate as an intermediate

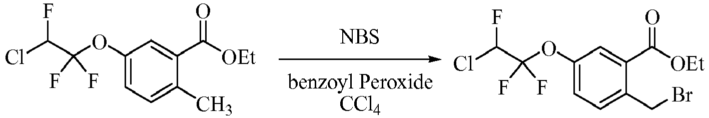

A mixture of ethyl 5-(2-chloro-1,1,2-trifluoroethoxy)-2-methylbenzoate (0.595 g, 2.01 mmol), *N*-bromosuccinimide (0.394 g, 2.20 mmol) and benzoyl peroxide (0.050 g, 0.20 mmol) in carbon tetrachloride (20 ml) was heated to 70 °C in an oil bath for 18 h. After this time, the mixture was cooled to room temperature and the solids removed by filtration. The filtrate was then concentrated under reduced pressure and purified by flash chromatography (silica, gradient 1-5%, ethyl acetate/hexanes) to afford ethyl 2-(bromomethyl)-5-(2-chloro-1,1,2-trifluoroethoxy)benzoate (0.619 g, 82%) as a clear oil: ¹H NMR (300 MHz, CDCl₃) δ 7.81-7.80 (m, 1H), 7.52-7.48 (m, 1H), 7.37-7.34 (m, 1H) 6.38-6.19 (m, 1H), 4.94 (s, 2H), 4.46-4.36 (m, 2H), 1.54-1.38 (m, 3H).

### Step C: Preparation of 6-(2-chloro-1,1,2-trifluoroethoxy)-2-(1-methyl-1H-indol-5-yl)isoindolin-1-one

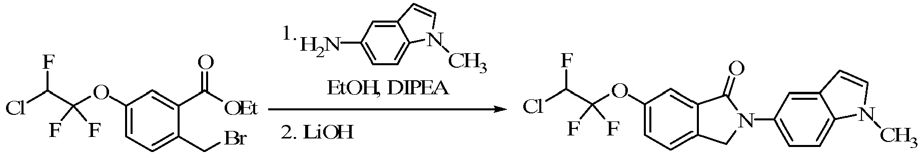

A mixture of ethyl 2-(bromomethyl)-5-(2-chloro-1,1,2-trifluoroethoxy)benzoate (0.250 g, 0.600 mmol), 1-methyl-1*H*-indol-5-amine (0.098 g, 0.66 mmol) and *N,N* diisopropylethylamine (0.117 mL, 0.66 mmol) in ethanol (10 mL) was heated in a sealed tube at 110 °C overnight. After this time, the mixture was cooled to room temperature and lithium hydroxide (0.076 g, 1.80 mmol) in water (2 mL) was added. The resulting mixture was stirred for 3 h and then concentrated under reduced pressure. Purification by flash chromatography (silica, gradient 1-5%,methanol/methylene chloride) afforded 6-(2-chloro-1,1,2-trifluoroethoxy)-2-(1-methyl-1H-indol-5-yl)isoindolin-1-one (0.110 g, 42%) as an off-white solid: mp 200-205 °C; ¹H NMR (500 MHz, DMSO-*d*₆) δ 7.95 (d, *J*= 1.8 Hz, 1H), 7.79-7.77 (m, 1H), 7.66 (dd, *J*= 2.0, 8.8 Hz, 1H), 7.57-7.56 (m, 2H), 7.50 (d, *J*= 8.9 Hz, 1H), 7.43-7.32 (m, 2H), 6.47-6.46 (m, 1H), 5.08 (s, 2H), 3.81 (s, 3H); ESI MS *m*/*z* 395 [M + H]⁺.

### EXAMPLE 5

This example illustrates a preparation of 6-hydroxy-2-(1-methyl-1*H-*indol-5-yl)isoindolin-1-one in an embodiment of the invention.

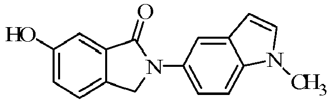

### Step A: Synthesis of ethyl 5-(tert-butyldimethylsilyloxy)-2-methylbenzoate as an intermediate

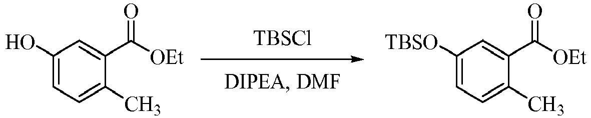

A mixture of ethyl 5-hydroxy-2-methylbenzoate (0.500 g, 2.70 mmol) and *N,N-*diisopropylethylamine (0.725 mL, 4.16 mmol) in *N,N-*dimethylformamide (5 mL) was cooled to 0 °C then *tert*-butyldimethylsilyl chloride (0.502 g, 3.3 mmol) was added. The resulting mixture was allowed to warm to room temperature and stirred for 18 h. After this time, the mixture was partitioned with ethyl acetate and water. The organic layer was collected, dried over sodium sulfate, and concentrated under reduced pressure. Purification by flash chromatography (silica, gradient 1-10%, ethyl acetate/hexanes) afforded ethyl 5-(*tert-*butyldimethylsilyloxy)-2-methylbenzoate (0.570 g, 70%) as a clear oil: ¹H NMR (300 MHz, CDCl₃) δ 7.37 (d, *J*= 2.7 Hz, 1H), 7.08 (d, *J*= 8.3 Hz, 1H), 6.87 (dd, *J*= 8.3, 2.7 Hz, 1H), 4.34 (q, *J* = 7.1 Hz, 2H), 2.50 (s, 3H), 1.39 (t, *J* = 7.1 Hz, 3H), 0.98 (s, 9H), 0.19 (s, 6H); ESI MS *m*/*z* 295 [M + H]⁺.

### Step B: Synthesis of ethyl 2-(bromomethyl)-5-(tert-butyldimethylsilyloxy)benzoate as an intermediate

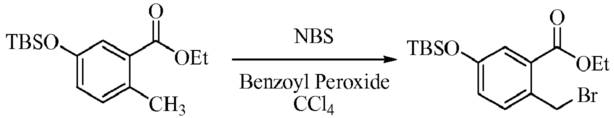

A mixture of ethyl 5-(*tert*-butyldimethylsilyloxy)-2-methylbenzoate (0.570 g, 1.90 mmol), *N*-bromosuccinimide (0.380 g, 2.10 mmol) and benzoyl peroxide (0.046 g, 0.190 mmol) in carbon tetrachloride (10 ml) was heated to 70 °C in an oil bath for 3.5 h. After this time, the mixture was cooled to room temperature and the solids removed by filtration. The filtrate was then concentrated under reduced pressure and purified by flash chromatography (silica, gradient 1-20%, ethyl acetate/hexanes) to afford ethyl 2-(bromomethyl)-5-(*tert-*butyldimethylsilyloxy)benzoate (0.660 g, 91%) as a clear oil: ¹H NMR (500 MHz, CDCl₃) δ 7.41 (d, *J*= 2.7 Hz, 1H), 7.31 (d, *J*= 8.4 Hz, 1H), 6.93 (dd, *J*= 8.4, 2.7 Hz, 1H), 5.30 (s, 2H), 4.40 (q, *J*= 7.2 Hz, 2H), 1.43 (t, *J*= 7.2 Hz, 3H), 0.98 (s, 9H), 0.21 (s, 6H).

### Step C: Preparation of 6-hydroxy-2-(1-methyl-1H-indol-5-yl)isoindolin-1-one

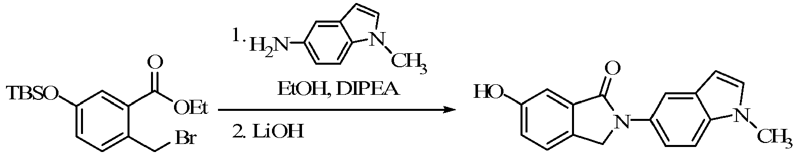

A mixture of ethyl 2-(bromomethyl)-5-(*tert*-butyldimethylsilyloxy)benzoate (1.75 g, 4.68 mmol), 1-methyl-1*H-*indol-5-amine (0.822 g, 5.60 mmol) and *N,N-*diisopropylethylamine (0.975 mL, 5.60 mmol) in ethanol (80 mL) was heated in a sealed tube at 80 °C overnight. The mixture was cooled to room temperature and lithium hydroxide (0.589 g, 14.0 mmol) in water (2 mL) was added. The resulting mixture was stirred for 3 h and then concentrated under reduced pressure. Purification by flash chromatography (silica, gradient 1-5%,methanol/methylene chloride) afforded 6-hydroxy-2-(1-methyl-1*H* indol-5-yl)isoindolin-1-one (0.360 g, 28% over 2 steps) as a brown solid: mp 230-235 °C; ¹H NMR (300 MHz, DMSO-*d*₆) δ 7.92 (d, *J*= 2.0 Hz, 1H), 7.68-7.64 (m, 1H), 7.49-7.35 (m, 3H), 7.09-7.03 (m, 2H), 6.44 (d, *J*= 3.0 Hz, 1H), 4.90 (s, 2H), 3.80 (s, 3H); ESI MS *m*/*z* 279 [M + H]⁺.

### EXAMPLE 6

This example illustrates a preparation of 6-ethoxy-2-(1-methyl-1*H-*indol-5-yl)isoindolin-1-one in an embodiment of the invention.

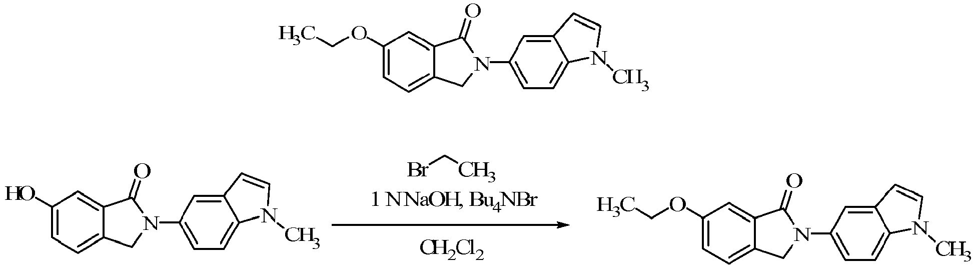

A mixture of 6-hydroxy-2-(1-methyl-1*H-*indol-5-yl)isoindolin-1-one (0.095 g, 0.340 mmol), tetrabutylammonium bromide (0.011 g, 0.034 mmol) and ethyl bromide (0.051 mL, 0.680 mmol) in methylene chloride (1 mL) and 1 N sodium hydroxide (1 mL) was vigorously stirred at room temperature overnight. After this time, the mixture was partitioned with methylene chloride and water. The organic layer was separated, dried over sodium sulfate, filtered, and concentrated under reduced pressure. Purification by flash chromatography (silica, gradient 1-5%,methanol/methylene chloride) afforded 6-ethoxy-2-(1-methyl-1*H*-indol-5-yl)isoindolin-1-one (0.092 g, 88%) as an off-white solid: mp 185-190 °C; ¹H NMR (300 MHz, CDCl₃) δ 7.89 (d, *J*= 1.9 Hz, 1H), 7.71 (dd, *J*= 2.1, 8.8 Hz, 1H), 7.42-7.38 (m, 3H), 7.14 (dd, *J*= 2.4, 8.3 Hz, 1H), 7.08 (d, *J*= 3.0 Hz, 1H), 6.50 (dd, *J*= 1.0, 3.1 Hz, 1H), 4.85 (s, 2H), 4.13 (q, *J*= 7.0 Hz, 2H), 3.82 (s, 3H); 1.46 (t, *J*= 7.0 Hz, 3H); ESI MS *m*/*z* 307 [M + H]⁺.

### EXAMPLE 7

This example illustrates a preparation of 2-(1-methyl-1*H*-indol-5-yl)-6-(2,2,2-trifluoroethoxy)isoindolin-1-one in an embodiment of the invention.

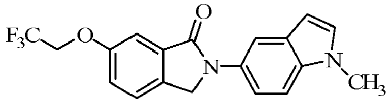

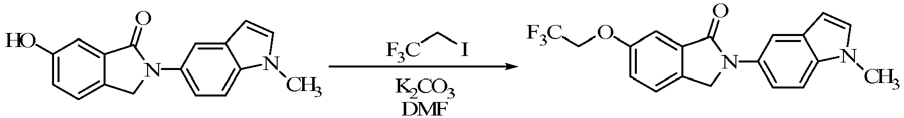

A mixture of 6-hydroxy-2-(1-methyl-1H-indol-5-yl)isoindolin-1-one (0.150 g, 0.54 mmol), potassium carbonate (0.372 g, 2.69 mmol), and *N,N*-dimethylformamide (5 mL) was strirred for 30 min then 1,1,1-trifluoro-2-iodoethane (0.105 mL, 1.07 mmol) was added. The mixture was then heated for 2 d at 100 °C. After this time, the mixture was cooled to room temperature and diluted with ethyl acetate (20 mL) and water (20 mL). The aqueous layer was extracted with ethyl acetate (100 mL) and the combined organics washed with 5% aqueous lithium chloride (50 mL), water (50 mL), brine (20 mL), dried over magnesium sulfate, and concentrated under reduced pressure. Purification by flash chromatography (silica, 1:200 methanol/methylene chloride) afforded 2-(1-methyl-1H-indol-5-yl)-6-(2,2,2-trifluoroethoxy)isoindolin-1-one as an off-white solid: mp 182-188 °C; ¹H NMR (500 MHz, CDCl₃) δ 7.89 (d, *J*= 2.0 Hz, 1H), 7.69 (dd, *J*= 2.0, 9.0 Hz, 1H), 7.45 (d, *J*= 8 Hz, 1H), 7.42 (d, *J* = 2.5 Hz, 1H), 7.36 (d, *J* = 9.0 Hz, 1H), 7.23 (dd, *J* = 2.0, 2.5, 8.0, 8.5 Hz, 1H), 7.09 (d, *J* = 3.0 Hz, 1H), 6.50 (d, *J* = 3.0 Hz, 1H), 4.87 (s, 2H), 4.44 (q, *J* = 8.0, 8.5 Hz, 2H), 3.82 (s, 3H); APCI MS *m*/*z* 361 [M + H]⁺.

### EXAMPLE 8

This example illustrates a preparation of N-(2-(1-methyl-1H-indol-5-yl)-3-oxoisoindolin-5-yl)acetamide in an embodiment of the invention.

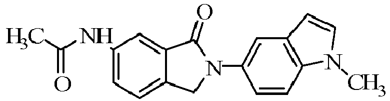

### Step A: Synthesis of 6-amino-2-(1-methyl-1H-indol-5-yl)isoindolin-1-one as an intermediate

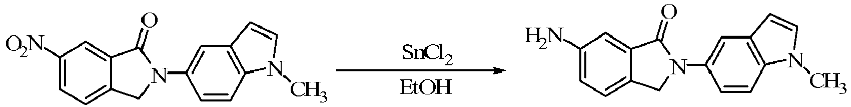

To a mixture of 2-(1-methyl-1H-indol-5-yl)-6-nitroisoindolin-1-one (0.158 g, 0.51 mmol) in ethanol (10 mL) was added tin (II) chloride (0.487 g, 2.57 mmol) portionwise over 30 minutes. The mixture was then heated to 80 °C overnight. After this time, the mixture was cooled to 0 °C with an ice bath and saturated aqueous potassium carbonate (30 mL) was added and the suspension filtered through diatomaceous earth, the organics extracted with ethyl acetate, dried over magnesium sulfate and concentrated under reduced pressure to afford 6-amino-2-(1-methyl-1H-indol-5-yl)isoindolin-1-one (0.075 g crude) as an impure solid. This material was used with no additional purification or characterization.

### Step B: Synthesis of N-(2-(1-methyl-1H-indol-5-yl)-3-oxoisoindolin-5-yl)acetamide

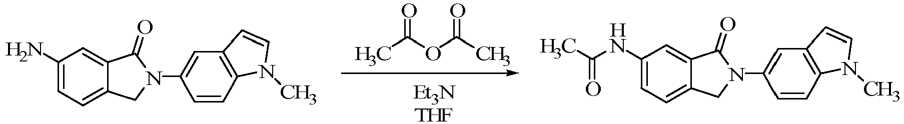

A mixture of 6-amino-2-(1-methyl-1H-indol-5-yl)isoindolin-1-one (0.075 g, 0.27 mmol), tetrahydrofuran (5 mL), triethylamine (0.075 mL, 0.54 mmol), and acetic anhydride (0.040 mL, 0.41 mmol) was strirred for 3 d at room temperature. After this time, the mixture was concentrated under reduced pressure and recrystallized from methanol/methylene chloride to afford N-(2-(1-methyl-1H-indol-5-yl)-3-oxoisoindolin-5-yl)acetamide as a brown-red solid: mp 275-280 °C; ¹H NMR (500 MHz, DMSO-*d*₆) δ 10.18 (s, 1H), 8.12 (d, *J*= 1.5 Hz, 1H), 7.94 (d, *J* = 2.0 Hz, 1H), 7.73 (dd, *J* = 2. 0, 8 .0 Hz, 1H), 7.67 (dd, *J* = 2.0, 8.5 Hz, 1H), 7.56 (d, *J* = 8.5 Hz, 1H), 7.48 (d, *J* = 8.5 Hz, 1H), 7.35 (d, *J* = 3.0 Hz, 1H), 6.44 (d, *J*= 3.0 Hz, 1H), 4.97 (s, 2H), 3.80 (s, 3H), 2.09 (s, 3H); APCI MS *m*/*z* 320 [M + H]⁺.

### EXAMPLE 9

This example illustrates a preparation of 4-amino-2-(1-methyl-1*H*-indol-5-yl)isoindolin-1-one in an embodiment of the invention.

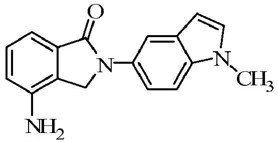

### Step A: Synthesis of ethyl 2-methyl-3-nitrobenzoate as an intermediate

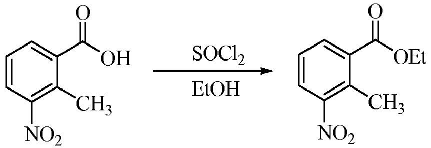

A mixture of2-methyl-3-nitrobenzoic acid (7.10g, 39.2 mmol) in ethanol (200 mL) was cooled to 0 °C with an ice bath, and thionyl chloride (17.11 mL, 235.6 mmol) was added dropwise over 30 minutes. After this time, the mixture was warmed to room temperature for 1 h, then transferred to an oil bath, and heated at 70 °C overnight. The mixture was then cooled to room temperature and concentrated under reduced pressure. The solids obtained were then redissolved in diethyl ether (200 mL) and washed with 1 N sodium hydroxide (200 mL), brine (200 mL), dried over magnesium sulfate, and concentrated under reduced pressure. Purification by flash chromatography (silica, 1:50 ethyl acetate/hexanes) afforded ethyl 2-methyl-3-nitrobenzoate (6.46 g, 79%) as a yellow oil: ¹H NMR (500 MHz, CDCl₃) δ 7.98 (dd, *J*= 1.0, 1.5, 7.5, 8.0 Hz, 1H), 7.84 (dd, *J*= 1.5, 8.0 Hz, 1H), 7.38 (t, *J*= 7.5, 8.0 Hz, 1H), 4.40 (q, *J* = 7.0 Hz, 2H), 2.62 (s, 3H), 1.41 (t,*J*= 7.0 Hz, 3H).

### Step B: Synthesis of ethyl 2-(bromomethyl)-3-nitrobenzoate as an intermediate

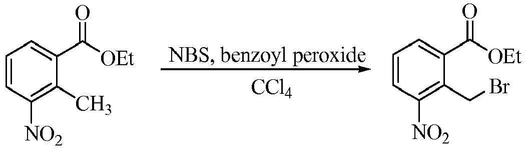

A mixture of ethyl 2-methyl-3-nitrobenzoate (3.07 g, 14.7 mmol) in carbon tetrachloride (70 mL) was heated to 78 °C in an oil bath then a mixture of *N-*bromosuccinimide (2.87 g, 16.1 mmol) and benzoyl peroxide (0.35 g, 1.46 mmol) was added portionwise over 4 h. The mixture was then stirred overnight at 78 °C. After this time, the reaction mixture was cooled to room temperature and the solids removed by filtration. The filtrate was concentrated under reduced pressure, and purified by flash chromatography (silica, 1:49 ethyl acetate/hexanes) to afford ethyl 2-(bromomethyl)-3-nitrobenzoate (2.60 g, 61%) as an off-white solid: ¹H NMR (300 MHz, CDCl₃) δ 8.09 (dd, *J*= 1.2, 7.8 Hz, 1H), 7.95 (dd, *J* = 1.2, 8.1 Hz, 1H), 7.53 (t, *J* = 7.8, 8.1 Hz, 1H), 5.15 (s, 2H), 4.46 (q, *J* = 6.9, 7.2 Hz, 2H), 1.44 (t, *J*= 7.2 Hz, 3H).

### Step C: Synthesis of 2-(1-methyl-1H-indol-5-yl)-4-nitroisoindolin-1-one

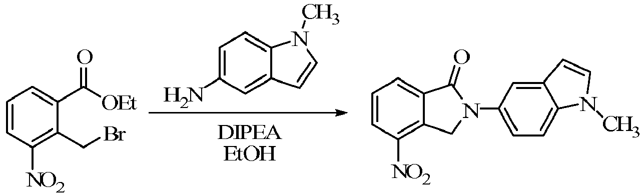

A mixture of 1-methyl-1H-indol-5-amine (0.38 g, 2.60 mmol), *N,N-*diisopropylethyl amine (0.45 mL, 2.60 mmol) in ethanol (50 mL) was stirred for 10 min in a sealed tube at room temperature. Ethyl 2-(bromomethyl)-3-nitrobenzoate (0.500 g, 1.73 mmol) was added portionwise over 3 h and the mixture was then heated to 100 °C overnight. The mixture was then cooled to room temperature, LiOH•H₂O was added as a solution in water (2 mL), and the mixture stirred a further night at room temperature. The solids were collected by filtration to afford 2-(1-methyl-1H-indol-5-yl)-4-nitroisoindolin-1-one (0.481 g, 90%) as a yellow solid: mp 267-271 °C; ¹H NMR (500 MHz, CDCl₃) δ 8.45 (d, *J* = 8.5 Hz, 1H), 8.29 (d, *J* = 7.5 Hz, 1H), 7.97 (d, *J* = 2.0 Hz, 1H), 7.75 (t, *J* = 7.5, 8.0 Hz, 1H), 7.72 (dd, *J*= 2.5, 9.0 Hz, 1H), 7.40 (d, *J*= 8.5 Hz, 1H), 7.11 (d, *J*= 3.0 Hz, 1H), 6.54 (d, *J*= 3.0 Hz, 1H), 5.39 (s, 2H), 3.83 (s, 3H); APCI MS *m*/*z* 308 [M + H]⁺.

### Step D: Synthesis of 4-amino-2-(1-methyl-1H-indol-5-yl)isoindolin-1-one

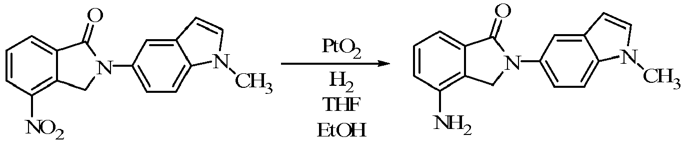

A mixture of 2-(1-methyl-1H-indol-5-yl)-4-nitroisoindolin-1-one (0.45 g, 1.45 mmol) and platinum (IV) oxide (0.045 g, 10% by weight) in ethanol (20 mL) and tetrahydrofuran (30 mL), was shaken under an atmosphere of hydrogen at 40 psi for 4 h. The mixture was filtered through diatomaceous earth and concentrated under reduced pressure. Recrystallization from methylene chloride afforded 4-amino-2-(1-methyl-1H-indol-5-yl)isoindolin-1-one (0.304 g, 75%) as a green solid: mp 198-203 °C; ¹H NMR (500 MHz, DMSO-*d*₆) δ 7.91 (d, *J*= 2.0 Hz, 1H), 7.66 (dd, *J*= 2.0, 2.5, 8.5, 9.0 Hz, 1H), 7.49 (d, *J*= 9.0 Hz, 1H), 7.36 (d, *J* = 3.0 Hz, 1H), 7.21 (t, *J* = 7.5, 8.0 Hz, 1H), 6.95 (d, *J* = 7.5 Hz, 1H), 6.81 (d, *J*= 7.5 Hz, 1H), 6.44 (d, *J*= 3.0 Hz, 1H), 5.50 (s, 2H), 4.77 (s, 3H), 3.80 (bs, 3H); APCI MS *m*/*z* 278 [M + H]⁺.

### EXAMPLE 10

This example illustrates a preparation of N-(2-(1-methyl-1H-indol-5-yl)-1-oxoisoindolin-4-yl)acetamide in an embodiment of the invention.

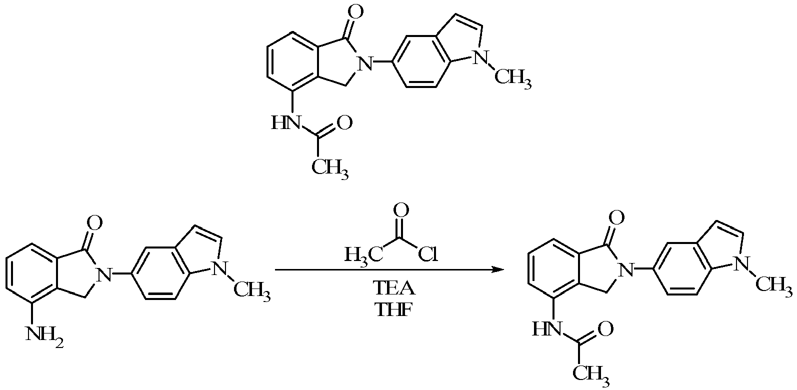

A mixture of 4-amino-2-(1-methyl-1H-indol-5-yl)isoindolin-1-one (0.151 g, 0.54 mmol) and triethylamine (0.152 mL, 1.09 mmol) in tetrahydrofuran (10 mL) was stirred for 10 min then acetyl chloride (0.043 mL, 0.60 mmol) was added, and the mixture was stirred overnight at room temperature. After this time, the solvents were removed under reduced pressure and purification by flash chromatography (silica, 3:1 ethyl acetate/hexanes) afforded N-(2-(1-methyl-1H-indol-5-yl)-1-oxoisoindolin-4-yl)acetamide (0.057 g, 33%) as a white solid: mp 239-243 °C; ¹H NMR (300 MHz, CDCl₃) δ 7.85 (d, *J*= 1.8 Hz, 1H), 7.77 (d, *J*= 7.2 Hz, 1H), 7.66 (dd, *J* = 2.1, 9.0 Hz, 1H), 7.61 (d, *J* = 7.8 Hz, 1H), 7.47 (t, J = 7.5, 7.8 Hz, 1H), 7.40, (bs, 1H), 7.30 (d, *J* = 8.7 Hz, 1H), 7.06 (d, *J* = 3.0 Hz, 1H), 6.46 (d, *J* = 3.0 Hz, 1H), 4.85 (s, 2H), 3.78 (s, 3H), 2.24 (s, 3H); APCI MS *m*/*z* 320 [M + H]⁺.

### EXAMPLE 11

This example illustrates a preparation of 6-bromo-2-(1-methyl-1H-indol-5-yl)isoindolin-1-one in an embodiment of the invention.

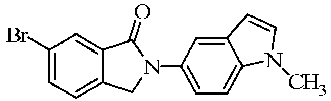

### Step A: Synthesis of ethyl 5-bromo-2-methylbenzoate as an intermediate

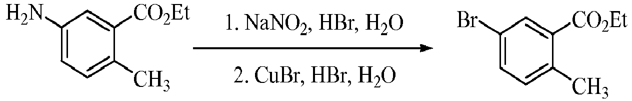

A mixture of ethyl 5-amino-2-methylbenzoate (1.50 g, 8.3 mmol) in 48% HBr (12 mL) and water (24 mL) was cooled with an ice-bath for 15 min. Sodium nitrite (0.59 g, 8.6 mmol) was added dropwise as a solution in water (2 mL), and the resulting mixture was stirred for 5 min. After this time, the mixture was added to an ice-cold mixture of copper (I) bromide (1.40 g, 9.90 mmol) in 48% HBr (5 mL) and water (12 mL). The resulting mixture was heated to 70 °C for 1 h. The reaction mixture was cooled to room temperature and diluted with diethyl ether (150 mL). The organic layer was washed with 1 N hydrochloric acid (50 mL), water (50 mL), and brine (50 mL) then dried over sodium sulfate, filtered, and concentrated under reduced pressure. Purification by flash chromatography (silica, gradient 1-10%, hexanes/ethyl acetate) afforded 1.4 g (69%) of ethyl 5-bromo-2-methylbenzoate as a colorless oil: ¹H NMR (500 MHz, CDCl₃) δ 8.03 (d, *J* = 2.2 Hz, 1H), 7.50 (dd, *J* = 8.2, 2.2 Hz, 1H), 7.12 (dd, *J*= 8.2 Hz, 1H), 4.36 (q, *J*= 7.1 Hz, 2H), 2.54 (s, 3H), 1.40 (t, *J*= 7.2 Hz, 3H); ESI MS *m*/*z* 404 [M + H]⁺.

### Step B: Synthesis of ethyl 5-bromo-2-(bromomethyl)benzoate as an intermediate

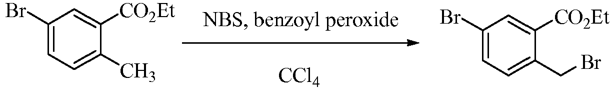

A mixture of ethyl 5-bromo-2-methylbenzoate (7.52 g, 30.9 mmol), *N-*bromosuccinimide (6.05 g, 34.0 mmol) and benzoyl peroxide (0.75 g, 3.10 mmol) in carbon tetrachloride (150 ml) was heated to 70 °C in an oil bath for 18 h. The mixture was cooled to room temperature, and the solids were removed by filtration. The filtrate was then concentrated under reduced pressure and purified by flash chromatography (silica, gradient 1-10%, ethyl acetate/hexanes) to afford ethyl 5-bromo-2-(bromomethyl)benzoate (7.57 g, 76%) as a clear oil: ¹H NMR (300 MHz, CDCl₃) 8.09 (d, *J* = 2.2 Hz, 1H), 7.61 (dd, *J* = 8.3, 2.2 Hz, 1H), 7.33 (d, *J*= 8.2 Hz, 1H), 4.90 (s, 2H), 4.42 (q, *J*= 7.2 Hz, 2H), 1.43 (t, *J*= 7.1 Hz, 3H).

### Step C: Preparation of 6-bromo-2-(1-methyl-1H-indol-5-yl)isoindolin-1-one

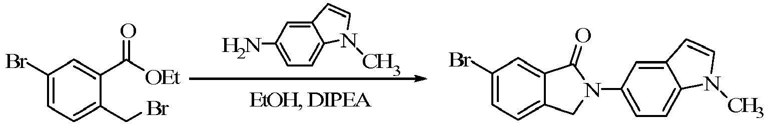

A mixture of ethyl5-bromo-2-(bromomethyl)benzoate (1.03 g, 3.20 mmol), 1-methyl-1*H*-indol-5-amine (0.561 g, 3.80 mmol) and *N,N*-diisopropylethylamine (0.662 mL, 3.80 mmol) in ethanol (40 mL) was heated in a sealed tube at 110 °C overnight. The mixture was cooled to room temperature and the solid collected by filtration to afford 6-bromo-2-(1-methyl-1*H*-indol-5-yl)isoindolin-1-one (0.575 g, 53%) as an off-white solid: mp 235-237 °C; ¹H NMR (500 MHz, CDCl₃) δ 8.08 (d, *J*= 1.6 Hz, 1H), 7.88 (d, *J*= 2.1 Hz, 1H), 7.70-7.67 (m, 2H), 7.40-7.36 (m, 2H), 7.09 (d, *J*= 3.1 Hz, 1H), 6.50 (d, *J*= 3.1 Hz, 1H), 4.86 (s, 2H), 3.81 (s, 3H); ESI MS *m*/*z* 341 [M + H]⁺.

### EXAMPLE 12

This example illustrates a preparation of 6-isobutyl-2-(1-methyl-1H-indol-5-yl)isoindolin-1-one in an embodiment of the invention.

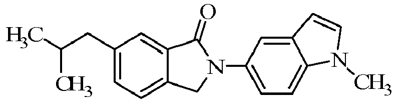

### Step A: Synthesis of 2-(1-methyl-1H-indol-5-yl)-6-(4,4,5,5-tetramethyl-1,3,2-dioxaborolan-2-yl)isoindolin-1-one as an intermediate

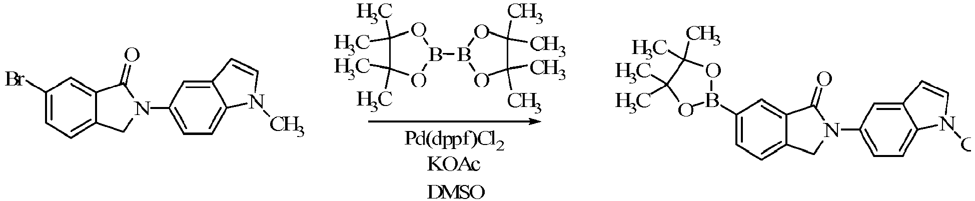

A mixture of 6-bromo-2-(1-methyl-1*H-*indol-5-yl)isoindoline-1-one (0.200 g, 0.59 mmol), bis(pinacolato)diboron (0.223 g, 0.88 mmol), dichloro[1,1'-bis(diphenylphosphino)ferrocene]palladium(II) (0.021 g, 0.029 mmol) and potassium acetate (0.173 g, 1.76 mmol) in DMSO (2 mL) was degassed with argon and heated at 80 °C overnight. The mixture was cooled to room temperature, diluted with ethyl acetate, and filtered through diatomaceous earth. The filtrate was washed with water and brine, dried with sodium sulfate, and filtered. Concentration and purification by flash chromatography (silica, gradient 0-5%, ethyl acetate/hexanes) afforded 2-(1-methyl-1H-indol-5-yl)-6-(4,4,5,5-tetramethyl-1,3,2-dioxaborolan-2-yl)isoindolin-1-one (0.175 g, 77%) as an off-white solid: ¹H NMR (500 MHz, CDCl₃) δ 8.42 (s, 1H), 8.01(dd, *J* = 7.5, 0.9 Hz, 1H), 7.91 (d, *J* = 6.1 Hz, 1H), 7.73 (dd, *J*= 8.8, 2.1 Hz, 1H), 7.51 (dd, *J*= 7.5, 0.6 Hz, 1H), 7.36 (d, *J*= 8.8 Hz, 1H), 7.08 (d, *J*= 3.0 Hz, 1H), 6.50 (d, *J*= 3.1 Hz, 1H), 4.92 (s, 2H), 3.81 (s, 3H), 1.37 (s, 12H); ESI MS *m*/*z* 389 [M+ H]⁺.

### Step B: Synthesis of 2-(1-methyl-1H-indol-5-yl)-6-(2-methylprop-1-enyl)isoindolin-1-one

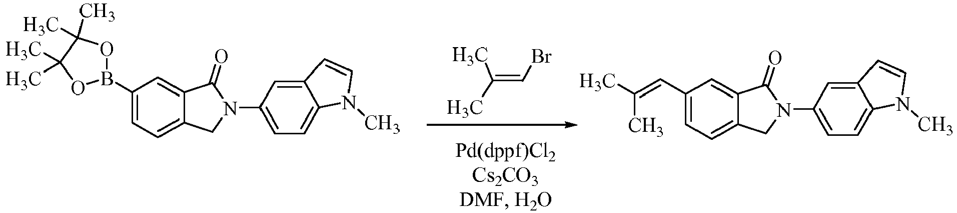

A mixture of 2-(1-methyl-1H-indol-5-yl)-6-(4,4,5,5-tetramethyl-1,3,2-dioxaborolan-2-yl)isoindolin-1-one (0.169 g, 0.12 mmol), 1-bromo-2-methylprop-1-ene (0.118 g, 0.87 mmol), dichloro[1,1'-bis(diphenylphosphino)ferrocene]palladium(II)dichloromethane adduct (0.036 g, 0.044 mmol) and cesium carbonate (0.425 g, 1.31 mmol) in 10:1 DMF/water (5.5 mL) was degassed with argon and heated to 100 °C for 3.5 h. The mixture was then cooled to room temperature, diluted with ethyl acetate, and filtered through diatomaceous earth. The filtrate was washed with water and the water layer re-extracted twice with ethyl acetate. The combined organic layers were washed with 5% aqueous lithium chloride, dried with sodium sulfate, filtered, and concentrated. The crude residue was dissolved in methylene chloride and filtered through a plug of silica gel (25% ethyl acetate/hexanes as eluent). The filtrate was concentrated and recrystallized from ethyl acetate/hexanes to afford 2-(1-methyl-1H-indol-5-yl)-6-(2-methylprop-1-enyl)isoindolin-1-one (0.054 g, 39%) as an off-white solid: ¹H NMR (500 MHz, CDCl₃) δ 7.90 (d, *J*= 2.02 Hz, 1H), 7.80 (s, 1H), 7.74 (dd, *J*= 8.8, 2.1 Hz, 1H), 7.47-7.40 (m, 2H), 7.37 (d, *J*= 8.8 Hz, 1H), 7.08 (d, *J*= 3.0 Hz, 1H), 6.50 (d, *J*= 3.0 Hz, 1H), 6.36 (s, 1H), 4.90 (s, 2H), 3.82 (s, 3H), 1.94 (d, *J*= 0.9 Hz, 3H), 1.94 (d, *J*= 0.8 Hz, 3H); ESI MS *m*/*z* 317 [M+ H]⁺.

### Step C: Synthesis of 6-isobutyl-2-(1-methyl-1H-indol-5-yl)isoindolin-1-one

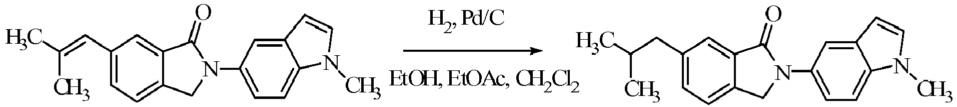

A mixture of 2-(1-methyl-1H-indol-5-yl)-6-(2-methylprop-1-enyl)isoindolin-1-one (0.052 g, 0.16 mmol) and 10% palladium on carbon (0.012 g, 23% by weight) in 5:5:1 ethanol/ethyl acetate/methylene chloride (11 mL) was stirred under an atmosphere of hydrogen for 1 h. After this time, the reaction was filtered through diatomaceous earth. Concentration and purification by flash chromatography (silica, gradient 0-2.5%, methanol/methylene chloride) afforded 6-isobutyl-2-(1-methyl-1H-indol-5-yl)isoindolin-1-one (0.020 g, 38%) as an orange oil: mp 193-194 °C; ¹H NMR (500 MHz, CDCl₃) δ 7.89 (d, *J*= 2.1 Hz, 1H), 7.74-7.71 (m, 2H), 7.41 (d, *J*= 7.71 Hz, 1H), 7.37-7.35 (m, 2H), 7.08 (d, *J* = 3.1 Hz, 1H), 6.50 (dd, *J*= 3.1, 0.5 Hz, 1H), 4.88 (s, 2H), 3.81 (s, 3H), 2.60 (d, *J*= 7.2 Hz, 2H) 1.93 (m, 1H), 0.93 (d, *J*= 6.6 Hz, 6H); ESI MS *m*/*z* 319 [M+ H]⁺.

### EXAMPLE 13

This example illustrates a preparation of 6-butyl-2-(1-methyl-1H-indol-5-yl)isoindolin-1-one in an embodiment of the invention.

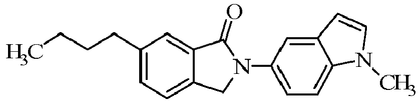

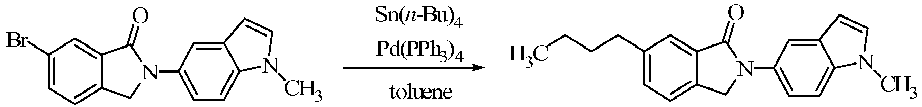

A mixture of 6-bromo-2-(1-methyl-1*H* indol-5-yl)isoindoline-1-one (0.150 g, 0.44 mmol), tetrabutyltin (0.632 g, 1.8 mmol) and tetrakis(triphenylphosphine)palladium (0) (0.203 g, 0.18 mmol) in toluene (5 mL) was heated to 125 °C for 60 h. The mixture was cooled to room temperature, diluted with ethyl acetate, and filtered through diatomaceous earth. The filtrate was concentrated and purified by flash chromatography (silica, gradient 3-20%, ethyl acetate/hexanes) to afford 6-butyl-2-(1-methyl-1H-indol-5-yl)isoindolin-1-one (0.024 g, 13%) as a white solid: mp 167-168 °C; ¹H NMR (500 MHz, CDCl₃) δ 7.89 (d, *J*= 2.0 Hz, 1H), 7.77 (s, 1H), 7.73 (dd, *J*= 8.8, 2.1 Hz, 1H), 7.42-7.38 (m, 2H), 7.36 (d, *J*= 8.8 Hz, 1H), 7.08 (d, *J*= 3.1 Hz, 1H), 6.50 (d, *J*= 2.8 Hz, 1H), 4.88 (s, 2H), 3.82 (s, 3H), 2.74 (t, *J*= 7.6 Hz, 2H), 1.66 (m, 2H), 1.37 (m, 2H), 0.94 (t, *J*= 7.4 Hz, 3H); ESI MS *m*/*z* 319 [M+ H]⁺.

### EXAMPLE 14

This example illustrates a preparation of (Z)-6-(but-2-en-2-yl)-2-(1-methyl-1H-indol-5-yl)isoindolin-1-one in an embodiment of the invention.

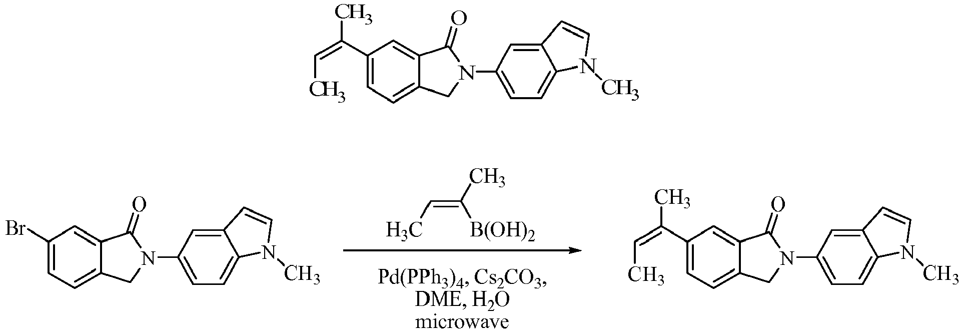

A mixture of 6-bromo-2-(1-methyl-1*H-*indol-5-yl)isoindoline-1-one (0.150 g, 0.44 mmol), *cis*-1-propene-1-boronic acid (0.264 g, 2.6 mmol), tetrakis(triphenylphosphine)palladium (0) (0.254 g, 0.22 mmol), and cesium carbonate (0.430 g, 1.32 mmol) in 5:2 DME/water (7 mL) was heated in a microwave reactor for 30 min at 120 °C. The mixture was cooled to room temperature and then diluted with ethyl acetate (8 mL). The reaction mixture was filtered and the water layer removed. The organic phase from the filtrate was dried with sodium sulfate, concentrated, and purified by flash chromatography (silica, gradient 0-1%, methanol/methylene chloride) to afford (Z)-6-(but-2-en-2-yl)-2-(1-methyl-1H-indol-5-yl)isoindolin-1-one (0.108 g, 78%) as a white solid: mp 144-145 °C; ¹H NMR (300 MHz, CDCl₃) δ 7.90 (d, *J*= 1.8 Hz, 1H), 7.78 (s, 1H), 7.74 (dd, *J*= 8.8, 2.1 Hz, 1H), 7.49 (dd, *J* = 12.9, 0.6 Hz, 1H), 7.41 (dd, *J* = 7.8, 1.6 Hz, 1H), 7.37 (d, *J* = 8.8 Hz, 1H), 7.09 (d, *J* = 3.1 Hz, 1H), 6.51 (dd, *J*= 3.1, 0.7 Hz, 1H), 5.64 (m, 1H), 4.91 (s, 2H), 3.82 (s, 3H), 2.07 (m, 3H), 1.61 (m, 3H); ESI MS *m*/*z* 317 [M+ H]⁺.

### EXAMPLE 15

This example illustrates a preparation of 6-sec-butyl-2-(1-methyl-1H-indol-5-yl)isoindolin-1-one in an embodiment of the invention.

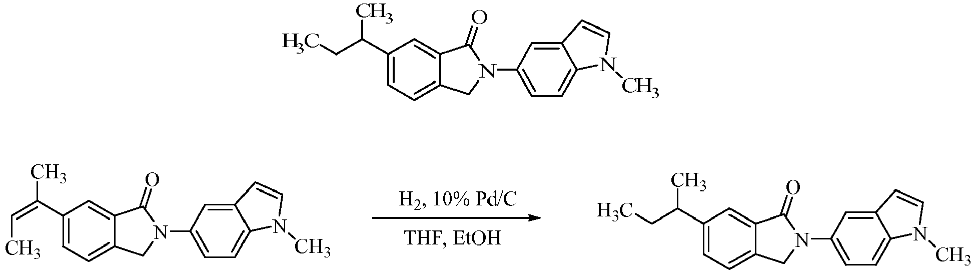

A mixture of (Z)-2-(1-methyl-1H-indol-5-yl)-6-(prop-1-enyl)isoindolin-1-one (0.094 g, 0.30 mmol) and 10% palladium on carbon (0.024 g, 25% by weight) in 9:1 ethanol/THF( 20 mL) was shaken under an atmosphere of hydrogen at 50 psi for 3 h. After this time, the reaction was filtered through diatomaceous earth, concentrated under reduced pressure, and purified by flash chromatography (silica, gradient 0-0.5%, methanol/methylene chloride) to afford 6-sec-butyl-2-(1-methyl-1H-indol-5-yl)isoindolin-1-one (0.070 g, 75%) as a white solid: mp 146-147 °C; ¹H NMR (500 MHz, CDCl₃) δ 7.89 (d, *J*= 2.0 Hz, 1H), 7.78 (s, 1H), 7.72 (dd, *J*= 8.8, 2.1 Hz, 1H), 7.43 (d, *J*= 7.7 Hz, 1H), 7.40 (dd, *J*= 7.7, 1.5 Hz, 1H), 7.36 (d, *J*= 8.8 Hz, 1H), 7.08 (d, *J*= 3.1 Hz, 1H), 6.50 (d, *J*= 2.9 Hz, 1H), 4.88 (s, 2H), 3.81 (s, 3H), 2.74 (m, 1H), 1.66 (m, 2H), 1.30 (d, *J* = 7.0 Hz, 3H), 0.84 (t, *J* = 7.4 Hz, 3H); ESI MS *m*/*z* 319 [M+ H]⁺.

### EXAMPLE 16

This example illustrates a preparation of 2-(1-methyl-1H-indol-5-yl)-6-(pyrrolidin-1-yl)isoindolin-1-one in an embodiment of the invention.

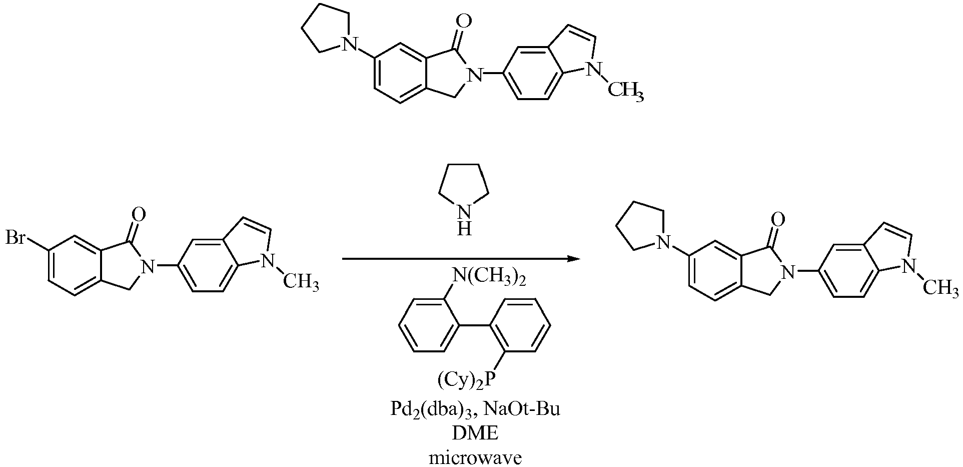

A mixture of 6-bromo-2-(1-methyl-1*H-*indol-5-yl)isoindoline-1-one (0.250 g, 0.73 mmol), pyrrolidine (0.079 g, 1.1 mmol), tris(dibenzylideacetone)dipalladium (0) (0.134 g, 0.15 mmol), 2'-(dicyclohexylphosphino)-N,N-dimethylbiphenyl-2-amine (0.058 g, 0.15 mmol), and sodium *tert*-butoxide (0.141 g, 1.46 mmol) in DME (10 mL) was heated in a microwave reactor for 20 min at 120 °C. The mixture was cooled to room temperature, diluted with 95:5 methylene chloride/methanol, and filtered through diatomaceous earth. The filtrate was concentrated and purified by flash chromatography (silica, gradient 5-40%, ethyl acetate/hexanes). Further purification by preparative HPLC (90:10 water (0.05% TFA) to 100% acetonitrile (0.05% TFA) over 20 minutes) afforded 2-(1-methyl-1H-indol-5-yl)-6-(pyrrolidin-1-yl)isoindolin-1-one (0.031 g, 13%) as an off-white solid: mp 252-253 °C; ¹H NMR (300 MHz, CDCl₃) δ 7.90 (s, 1H), 7.75 (d, *J*= 9.0 Hz, 1H), 7.34 (m, 2H), 7.08 (d, *J*= 2.5 Hz, 2H), 6.78 (d, *J*= 8.2 Hz, 1H), 6.50 (d, *J*= 2.9 Hz, 1H), 4.82 (s, 2H), 3.81 (s, 3H), 3.36 (t, *J*= 6.3 Hz, 3H), (t, *J*= 6.0 Hz, 3H); ESI MS *m*/*z* 332 [M+ H]⁺.

### EXAMPLE 17

This example illustrates a preparation of 2-(1-isopropyl-1H-indol-5-yl)-6-methoxyisoindolin-1-one in an embodiment of the invention.

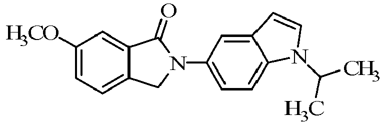

### Step A: Synthesis of 1-isopropyl-5-nitro-1H-indole as an intermediate

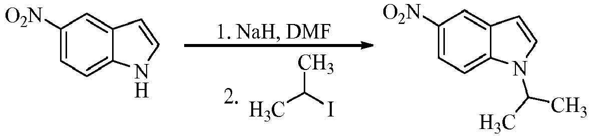

To a mixture of 5-nitro-1H-indole (1.0 g, 6.16 mmol) in N,N-dimethylformamide (10.0 mL) was added sodium hydride (60% in mineral oil, 0.272 g, 6.77 mmol), and the mixture was stirred for 1 h at room temperature. After this time, 2-iodopropane (0.678 mL, 6.77 mmol) was added, and the mixture was stirred at room temperature overnight. Saturated aqueous ammonium chloride (25 mL) was added and the organics extracted with ethyl acetate (50 mL). The organics were then washed with 5% aqueous lithium chloride (100 mL), water (100 mL), and brine (50 mL), dried over magnesium sulfate and concentrated under reduced pressure. Purification by flash chromatography (silica, 1:9 ethyl acetate/hexanes) afforded 1-isopropyl-5-nitro-1H-indole (0.62 g, 49%) as a yellow/green solid: ¹H NMR (500 MHz, CDCl₃) δ 8.59 (d, *J*= 2.0 Hz, 1H), 8.11 (dd, *J*= 2.0, 2.5, 9.0, 9.5 Hz, 1H), 7.39 (s, 1H), 7.37 (d, *J*= 3.5 Hz, 1H), 6.70 (d, *J*= 3.0 Hz, 1H), 4.72 (m, 1H), 1.56 (d, *J=* 6.5 Hz, 6H).

### Step B: Synthesis of 1-isopropyl-1H-indol-5-amine as an intermediate

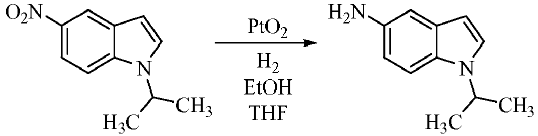

A mixture of 1-isopropyl-5-nitro-1H-indole (0.622 g, 3.04 mmol) and platinum (IV) oxide (0.062 g, 10% by weight) in tetrahydrofuran (30 mL) was shaken under an atmosphere of hydrogen at 40 psi for 4 h. The mixture was filtered through diatomaceous earth and concentrated under reduced pressure. Purification by flash chromatography (silica, 1:4, ethyl acetate/hexanes) afforded 1-isopropyl-1H-indol-5-amine (0.228 g, 43%) as a purple oil: ¹H NMR (500 MHz, CDCl₃) δ 7.17 (d, *J*= 8.5 Hz, 1H), 7.13 (d, *J*= 3.5 Hz, 1H), 6.92 (d, *J*= 3.5 Hz, 1H), 6.67 (dd, *J*= 2.0, 2.5, 8.5, 9.0 Hz, 1H), 6.31 (d, *J*= 3.0 Hz, 1H), 4.57 (m, 1H), 3.47 (bs, 2H), 1.49 (d, *J*= 6.5 Hz, 6H).

### Step C: Synthesis of 2-(1-isopropyl-1H-indol-5-yl)-6-methoxyisoindolin-1-one

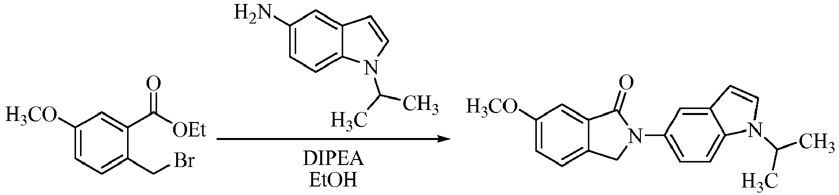

A mixture of 1-isopropyl-1H-indol-5-amine (0.212 g, 1.22 mmol), *N,N-*diisopropylethyl amine (0.212 mL, 1.22 mmol) in ethanol (30 mL) was stirred for 10 min in a sealed tube at room temperature. After this time, ethyl 2-(bromomethyl)-5-methoxybenzoate (0.222 g, 0.81 mmol) was added portionwise over 3 h, and the mixture was heated to 100 °C overnight. The mixture was cooled to room temperature, LiOH•H₂O was added as a solution in water (2 mL), and the mixture was stirred a overnight at room temperature. The mixture was concentrated under reduced pressure and purification by flash chromatography (silica, 1:4 ethyl acetate/hexanes) afforded 2-(1-isopropyl-1H-indol-5-yl)-6-methoxyisoindolin-1-one (0.065 g, 25%) as a white solid: mp 144-148 °C; ¹H NMR (500 MHz, CDCl₃) δ 7.86 (d, *J=* 2.0 Hz, 1H), 7.69 (dd, *J*= 2.0, 2.5, 8.5, 9.0 Hz, 1H), 7.43-7.38 (m, 3H), 7.25 (s, 1H), 7.14 (dd, *J*= 2.5, 8.5 Hz, 1H), 6.53 (d, *J*= 3.0 Hz, 1H), 4.85 (s, 2H), 4.68 (m, 1H), 3.90 (s, 3H), 1.54 (d, *J*= 6.5 Hz, 6H); APCI MS *m*/*z* 321 [M + H]⁺.

### EXAMPLE 18

This example illustrates a preparation of 2-(1,2-dimethyl-1H-indol-5-yl)-6-methoxyisoindolin-1-one in an embodiment of the invention.

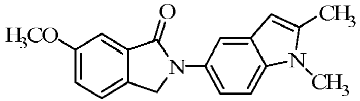

### Step A: Synthesis of 1,2-dimethyl-5-nitro-1H-indole as an intermediate

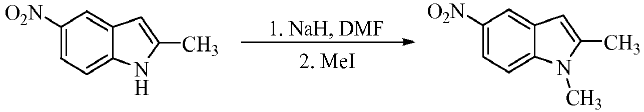

To a mixture of 2-methyl-5-nitro-1H-indole (0.50 g, 2.83 mmol) in *N,N-*dimethylformamide (10.0 mL) was added sodium hydride (60% in mineral oil, 0.125 g, 3.11 mmol), and the mixture was stirred for 1 h at room temperature. After this time, iodomethane (0.195 mL, 3.11 mmol) was added, and the mixture was stirred at room temperature overnight. After this time, saturated aqueous ammonium chloride (25 mL) was added and the organics extracted with ethyl acetate (50 mL). The organics were washed with 5% aqueous lithium chloride (100 mL), water (100 mL), and brine (50 mL), dried over magnesium sulfate and concentrated under reduced pressure to afford 1,2-dimethyl-5-nitro-1H-indole (0.52 g, 96%) as a yellow solid: ¹H NMR (500 MHz, CDCl₃) δ 8.46 (d, *J*= 2.0 Hz, 1H), 8.06 (dd, *J*= 22.5, 9.0 Hz, 1H), 7.25 (d, 1H), 6.42 (s, 1H), 3.72 (s, 3H), 2.46 (s, 3H).

### Step B: Synthesis of 1,2-dimethyl-1H-indol-5-amine as an intermediate

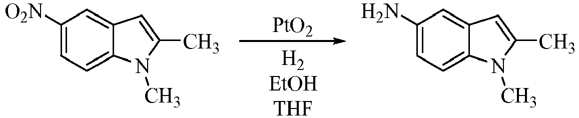

A mixture of 1,2-dimethyl-5-nitro-1H-indole (0.515 g, 2.70 mmol) and platinum (IV) oxide (0.051 g, 10% by weight) in ethanol (30 mL) and tetrahydrofuran (5 mL), was shaken under an atmosphere of hydrogen at 40 psi for 4 h. After this time, the mixture was filtered through diatomaceous earth and concentrated under reduced pressure. Purification by flash chromatography (silica, 1:4, ethyl acetate/hexanes) afforded 1,2-dimethyl-1H-indol-5-amine (0.273 g, 63%) as a pink solid: ¹H NMR (300 MHz, CDCl₃) δ 7.04 (d, *J*= 8.7 Hz, 1H), 6.83 (d, *J*= 2.1 Hz, 1H), 6.60 (dd, *J*= 2.1, 2.4, 8.4, 8.7 Hz, 1H), 6.05 (s, 1H), 3.59 (s, 3H), 2.37 (s, 3H), 1.56 (bs, 2H).

### Step C: Synthesis of 2-(1,2-dimethyl-1H-indol-5-yl)-6-methoxyisoindolin-1-one

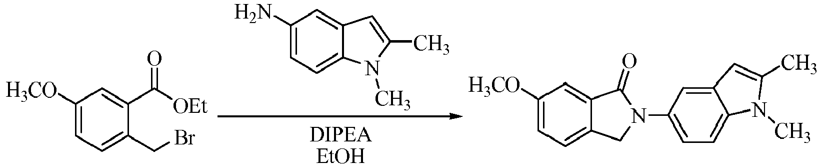

A mixture of 1,2-dimethyl-1H-indol-5-amine (0.273 g, 1.70 mmol), *N,N-*diisopropylethyl amine (0.296 mL, 1.70 mmol) in ethanol (30 mL) was stirred for 10 min in a sealed tube at room temperature. After this time, ethyl 2-(bromomethyl)-5-methoxybenzoate (0.233 g, 0.85 mmol) was added portionwise over 3 h, and the mixture was heated to 100 °C overnight. The mixture was cooled to room temperature, LiOH•H₂O was added as a solution in water (2 mL), and the mixture was stirred overnight at room temperature. The solids were collected by filtration to afford 2-(1,2-dimethyl-1H-indol-5-yl)-6-methoxyisoindolin-1-one (0.122 g, 47%) as a brown/red solid: mp 209-213 °C; ¹H NMR (300 MHz, CDCl₃) δ 7.78 (d, *J =* 2.1 Hz, 1H), 7.62 (dd, *J* = 2.1, 8.7 Hz, 1H), 7.42 (d, *J* = 2.4 Hz, 1H), 7.39 (d, *J* = 8.4 Hz, 1H), 7.28 (d, *J*= 9.0 Hz, 1H), 7.13 (dd, *J* = 2.4, 8.4 Hz, 1H), 6.26 (s, 1H), 4.83 (s, 2H), 3.89 (s, 3H), 3.67 (s, 3H), 2.43 (s, 3H); APCI MS *m*/*z* 307 [M + H]⁺.

### EXAMPLE 19

This example illustrates a preparation of 5-(5-methoxyisoindolin-2-yl)-1-methyl-1H-indole in an embodiment of the invention.

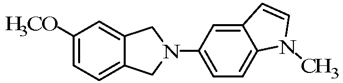

### Step A: Synthesis of 1,2-bis(bromomethyl)-4-methoxybenzene as an intermediate

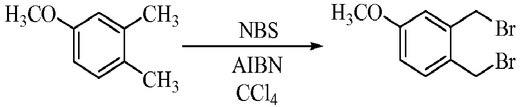

To a mixture of 4-methoxy-1,2-dimethylbenzene (1.02 mL, 7.34 mmol) in carbon tetrachloride (25 mL) was added *N*-bromosuccinimide (2.74 g, 15.4 mmol), followed by 2,2'-azobis(2-methylpropionitrile) (0.03 g, 0.18 mmol) and the mixture stirred at reflux for 4 h. After this time, the mixture was cooled to 50 °C, and the solids were removed by filtration. The filtrate was concentrated under reduced pressure and purification by flash chromatography (silica, 1:99 ethyl acetate/hexanes) afforded 1,2-bis(bromomethyl)-4-methoxybenzene (0.46 g, 21%) as a yellow oil: ¹H NMR (300 MHz, CDCl₃) δ 7.29 (d, *J*= 8.7 Hz, 1H), 6.90 (d, *J* = 2.4 Hz, 1H), 6.82 (dd, *J* = 2.4, 2.7, 8.4, 8.7 Hz, 1H), 4.65 (s, 2H), 4.62 (s, 2H), 3.81 (s, 3H).

### Step B: Synthesis of 5-(5-methoxyisoindolin-2-yl)-1-methyl-1H-indole

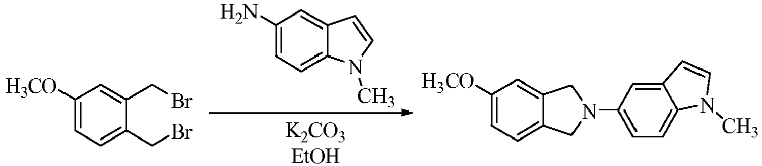

A mixture of 1-methyl-1H-indol-5-amine (0.23 g, 1.56 mmol) and potassium carbonate (0.32 g, 2.34 mmol) in ethanol (30 mL) was stirred for 30 min then 1,2-bis(bromomethyl)-4-methoxybenzene (0.46 g, 1.56 mmol) was added. The mixture was then stirred at room temperature overnight. The solvents were removed under reduced pressure and purification by flash chromatography (silica, 1:20 ethyl acetate/hexanes) and then recrystallization from methanol afforded 5-(5-methoxyisoindolin-2-yl)-1-methyl-1H-indole as a white solid: mp 193-196 °C; ¹H NMR (500 MHz, CDCl₃) δ 7.25 (m, 2H), 6.98 (d, *J* = 3.0 Hz, 1H), 6.90 (d, *J*= 2.0 Hz, 1H), 6.84 (m, 2H), 6.76 (dd, *J*= 2.0, 2.5, 8.5, 9.0 Hz, 1H), 6.37 (d, *J*= 3.0 Hz, 1H), 4.65 (s, 2H), 4.62 (s, 2H), 3.84 (s, 3H), 3.76 (s, 3H); APCI MS *m*/*z* 279 [M + H]⁺.

### EXAMPLE 20

This example illustrates a preparation of 6-methoxy-2-(1-methyl-1*H*-indol-6-yl)isoindolin-1-one in an embodiment of the invention.

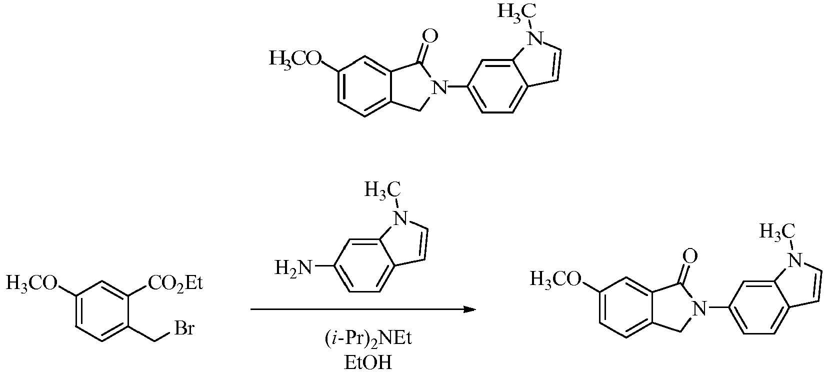

A mixture of 1-methyl-1*H-*indol-6-amine (0.144 g, 0.984 mmol), ethyl 2-(bromomethyl)-5-methoxybenzoate (0.224 g, 0.820 mmol) and *N,N*-diisopropylethylamine (0.106 g, 0.820 mmol) in ethanol (10 mL) was heated in a sealed tube at 110 °C overnight. After cooling to room temperature, a solution of LiOH•H₂O (0.103 g, 2.46 mmol) in water (1 mL) was added, and the mixture was stirred overnight. The mixture was poured into water and extracted with dichloromethane. The organic layer was separated, dried over sodium sulfate, and concentrated. The residue was purified by chromatography (silica, 0-20% ethyl acetate/hexanes) to give a solid which was triturated with ethanol to afford 6-methoxy-2-(1-methyl-1*H-*indol-6-yl)isoindolin-1-one (0.080 g, 33%) as a white solid: mp 158-161 °C; ¹H NMR (500 MHz, CDCl₃) δ 8.12 (m, 1H), 7.63 (d, *J*= 8.5 Hz, 1H), 7.42 (m, 2H), 7.30 (dd, *J* = 8.5, 1.9 Hz, 1H), 7.16 (dd, *J*= 8.2, 2.5 Hz, 1H), 7.07 (d, *J*= 3.1 Hz, 1H), 6.47 (m, 1H), 4.88 (s, 2H), 3.90 (s, 3H), 3.82 (s, 3H); ESI MS *m*/*z* 293 [M + H]⁺.

### EXAMPLE 21

This example illustrates a preparation of N-(2-(1-methyl-1H-indol-6-yl)-1-oxoisoindolin-4-yl)acetamide in an embodiment of the invention.

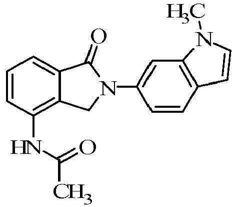

### Step A: Synthesis of 2-(1-methyl-1H-indol-6-yl)-4-nitroisoindolin-1-one

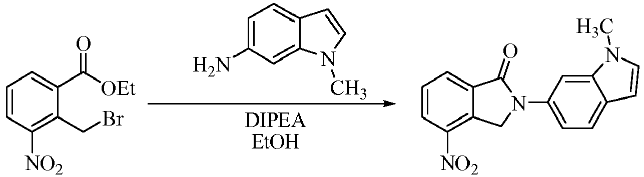

A mixture of 1-methyl-1H-indol-6-amine (0.304 g, 2.08 mmol), *N,N-*diisopropylethyl amine (0.362 mL, 2.08 mmol) in ethanol (40 mL) was stirred for 10 min in a sealed tube at room temperature. Ethyl 2-(bromomethyl)-3-nitrobenzoate (0.400 g, 1.39 mmol) was added portionwise over 3 h, and the mixture was heated to 100 °C overnight. The mixture was cooled to room temperature, LiOH•H₂O was added as a solution in water (2 mL), and the mixture was stirred overnight at room temperature. The solids were collected by filtration to afford 2-(1-methyl-1H-indol-6-yl)-4-nitroisoindolin-1-one (0.308 g, 72%) as a tan solid: mp 225-262 °C; ¹H NMR (500 MHz, CDCl₃) δ 8.45 (d, *J* = 8.5 Hz, 1H), 8.28 (d, *J* = 7.0 Hz, 1H), 8.07 (d, *J*= 1.5 Hz, 1H), 7.76 (t, *J*= 7.5, 8.0 Hz, 1H), 7.68 (d, J = 8.5 Hz, 1H), 7.41 (dd, *J* = 2.0, 8.5 Hz, 1H), 7.10 (d, *J* = 3.0 Hz, 1H), 6.50 (d, *J* = 3.0 Hz, 1H), 5.42 (s, 2H), 3.84 (s, 3H); APCI MS *m*/*z* 308 [M + H]⁺.

### Step B: Synthesis of 4-amino-2-(1-methyl-1H-indol-6-yl)isoindolin-1-one

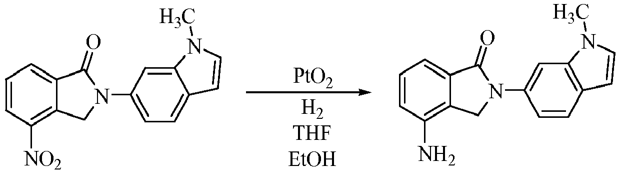

A mixture of 2-(1-methyl-1H-indol-6-yl)-4-nitroisoindolin-1-one (0.118 g, 0.38 mmol) and platinum (IV) oxide (0.012 g, 10% by weight) in ethanol (20 mL) and tetrahydrofuran (5 mL) was shaken under an atmosphere of hydrogen at 40 psi for 4 h. The mixture was filtered through diatomaceous earth and concentrated under reduced pressure to afford 4-amino-2-(1-methyl-1H-indol-6-yl)isoindolin-1-one (0.101 g, crude) as an impure oil. This material was used without additional purification or characterization.

### Step C: Synthesis of N-(2-(1-methyl-1H-indol-6-yl)-1-oxoisoindolin-4-yl)acetamide

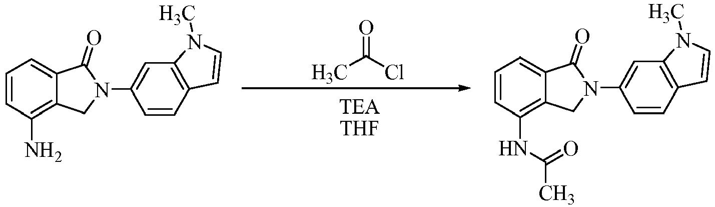

A mixture of 4-amino-2-(1-methyl-1H-indol-6-yl)isoindolin-1-one (0.092 g, 0.33 mmol) and triethylamine (0.093 mL, 0.66 mmol) in tetrahydrofuran (10 mL) was stirred for 10 min. Acetyl chloride (0.026 mL, 0.36 mmol) was added, and the mixture was stirred at room temperature overnight. The solvents were removed under reduced pressure and purification by flash chromatography (silica, 3:1 1 ethyl acetate/hexanes) afforded N-(2-(1-methyl-1H-indol-6-yl)-1-oxoisoindolin-4-yl)acetamide (0.030 g, 28%) as a white solid: mp 180-184 °C; ¹H NMR (300 MHz, CDCl₃) δ 8.10 (s, 1H), 7.79 (d, *J*= 7.2 Hz, 1H), 7.61 (d, *J* = 8.7 Hz, 2H), 7.49 (t, *J* = 7.5, 7.8 Hz, 1H), 7.29 (dd, *J* = 1.8, 2.1, 8.4, 8.7 Hz, 1H), 7.06 (d, J = 3.0 Hz, 1H), 6.46 (d, *J*= 2.7 Hz, 1H), 4.91 (s, 2H), 3.80 (s, 3H), 2.27 (s, 3H); APCI MS *m*/*z* 320 [M + H]⁺.

### EXAMPLE 22

This example illustrates a preparation of 2-(6-(6-methoxy-1-oxoisoindolin-2-yl)-1*H*-indol-1-yl)acetonitrile as an intermediate in an embodiment of the invention.

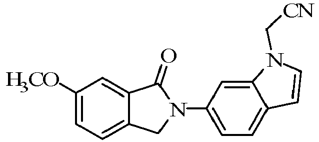

### Step A: Synthesis of 2-(6-nitro-1H-indol-1-yl)acetonitrile

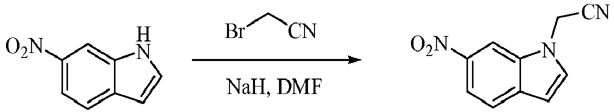

To a solution of 6-nitroindole (1.22 g, 7.52 mmol) in dimethylformamide (15 mL) was added sodium hydride (0.361 g, 9.02 mmol). After stirring for 15 min, bromoacetonitrile (1.36 g, 11.3 mmol) was added, and the mixture was stirred overnight. The mixture was poured into water and extracted with ethyl acetate. The organic layer was separated, washed with brine, dried over sodium sulfate, and filtered. Evaporation and purification by chromatography (silica, 0-30% ethyl acetate in 1:1 dichloromethane/hexanes) gave 2-(6-nitro-1*H-*indol-1-yl)acetonitrile (0.830 g, 55%) as a yellow solid: ¹H NMR (500 MHz, CDCl₃) δ 8.73 (d, *J*= 1.8 Hz, 1H), 8.00 (dd, *J*= 8.8, 2.0 Hz, 1H), 7.87 (d, *J*= 3.2 Hz, 1H), 7.82 (d, *J* = 8.8 Hz, 1H), 6.79 (dd, *J* = 3.2, 0.7 Hz, 1H), 5.74 (s, 2H).

### Step B: Synthesis of 2-(6-amino-1H-indol-1-yl)acetonitrile as an intermediate

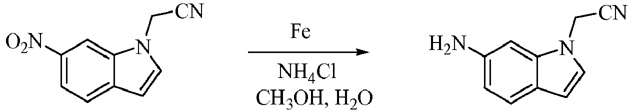

A mixture of 2-(6-nitro-1*H-*indol-1-yl)acetonitrile (0.830 g, 4.12 mmol), iron powder (1.15 g, 20.6 mmol), ammonium chloride (1.10 g, 20.6 mmol), methanol (30 mL), and water (30 mL) was refluxed for 1 h. The mixture was cooled to room temperature, diluted with ethyl acetate, and filtered. The filtrate was partitioned between water and ethyl acetate, and the organic layer was dried over sodium sulfate. Filtration and evaporation gave 2-(6-amino-1*H*-indol-1-yl)acetonitrile (0.725 g, 100%) as a brown solid: ¹H NMR (500 MHz, CDCl₃) δ 7.40 (m, 1H), 6.87 (d, *J* = 3.3 Hz, 1H), 6.62 (m, 2H), 6.46 (d, *J* = 3.3 Hz, 1H), 4.87 (s, 2H), 3.74 (br s, 2H); ESI MS *m*/*z* 172 [M + H]⁺.

### Step C: Synthesis of 2-(6-(6-methoxy-1-oxoisoindolin-2-yl)-1H-indol-1-yl)acetonitrile

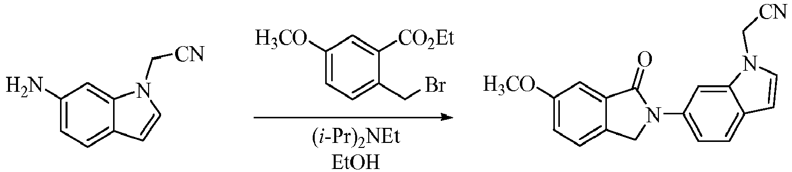

A mixture of 2-(6-amino-1*H-*indol-1-yl)acetonitrile (0.150 g, 0.878 mmol), ethyl 2-(bromomethyl)-5-methoxybenzoate (0.200 g, 0.732 mmol), diisopropylethylamine (0.095 g, 0.732 mmol) in ethanol (8 mL) was heated in a sealed tube at 110 °C overnight. After cooling to room temperature, the mixture was concentrated. The residue was purified by chromatography (silica, 0-10% ethyl acetate in dichloromethane) to give 2-(6-(6-methoxy-1-oxoisoindolin-2-yl)-1*H-*indol-1-yl)acetonitrile (0.057 g, 24%) as a white solid: mp 218-220 °C; ¹H NMR (500 MHz, CDCl₃) δ 8.26 (d, *J*= 0.8 Hz, 1H), 7.67 (d, *J*= 8.6 Hz, 1H), 7.43-7.37 (m, 3H), 7.17 (dd, *J* = 8.3, 2.4 Hz, 1H), 7.13 (d, *J* = 3.3 Hz, 1H), 6.60 (dd, *J* = 3.3, 0.7 Hz, 1H), 5.05 (s, 2H), 4.89 (s, 2H), 3.90 (s, 3H); ESI MS *m*/*z* 318 [M + H]⁺.

### EXAMPLE 23

This example illustrates a preparation of 2-(1-(2-aminoethyl)-1*H-*indol-6-yl)-6-methoxyisoindolin-1-one hydrochloride in an embodiment of the invention.

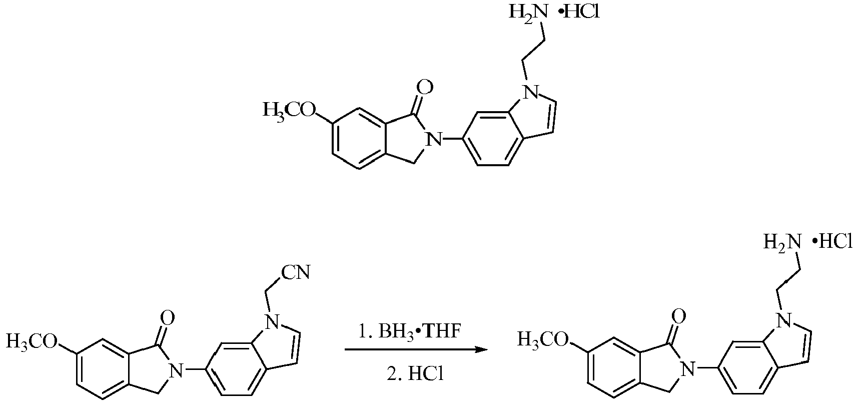

To a solution of 2-(6-(6-methoxy-1-oxoisoindolin-2-yl)-1*H*-indol-1-yl)acetonitrile (0.164 g, 0.517 mmol) in tetrahydrofuran (15 mL) was added boran tetrahydrofuran complex solution (1 M, 2.55 mL, 2.55 mmol) at 0 °C. The mixture was stirred at room temperature for 5 h and then quenched with saturated aqueous sodium bicarbonate at 0 °C. The mixture was extracted with dichloromethane and the combined organic layers dried over sodium sulfate, filtered, and concentrated. The residue was purified by chromatography (silica, 0-10% methanol in dichloromethane) to give 2-(1-(2-aminoethyl)-1H-indol-6-yl)-6-methoxyisoindolin-1-one (0.076 g, 45%) as a thick colorless oil. This material was dissolved in methanol (5 mL) and hydrochloric acid (2 M, 118 µL) was added. The mixture was evaporated and dried under vacuum to afford 2-(1-(2-aminoethyl)-1*H-*indol-6-yl)-6-methoxyisoindolin-1-one hydrochloride (0.088 g, 100%) as an off-white solid: mp 280-282 °C dec; ¹H NMR (500 MHz, DMSO-*d*₆) *δ* 8.11 (br s, 3H), 8.00 (s, 1H), 7.68-7.57 (m, 3H), 7.42 (d, *J*= 3.1 Hz, 1H), 7.25 (m, 2H), 6.50 (d, *J*= 3.1 Hz, 1H), 5.03 (s, 2H), 4.46 (t, *J*= 6.6 Hz, 2H), 3.86 (s, 3H), 3.24 (br s, 2H); ESI MS 322 *m*/*z* [M + H]⁺.

### EXAMPLE 24

This example illustrates a preparation of 2-(1-methyl-1H-indol-6-yl)-6-(pyridin-3-yl)isoindolin-1-one in an embodiment of the invention.

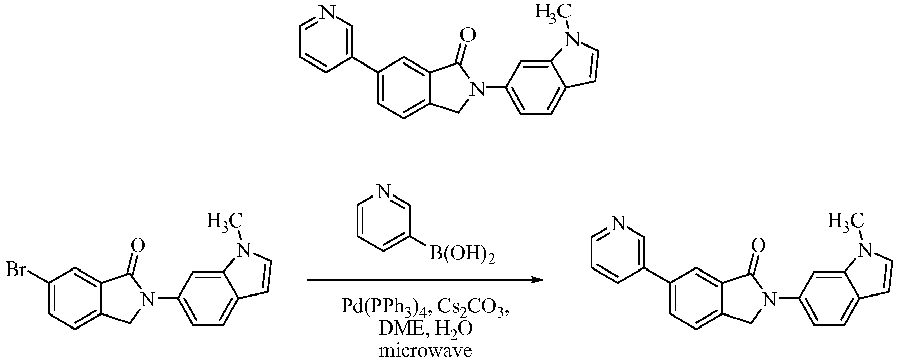

A mixture of 6-bromo-2-(1-methyl-1*H-*indol-6-yl)isoindoline-1-one (0.120 g, 0.35 mmol), pyridin-3-ylboronic acid (0.173 g, 1.41 mmol), tetrakis(triphenylphosphine)palladium (0) (0.081 g, 0.070 mmol), and cesium carbonate (0.344 g, 1.05 mmol) in 5:2 DME/water (7 mL) was heated in a microwave reactor for 30 min at 120 °C. The mixture was cooled to room temperature and diluted with 95:5 methylene chloride/methanol. The reaction mixture was filtered through diatomaceous earth and the water layer separated. The organic phase was concentrated and purified by flash chromatography (silica, gradient 0-4%, methanol/methylene chloride) to afford 2-(1-methyl-1H-indol-6-yl)-6-(pyridin-3-yl)isoindolin-1-one (0.098 g, 82%) as an off-white solid: mp 187-188 °C; ¹H NMR (500 MHz, CDCl₃) δ 8.93 (dd, *J*= 2.3, 0.6 Hz, 1H), 8.65 (dd, *J*= 4.8, 1.6 Hz, 1H), 8.16 (s, 2H), 7.98-7.95 (m, 1H), 7.82 (dd, *J*= 7.8, 1.7 Hz, 1H), 7.66 (d, *J*= 8.5 Hz, 1H), 7.65 (dd, *J*= 7.2, 0.4 Hz, 1H), 7.43-7.41 (m, 1H), 7.32 (dd, *J* = 8.5, 2.0 Hz, 1H), 7.09 (d, *J* = 3.1 Hz, 1H), 6.49 (dd, *J* = 3.0, 0.7 Hz, 1H), 5.01 (s, 2H), 3.83 (s, 3H); ESI MS *m*/*z* 340 [M+ H]⁺.

### EXAMPLE 25

This example illustrates a preparation of 2-(benzo[b]thiophen-5-yl)-5-methoxyisoindolin-1-one in an embodiment of the invention.

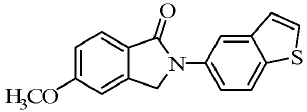

### Step A: Synthesis of ethyl 4-methoxy-2-methylbenzoate as an intermediate

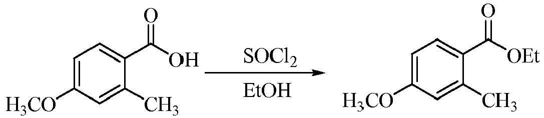

A mixture of 4-methoxy-2-methylbenzoic acid (2.00g, 12.0 mmol) in ethanol (50 mL) was cooled to 0 °C with an ice bath, and thionyl chloride (4.4 mL, 60.0 mmol) was added dropwise over 30 min. The mixture was warmed to room temperature for 1 h, then transferred to an oil bath, and heated at 70 °C overnight. The mixture was cooled to room temperature and concentrated under reduced pressure. The solids obtained were redissolved in diethyl ether (50 mL) and washed with 1 N sodium hydroxide (50 mL), brine (50 mL), dried over magnesium sulfate, and concentrated under reduced pressure. Purification by flash chromatography (silica, 1:20 ethyl acetate/hexanes) afforded ethyl 4-methoxy-2-methylbenzoate (2.24 g, 95%) as a clear oil.

### Step B: Synthesis of ethyl 2-(bromomethyl)-4-methoxybenzoate as an intermediate

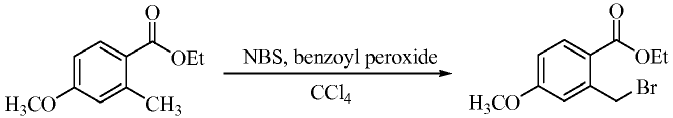

A mixture of ethyl 4-methoxy-2-methylbenzoate (2.24 g, 11.5 mmol) in carbon tetrachloride (20 mL) was heated to 78 °C in an oil bath then a mixture of N-bromosuccinimide (2.26 g, 12.6 mmol) and benzoyl peroxide (0.28 g, 1.15 mmol) was added portionwise over 4 h. The mixture was stirred overnight at 78 °C. After this time, the reaction mixture was cooled to room temperature and the solids removed by filtration. The filtrate was concentrated under reduced pressure and purification by flash chromatography (silica, 1:50 ethyl acetate/hexanes) afforded ethyl 2-(bromomethyl)-4-methoxybenzoate (1.60 g, 52%) as a clear oil: ¹H NMR (300 MHz, CDCl₃) δ 7.99 (d, *J*= 8.7 Hz, 1H), 6.96 (s, 1H), 6.86 (dd, *J* = 8.7, 2.6, 1H), 4.96 (s, 2H), 4.37 (q, *J* = 7.1, 2H), 3.86 (s, 3H), 1.41 (t, *J* = 7.1 Hz, 3H); ESI MS *m*/*z* 273 [M+ H]⁺.

### Step C: Synthesis of 2-(benzo[b]thiophen-5-yl)-5-methoxyisoindolin-1-one

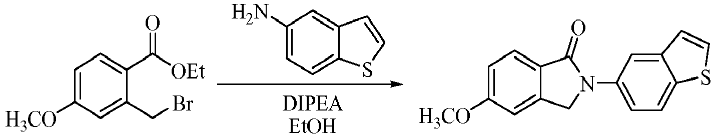

A mixture of benzo[*b*]thiophen-5-amine (0.163 g, 1.10 mmol), *N,N-*diisopropylethyl amine (0.255 mL, 1.46 mmol) in ethanol (30 mL) was stirred for 10 min in a sealed tube at room temperature. Ethyl 2-(bromomethyl)-5-methoxybenzoate (0.200 g, 0.73 mmol) was added portionwise over 3 h, and the mixture was heated to 100 °C overnight. The mixture was cooled to room temperature, LiOH•H₂O was added as a solution in water (2 mL), and the mixture was stirred overnight at room temperature. The mixture was concentrated under reduced pressure and purification by flash chromatography (silica, 1:3 ethylacetate/hexanes) afforded 2-(benzo[*b*]thiophen -5-yl)-5-methoxyisoindolin-1-one (0.063 g, 29%) as an off-white solid: mp 183-186 °C; ¹H NMR (300 MHz, CDCl₃) δ 8.27 (s, 1H), 7.88 (m, 2H), 7.84 (s, 1H), 7.48 (d, *J*= 5.4 Hz, 1H), 7.35 (d, *J*= 5.4 Hz, 1H), 7.05 (d, *J*= 2.1 Hz, 1H), 7.02 (m, 1H), 4.88 (s, 2H), 3.91 (s, 3H); APCI MS *m*/*z* 296 [M + H]⁺.

### EXAMPLE 26

This example illustrates a preparation of 2-(benzo[b]thiophen-5-yl)-6-hydroxyisoindolin-1-one in an embodiment of the invention.

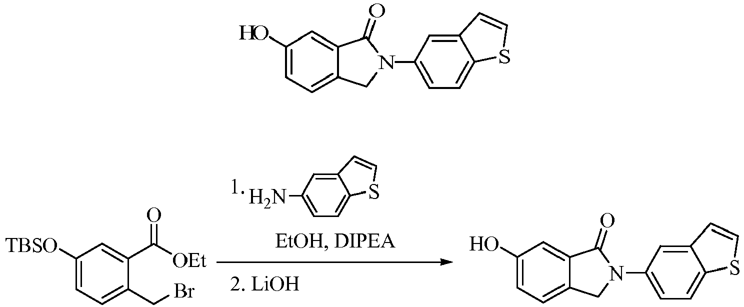

A mixture of ethyl 2-(bromomethyl)-5-(*tert*-butyldimethylsilyloxy)benzoate (1.69 g, 4.50 mmol), benzo[*b*]thiophen-5-amine (0.742 g, 4.97 mmol) and *N,N-*diisopropylethylamine (0.866 mL, 4.97 mmol) in ethanol (70 mL) was heated in a sealed tube at 80 °C overnight. The mixture was cooled to room temperature and lithium hydroxide (0.556 g, 13.5 mmol) in water (2 mL) was added. The resulting mixture was stirred for 3 h and then concentrated under reduced pressure. Purification by flash chromatography (silica, gradient 1-5%,methanol/methylene chloride) afforded 2-(benzo[*b*]thiophen-5-yl)-6-hydroxyisoindolin-1-one (0.319 g, 25% over 2 steps) as an off-white solid: mp 226-228 °C; ¹H NMR (300 MHz, CDCl₃ spiked with CD₃OD) δ 8.22 (s, 1H), 7.93 (d, *J*= 8.4 Hz, 1H), 7.81 (d, *J*= 8.8 Hz, 1H), 7.53-7.52 (m, 1H), 7.41-7.26 (m, 3H), 7.13 (d, *J*= 8.2 Hz, 1H), 4.89 (s, 2H), 3.42 (b s, 1H); ESI MS *m*/*z* 282 [M + H]⁺.

### EXAMPLE 27

This example illustrates a preparation of 2-(benzo[b]thiophen-5-yl)-6-ethoxyisoindolin-1-one in an embodiment of the invention.

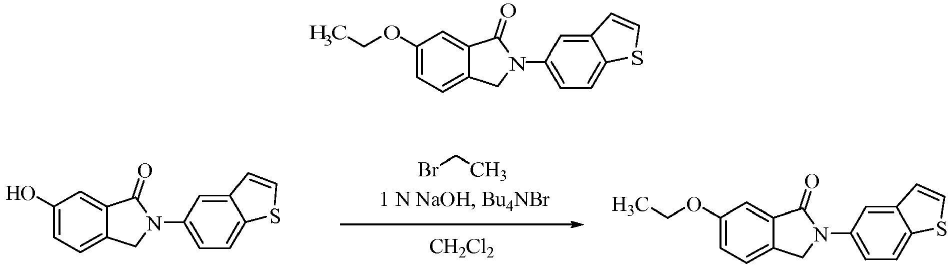

A mixture of 2-(benzo[b]thiophen-5-yl)-6-hydroxyisoindolin-1-one (0.080 g, 0.280 mmol), tetrabutylammonium bromide (0.010 g, 0.028 mmol) and ethyl bromide (0.042 mL, 0.560 mmol) in methylene chloride (1 mL) and 1 N sodium hydroxide (1.5 mL) was vigorously stirred at room temperature overnight. The mixture was partitioned with methylene chloride and water. The organic layer was separated, dried over sodium sulfate, filtered, and concentrated under reduced pressure. Purification by flash chromatography (silica, gradient 1-5%, methanol/methylene chloride) afforded 2-(benzo[*b*]thiophen-5-yl)-6-ethoxyisoindolin-1-one (0.025 g, 28%) as a white solid: mp 127-129 °C; ¹H NMR (500 MHz, CDCl₃) δ 8.29 (d, *J*= 2.0 Hz, 1H), 7.92-7.86 (m, 2H), 7.49-7.40 (m, 4H), 7.16 (dd, *J*= 2.4, 8.3 Hz, 1H), 4.88 (s, 2H), 4.14 (q, *J*= 7.0 Hz, 2H); 1.46 (t, *J*= 7.0 Hz, 3H); ESI MS *m*/*z* 310 [M + H]⁺.

### EXAMPLE 28

This example illustrates a preparation of 2-(benzo[b]thiophen-6-yl)-6-nitroisoindolin-1-one in an embodiment of the invention.

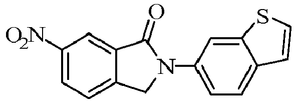

### Step A: Synthesis of (3-bromophenyl)(2,2-diethoxyethyl)sulfane as an intermediate

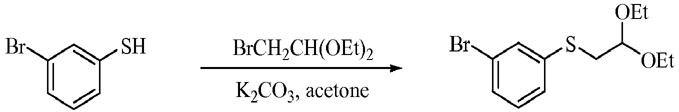

A mixture of 3-bromothiophene (2.52 g, 13.3 mmol), 2-bromo-1,1-diethoxyethane (3.92 g, 19.9 mmol), potassium carbonate (2.75 g, 19.9 mmol), and acetone (10 mL) was stirred at room temperature overnight. After this time, one more equivalent of 2-bromo-1,1-diethoxyethane was added, and the mixture was stirred for another 2 h. The mixture was poured into water and extracted with ethyl acetate. The organic layer was separated, dried over sodium sulfate, filtered, and concentrated. The residue was purified by chromatography (silica, 0-30% ethyl acetate/hexanes) to give (3-bromophenyl)(2,2-diethoxyethyl)sulfane (3.42 g, 84%) as a colorless liquid: ¹H NMR (500 MHz, CDCl₃) δ 7.63 (t, *J* = 1.8 Hz, 1H), 7.29 (m, 2H), 7.12 (d, *J*= 7.9 Hz, 1H), 4.65 (t, *J*= 5.5 Hz, 1H), 3.71-3.65 (m, 2H), 3.58-3.51 (m, 2H), 3.13 (d, *J*= 5.5 Hz, 2H), 1.20 (t, *J*= 7.0 Hz, 6H).

### Step B: Synthesis of 6-bromobenzo[b]thiophene as an intermediate

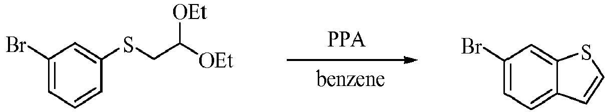

A mixture of (3-bromophenyl)(2,2-diethoxyethyl)sulfane (3.42 g, 11.2 mmol), PPA (1.0 g), and benzene (30 mL) was refluxed for 6 h. After cooling to room temperature, the mixture was poured into water and extracted with ethyl acetate. The organic layer was separated, washed with brine, dried over sodium sulfate, and filtered. Evaporation of the solvents and purification by chromatography (silica, hexanes) gave 6-bromobenzo[*b*]thiophene (0.607 g, 25%) as a white solid: ¹H NMR (500 MHz, CDCl₃) δ 7.63 (d, J= 8.1 Hz, 1H), 7.54-7.47 (m, 3H), 7.19 (t, J= 7.8 Hz, 1H).

### Step C: Synthesis of benzo[b]thiophen-6-amine as an intermediate

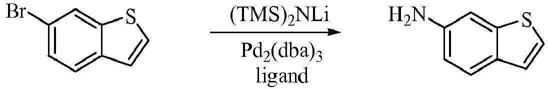

A mixture of 6-bromobenzo[*b*]thiophene (0.607 g, 2.85 mmol), lithium bis(trimethylsilyl)amide (1 M in THF, 4.28 mL, 4.28 mmol) and tris(dibenzylideneacetone)dipalladium chloroform adduct (0.089 g, 0.086 mmol) in tetrahydrofuran (5 mL) was sealed under nitrogen and heated for 5 h. After cooling to room temperature, aqueous 2 N HCl (10 mL) was added, and the mixture was stirred for 30 min. After this time, the mixture was basified with 2 N sodium hydroxide and extracted with ethyl acetate. The organic layer was dried over sodium sulfate, filtered, and concentrated. The residue was purified by chromatography (silica, 0-30% ethyl acetate/hexanes) to afford benzo[*b*]thiophen-6-amine (0.158 g, 37%) as a yellow solid: ¹H NMR (500 MHz, CDCl₃) δ 7.59 (d, *J=* 8.4 Hz, 1H), 7.18-7.12 (m, 3H), 6.76 (dd, *J=* 8.4, 2.1 Hz, 1H), 3.74 (br s, 2H); ESI MS *m*/*z* 150 [M + H]⁺.

### Step D: Synthesis of 2-(benzo[b]thiophen-6-yl)-6-nitroisoindolin-1-one

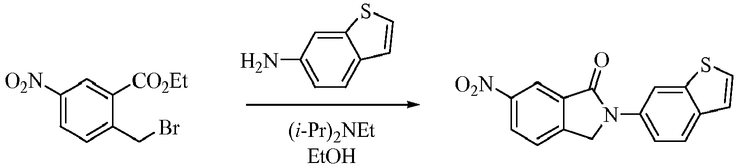

A mixture of benzo[*b*]thiophen-6-amine (0.176 g, 1.18 mmol), ethyl 2-(bromomethyl)-5-nitrobenzoate (0.340 g, 1.18 mmol) and *N,N*-diisopropylethylamine (0.152 g, 1.18 mmol) in ethanol (12 mL) was heated in a sealed tube at 110 °C overnight. After cooling to room temperature, the solid was collected by filtration and washed with ethanol and dichloromethane to give 2-(benzo[*b*]thiophen-6-yl)-6-nitroisoindolin-1-one (0.168 g, 46%) as a gray solid: ¹H NMR (500 MHz, DMSO-*d*₆) δ 8.54 (m, 2H), 8.47 (d, *J=* 2.1 Hz, 1H), 7.98 (m, 3H), 7.76 (d, *J =* 5.4 Hz, 1H), 7.47 (d, *J =* 5.4 Hz, 1H), 5.28 (s, 2H).

### EXAMPLE 29

This example illustrates a preparation of 6-amino-2-(benzo[b]thiophen-6-yl)isoindolin-1-one in an embodiment of the invention.

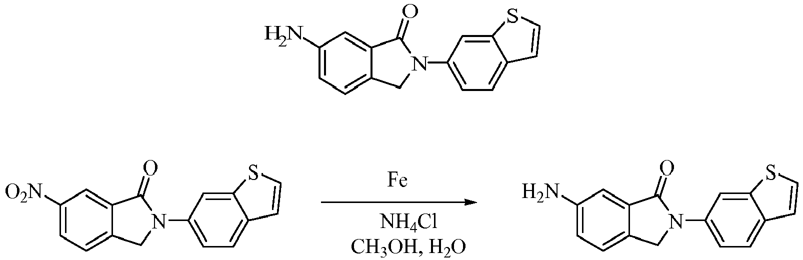

A mixture of 2-(benzo[*b*]thiophen-6-yl)-6-nitroisoindolin-1-one (0.168 g, 0.541 mmol), iron power (0.151 g, 2.70 mmol), ammonium chloride (0.144 g, 2.70 mmol), methanol (20 mL), and water (5 mL) was refluxed for 2 h. After cooling to room temperature, the solids were removed by filtration. The filtrate was evaporated, and the solid obtained was washed with water and dried under vacuum to afford 6-amino-2-(benzo[*b*]thiophen-6-yl)isoindolin-1-one (0.134 g, 88%) as a brown solid: mp 233-236 °C; ¹H NMR (500 MHz, DMSO-*d*₆) δ 8.50 (s, 1H), 7.96 (d, *J=* 7.9Hz, 1H), 7.90 (d, *J=* 8.4 Hz, 1H), 7.69 (d, *J =* 4.3 Hz, 1H), 7.43 (d, *J =* 4.1 Hz, 1H), 7.30 (d, *J =* 7.7 Hz, 1H), 6.94 (s, 1H), 6.89 (d, *J=* 7.1 Hz, 1H), 5.40 (s, 2H), 4.90 (s, 2H); ESI MS *m*/*z* 281 [M + H]⁺.

### EXAMPLE 30

This example illustrates a preparation of 2-(benzo[b]thiophen-6-yl)-6-(dimethylamino)isoindolin-1-one in an embodiment of the invention.

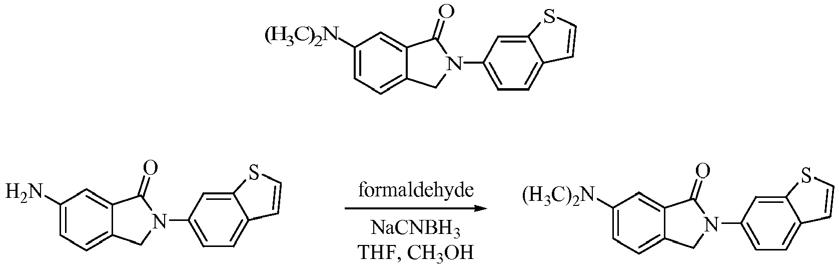

To a mixture of 6-amino-2-(benzo[*b*]thiophen-6-yl)isoindolin-1-one (0.065 g, 0.232 mmol), tetrahydrofuran (10 mL), and methanol (3 mL) was added formaldehyde (37% aqueous, 0.80 mL). After stirring for 20 min, sodium cyanoborohydride (0.146 g, 2.32 mmol) was added, and the mixture was stirred overnight. The mixture was concentrated and the residue purified by chromatography (silica, 0-20% ethyl acetate in 1:1 dichloromethane/hexanes) to give 2-(benzo[*b*]thiophen-6-yl)-6-(dimethylamino)isoindolin-1-one (0.014 g, 15%) as an off-white solid: mp 195-197 °C; ¹H NMR (500 MHz, CDCl₃) δ 8.47 (m, 1H), 7.85 (m, 2H), 7.39 (d, *J* = 5.4 Hz, 1H), 7.36 (d, *J* = 8.3 Hz, 1H), 7.30 (m, 1H), 7.23 (d, *J* = 2.5 Hz, 1H), 6.98 (dd, *J* = 8.4, 2.5 Hz, 1H), 4.84 (s, 2H), 3.04 (s, 6H); ESI MS *m*/*z* 309 [M + H]⁺.

### EXAMPLE 31

This example illustrates a preparation of 6-(dimethylamino)-2-(1-methylindolin-5-yl)isoindolin-1-one in an embodiment of the invention.

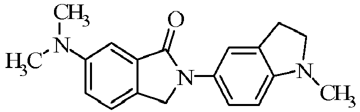

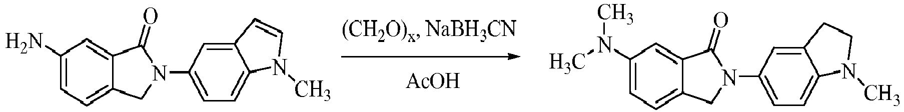

A mixture of 6-amino-2-(1-methyl-1*H*-indol-5-yl)isoindolin-1-one (0.150 g, 0.54 mmol), acetic acid (15 mL), and paraformaldehyde (0.162 g, 5.40 mmol) was strirred for 30 min under a nitrogen atmosphere, then sodium cyanoborohydride (0.170 g, 2.70 mmol) was added. The mixture was stirred overnight at room temperature. After this time, the mixture was poured into 2 N sodium hydroxide (50 mL), and the organic layer was diluted with ethyl acetate (100 mL). The organic layer was dried over sodium sulfate, filtered, concentrated under reduced pressure, and purified by flash chromatography (silica, 2:1 ethyl acetate/hexanes) to afford 6-(dimethylamino)-2-(1-methylindolin-5-yl)isoindolin-1-one (0.060 g, 36%) as a yellow solid: mp 268-270 °C; ¹H NMR (500 MHz, CDCl₃) δ 7.59 (d, *J=* 1.0 Hz, 1H), 7.35-7.33 (m, 2H), 7.21 (d, *J*= 2.4 Hz, 1H), 6.94 (dd, *J*= 8.4, 2.4 Hz, 1H), 6.51 (d, J = 8.4 Hz, 1H), 4.69 (s, 2H), 3.32 (t, *J* = 8.1 Hz, 2H), 3.02 (s, 6H), 2.99 (t, *J* = 8.1 Hz, 2H), 2.77 (s, 3H); ESI MS *m*/*z* 308 [M + H]⁺.

### EXAMPLE 32

This example illustrates a preparation of 6-methoxy-2-(quinolin-6-yl)isoindolin-1-one in an embodiment of the invention.

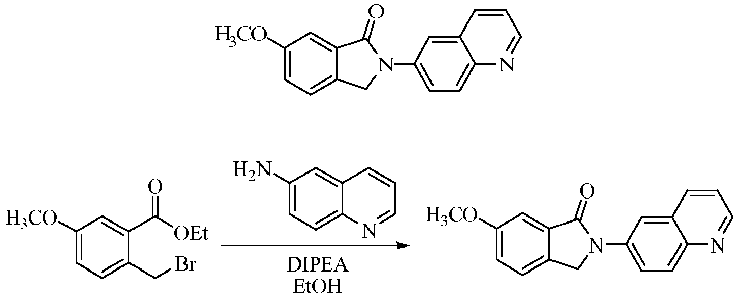

A mixture of quinolin-6-amine (0.528 g, 3.66 mmol), *N,N*-diisopropylethyl amine (0.636 mL, 3.66 mmol) in ethanol (60 mL) was stirred for 10 min in a sealed tube at room temperature. Ethyl 2-(bromomethyl)-5-methoxybenzoate (0.500 g, 1.83 mmol) was added portionwise over 3 h and the mixture was then heated to 100 °C overnight. The mixture was cooled to room temperature, LiOH•H₂O was added as a solution in water (2 mL), and the mixture stirred an additional night at room temperature. The mixture was concentrated under reduced pressure and purification by flash chromatography (silica, 2:1 ethyl acetate/hexanes) afforded 6-methoxy-2-(quinolin-6-yl)isoindolin-1-one (0.177 g, 33%) as a brown solid: mp 194-197 °C; ¹H NMR (300 MHz, CDCl₃) δ 8.87 (dd, *J*=4.2, 1.6 Hz, 1H), 8.35-8.28 (m, 2H), 8.20-8.15 (m, 2H), 7.47-7.40 (m, 3H), 7.20 (dd, *J*= 8.3, 2.6 Hz, 1H), 4.95 (s, 2H), 3.92 (s, 3H); ESI MS *m*/*z* 291 [M + H]⁺.

### EXAMPLE 33

This example illustrates a preparation of 6-(6-methoxy-1-oxoisoindolin-2-yl)quinoline 1-oxide in an embodiment of the invention.

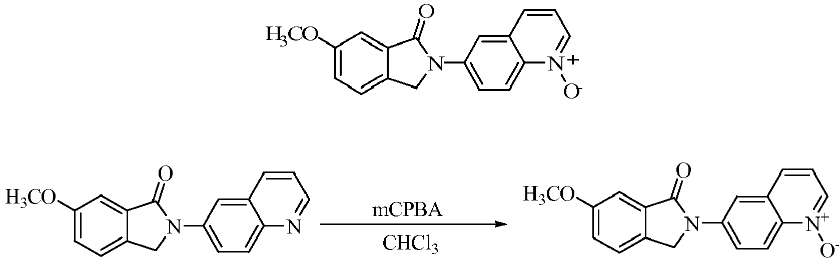

A mixture of 6-methoxy-2-(quinolin-6-yl)isoindolin-1-one (0.050 g, 0.17 mmol) and 3-chlorobenzoperoxoic acid (0.58 g, 0.33 mmol) in chloroform (5 mL) was stirred overnight at room temperature. The mixture was diluted with chloroform and the organics washed with saturated aqueous sodium bicarbonate (50 mL), water (20 mL), dried over magnesium sulfate, and the solvents removed under reduced pressure. The mixture was recrystallized from methanol to afford 6-(6-methoxy-1-oxoisoindolin-2-yl)quinoline 1-oxide (0.025g, 47%) as an off-white solid: mp 251-255 °C; ¹H NMR (500 MHz, DMSO-*d*₆) δ 8.59-8.51 (m, 3H), 8.46 (d, *J*= 2.5 Hz, 1H), 7.92 (d, *J*= 8.5 Hz, 1H), 7.61 (d, *J*= 8.5 Hz, 1H), 7.47 (q, *J*= 6.0 Hz, 1H), 7.30 (m, 2H), 5.10 (s, 2H), 3.87 (s, 3H); APCI MS *m*/*z* 307 [M + H]⁺.

### EXAMPLE 34

This example illustrates a preparation of 6-(6-methoxy-1-oxoisoindolin-2-yl)quinolin-2(1*H*)-one in an embodiment of the invention.

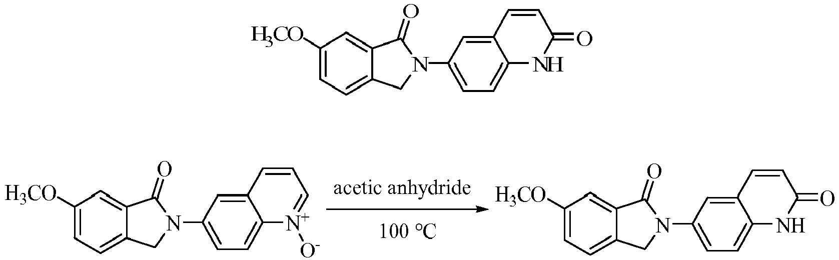

A mixture of 6-(6-methoxy-1-oxoisoindolin-2-yl)quinoline 1-oxide (0.570 g, 1.90 mmol) and acetic anhydride (10 ml) was heated to 100 °C in an oil bath for 18 h. The mixture was cooled to room temperature and water (10 mL) was added. The resulting mixture was stirred at room temperature for 72 h. The reaction mixture was then concentrated under reduced pressure. Purification by flash chromatography (silica, gradient 1-5%, methanol/methylene chloride) afforded 6-(6-methoxy-1-oxoisoindolin-2-yl)quinolin-2(1*H*)-one (0.026 g, 24%) as an off-white solid: mp 305-310°C; ¹H NMR (300 MHz, DMSO-*d*₆) δ 11.79 (s, 1H), 8.11-8.08 (m, 2H), 7.92 (d, *J* = 9.6 Hz, 1H), 7.58 (d, *J*= 7.7 Hz, 1H), 7.36 (d, *J*= 8.4 Hz, 1H), 7.27-7.24 (m, 2H), 6.54 (d, *J*= 9.6 Hz, 1H), 4.97 (s, 2H), 3.86 (s, 3H); ESI MS *m*/*z* 307 [M + H]⁺.

### EXAMPLE 35

This example illustrates a preparation of 5-(6-methoxy-1-oxoisoindolin-2-yl)-1*H-*benzo[*d*]imidazol-2(3*H*)-one in an embodiment of the invention.

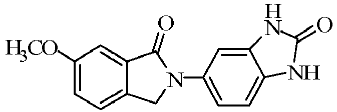

### Step A: Synthesis of 5-amino-1H-benzo[d]imidazol-2(3H)-one as an intermediate

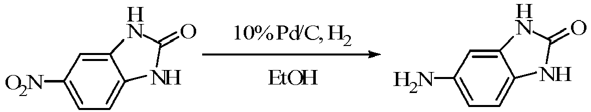

A mixture of 5-nitro-1*H-*benzo[*d*]imidazol-2(3*H*)-one (0.250 g, 1.39 mmol) and 10% Pd/C (0.025 g) in ethanol (5 mL) was shaken under an atmosphere of hydrogen at 35 psi for 3 h. After this time, the mixture was filtered through diatomaceous earth and concentrated under reduced pressure to provide 5-amino-1*H*-benzo[*d*]imidazol-2(3*H*)-one (0.163 g, 78%) as a pink solid: ¹H NMR (300 MHz, CDCl₃) δ 6.75-6.73 (m, 1H), 6.41-6.35 (m, 2H); ESI MS *m*/*z* 150 [M + H]⁺.

### Step B: Preparation of 5-(6-methoxy-1-oxoisoindolin-2-yl)-1H-benzo[d]imidazol-2(3H)-one

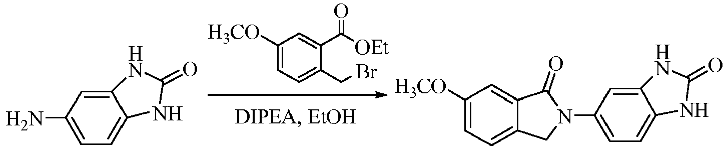

A mixture of ethyl 2-(bromomethyl)-5-methoxybenzoate (0.250 g, 0.910 mmol), 5-amino-1*H*-benzo[*d*]imidazol-2(3*H*)-one (0.163 g, 1.11 mmol) *and N,N-*diisopropylethylamine (0.192 mL, 1.11 mmol) in ethanol (10 mL) was heated in a sealed tube at 110 °C overnight. After this time, the mixture was cooled to room temperature and then for 30 min in an ice-bath. The solid was then collected by filtration to afford 5-(6-methoxy-1-oxoisoindolin-2-yl)-1*H-*benzo[*d*]imidazol-2(3*H*)-one (0.103 g, 38%) as an off-white solid: mp 355-360 °C dec; ¹H NMR (500 MHz, DMSO-*d*₆) δ 10.69 (s, 1H), 10.61 (s, 1H), 7.66-7.66 (m, 1H), 7.54 (d, *J* = 8.3 Hz, 1H), 7.28 (dd, *J* = 8.4, 2.1 Hz, 1H), 7.25-7.21 (m, 2H), 6.96 (d, *J*= 8.5 Hz, 1H), 4.90 (s, 2H), 3.85 (s, 3H); ESI MS *m*/*z* 296 [M + H]⁺.

### EXAMPLE 36

This example illustrates a preparation of 5-(6-methoxy-1-oxoisoindolin-2-yl)-1,3-dimethyl-1*H*-benzo[*d*]imidazol-2(3*H*)-one in an embodiment of the invention.

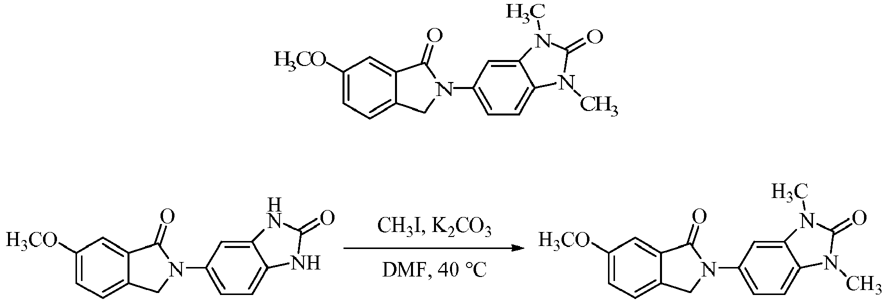

A mixture of 5-(6-methoxy-1-oxoisoindolin-2-yl)-1*H-*benzo[*d*]imidazol-2(3*H*)-one (0.081 g, 0.27 mmol), potassium carbonate (0.150 g, 1.08 mmol) and iodomethane (0.080 mL, 1.20 mmol) in *N,N*-dimethylformamide (3 mL) was heated to 60 °C for 18 h. After this time, the mixture was cooled to room temperature and partitioned between water and ethyl acetate. The organic layer was separated, washed with 5% aqueous lithium chloride, dried over sodium sulfate, filtered, and concentrated under reduced pressure to afford a crude yellow residue. Purification by flash chromatography (silica, gradient 1-50%, ethyl acetate/hexanes) afforded 5-(6-methoxy-1-oxoisoindolin-2-yl)-1,3-dimethyl-1*H-*benzo[*d*]imidazol-2(3*H*)-one (0.013 g, 15%) as an off-white solid: mp 268-270 °C; ¹H NMR (300 MHz, CDCl₃) δ 7.82-7.81 (m, 1H), 7.42-7.40 (m, 2H), 7.28-7.27 (m, 1H), 7.19-7.15 (m, 1H), 6.99 (d, *J*= 8.4 Hz, 1H), 4.83 (s, 2H), 3.90 (s, 3H), 3.46 (s, 3H), 3.44 (s, 3H); ESI MS *m*/*z* 324 [M + H]⁺.

### EXAMPLE 37

This example illustrates a preparation of 6-methoxy-2-(1,2,3,4-tetrahydroquinoxalin-6-yl)isoindolin-1-one in an embodiment of the invention.

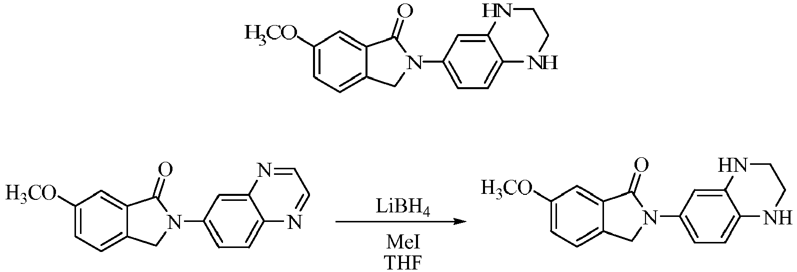

A mixture of lithium borohydride (0.041 g, 1.87 mmol) in anhydrous tetrahydrofuran (5 mL) was degassed under nitrogen then iodomethane (0.106 mL, 1.70 mmol) was added, and the mixture stirred at room temperature for 30 min. After this time, 6-methoxy-2-(quinoxalin-6-yl)isoindolin-1-one (0.160 g, 0.55 mmol) was added as a solution in anhydrous tetrahydrofuran (5 mL) and the mixture was stirred for 2 h. Methanol (5 mL) was then added slowly and the mixture concentrated under reduced pressure. The oil was redissolved in methylene chloride (50 mL) and 2 N sodium hydroxide (50 mL) added. The aqueous layer was extracted with methylene chloride (100 mL) and the combined organics dried over magnesium sulfate and concentrated under reduced pressure. Purification by flash chromatography (silica, 1:99 methanol/methylene chloride) afforded 6-methoxy-2-(1,2,3,4-tetrahydroquinoxalin-6-yl)isoindolin-1-one (0.098 g, 60%) as a yellow solid: mp 159-163 °C; ¹H NMR (500 MHz, CDCl₃) δ 7.37 (d, J= 2.5 Hz, 1H), 7.35 (d, *J*= 8.0 Hz, 1H), 7.19 (d, *J*= 2.0 Hz, 1H), 7.11 (dd, *J* = 2.0, 2.5, 8.0, 8.5 Hz, 1H), 6.77 (dd, *J* = 2.0, 2.5, 8.0, 8.5 Hz, 1H), 6.51 (d, *J*= 8.0 Hz, 1H), 4.69 (s, 2H), 3.88 (s, 3H), 3.80 (bs, 1H), 3.67 (bs, 1H), 3.43 (m, 4H); APCI MS *m*/*z* 296 [M + H]⁺.

### EXAMPLE 38

This example illustrates a preparation of 2-(1,4-dimethyl-1,2,3,4-tetrahydroquinoxalin-6-yl)-6-methoxyisoindolin-1-one in an embodiment of the invention.

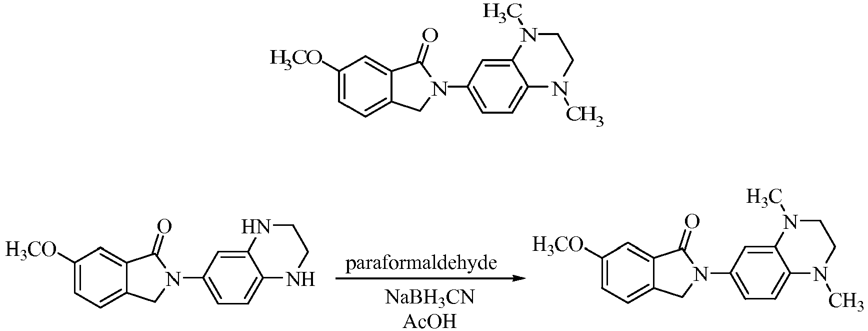

A mixture of 6-methoxy-2-(1,2,3,4-tetrahydroquinoxalin-6-yl)isoindolin-1-one (0.098 g, 0.33 mmol), acetic acid (10 mL), and paraformaldehyde (0.100 g, 3.31 mmol) was strirred for 30 min under a nitrogen atmosphere, then sodium cyanoborohydride (0.104 g, 1.66 mmol) was added. The mixture was stirred overnight at room temperature. The mixture was poured into 2 N sodium hydroxide (50 mL) and the organics extracted with ethyl acetate (100 mL). The combined organics were dried over magnesium sulfate, concentrated under reduced pressure, and purified by flash chromatography (silica, 2:1 ethyl acetate/hexanes) to afford 2-(1,4-dimethyl-1,2,3,4-tetrahydroquinoxalin-6-yl)-6-methoxyisoindolin-1-one (0.077 g, 72%) as a yellow solid: mp 137-142 °C; ¹H NMR (500 MHz, CDCl₃) δ 7.38 (d, *J*= 2.5 Hz, 1H), 7.35 (d, *J* = 8.5 Hz, 1H), 7.23 (s, 1H), 7.11 (dd, *J* = 2.0, 2.5, 8.0, 8.5 Hz, 1H), 6.81 (d, *J*= 7.0 Hz, 1H), 6.52 (d, *J*= 8.5 Hz, 1H), 4.73 (s, 2H), 3.87 (s, 3H), 3.38 (m, 2H), 3.31 (m, 2H), 2.92 (s, 3H), 2.87 (s, 3H); APCI MS *m*/*z* 324 [M + H]⁺.

### EXAMPLE 39

This example illustrates a preparation of 2-(5-isopropylpyridin-2-yl)-6-methoxyisoindolin-1-one in an embodiment of the invention.

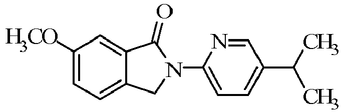

### Step A: Synthesis of 2-nitro-5-(prop-1-en-2-yl)pyridine as an intermediate

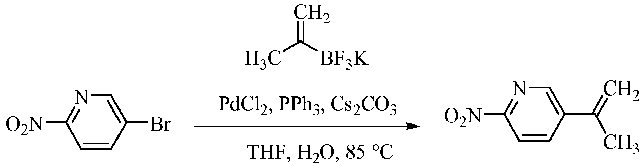

A mixture of 5-bromo-2-nitropyridine (0.219 g, 1.08 mmol), potassium isopropenyltrifluoroborate (0.160 g, 1.08 mmol), palladium (II) chloride (0.0038 g, 0.022 mmol), triphenylphosphine (0.017 g, 0.065 mmol) and cesium carbonate (1.06 g, 3.24 mmol) in 9:1 THF/water (2.16 mL) was heated to 85 °C for 22 h. The mixture was cooled to room temperature, diluted with water (3.3 mL), and extracted three times with methylene chloride. Concentration of the organic layer and purification of the residue obtained by flash chromatography (silica, gradient 0-10%, ethyl acetate/hexanes) afforded 2-nitro-5-(prop-1-en-2-yl)pyridine (0.104 g, 59%) as a white solid: ¹H NMR (500 MHz, CDCl3) δ 8.71(d, *J* = 2.3 Hz, 1H), 8.23 (d, *J* = 8.5 Hz, 1H), 8.03 (dd, *J* = 8.5, 2.3 Hz, 1H), 5.60 (s, 1H), 5.41 (s, 1H), 2.23 (s, 3H); ESI MS *m*/*z* 165 [M+ H]⁺.

### Step B: Synthesis of 5-isopropylpyridin-2-amine as an intermediate

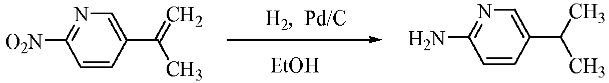

A mixture of 2-nitro-5-(prop-1-en-2-yl)pyridine (0.104 g, 0.63 mmol) and 10% palladium on carbon (0.011 g, 10% by weight) in ethanol (10 mL) was stirred under hydrogen at atmospheric pressure overnight. The reaction was filtered through diatomaceous earth and concentrated under reduced pressure to afford 5-isopropylpyridin-2-amine (0.120 g, 139%) as an oil: ¹H NMR (500 MHz, CDCl₃) δ 7.68 (d, *J*= 8.1 Hz, 1H), 7.51 (s, 1H), 7.48 (s, 2H), 7.14 (d, *J*= 9.1 Hz, 1H), 3.45-3.42 (m, 1H), 1.38 (d, *J*= 8.6 Hz, 6H); ESI MS *m*/*z* 137 [M+ H]⁺.

### Step C: Synthesis of 5 2-(5-isopropylpyridin-2-yl)-6-methoxyisoindolin-1-one

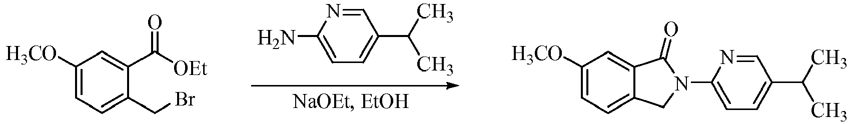

A mixture of ethyl 2-(bromomethyl)-5-methoxybenzoate (0.200 g, 0.73 mmol), 5-isopropylpyridin-2-amine (0.118 g, 0.87 mmol) and sodium ethoxide (0.149 g, 2.19 mmol) in ethanol (15 mL) was heated to 120 °C overnight. The mixture was cooled to room temperature and concentrated. The residue obtained was partitioned between water and ethyl acetate. The organic layer was separated, washed with brine solution, dried over sodium sulfate, filtered, and concentrated. Purification by flash chromatography (silica, gradient 0-20%, ethyl acetate/hexanes) afforded 2-(5-isopropylpyridin-2-yl)-6-methoxyisoindolin-1-one (0.023 g, 9%) as an off-white solid: m.p. 109-110 °C; ¹H NMR (500 MHz, CDCl₃) δ 8.56 (d, *J* = 8.6 Hz, 1H), 8.25 (d, *J* = 2.4 Hz, 1H), 7.64 (dd, *J* = 8.6, 2.4 Hz, 1H), 7.43-7.39 (m, 2H), 7.17 (dd, *J*= 8.3, 2.4 Hz, 1H), 5.03 (s, 2H), 3.89 (s, 3H), 2.97-2.92 (m, 1H), 1.29 (d, *J*= 7.0 Hz, 6H); ESI MS *m*/*z* 283 [M+ H]⁺.

### EXAMPLE 40

This example illustrates a preparation of 6-methoxy-2-(1-methyl-1*H*-indazol-6-yl)isoindolin-1-one in an embodiment of the invention.

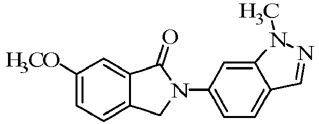

### Step A: Synthesis of 1-methyl-6-nitro-1H-indazole

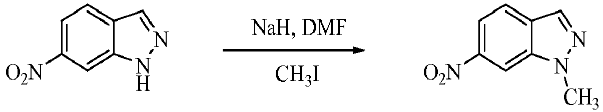

To a solution of 6-nitroindazole (836 mg, 5.12 mmol) in dimethylformamide (10 mL) was added sodium hydride (60% in mineral oil, 246 mg, 6.14 mmol). The mixture was stirred at room temperature for 20 min. Iodomethane (1.09 g, 7.68 mmol) was added, and the mixture was stirred overnight. The contents were poured into water and extracted with ethyl acetate. The organic layer was separated, washed with brine, filtered, and concentrated. Purification of the residue by chromatography (0-40% ethyl acetate in 1:1 dichloromethane/hexanes) gave 1-methyl-6-nitro-1*H-*indazole (0.485 g, 53%) as a yellow solid: ¹H NMR (500 MHz, CDCl₃) δ 8.39 (s, 1H), 8.11 (s, 1H), 8.02 (dd, *J*= 8.8, 1.8 Hz, 1H), 7.84 (d, *J*= 8.8 Hz, 1H), 4.19 (s, 3H);); ESI MS *m*/*z* 178 [M + H]⁺.

### Step B: Synthesis of 1-methyl-1H-indazol-6-amine

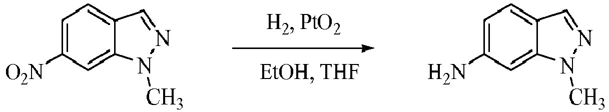

A mixture of 1-methyl-6-nitro-1*H-*indazole (0.480 g, 2.71 mmol), platinum oxide (50 mg), ethanol (20 mL), and tetrahydrofuran (5 mL) was shaken under an atmosphere of hydrogen at 30 psi for 1.5 h. The mixture was filtered and the filtrate evaporated to give 1-methyl-1*H-*indazol-6-amine (0.395 g, 99%) as an off-white solid: ¹H NMR (500 MHz, CDCl₃) δ 7.80 (s, 1H), 7.48 (d, *J* = 8.5 Hz, 1H), 6.57 (dd, *J* = 8.5, 1.8 Hz, 1H), 6.53 (s, 1H), 3.94 (s, 3H), 3.86 (br s, 2H); ESI MS *m*/*z* 148 [M + H]⁺.

### Step C: Synthesis of 6-methoxy-2-(1-methyl-1H-indazol-6-yl)isoindolin-1-one

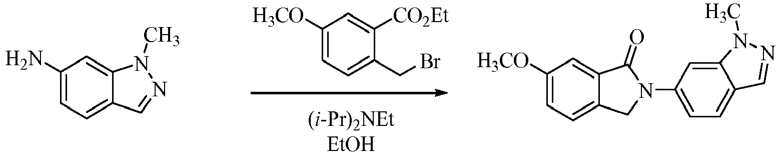

A mixture of 1-methyl-1*H-*indazol-6-amine (0.129 g, 0.878 mmol), ethyl 2-(bromomethyl)-5-methoxybenzoate (0.200 g, 0.732 mmol) and *N,N*-diisopropylethylamine (0.095 g, 0.732 mmol) in ethanol (8 mL) was heated in a sealed tube at 110 °C overnight. After cooling to room temperature, the mixture was concentrated, and the residue was purified by chromatography (silica, 0-50% ethyl acetate in 1:1 dichloromethane/hexanes) to give 6-methoxy-2-(1-methyl-1*H-*indazol-6-yl)isoindolin-1-one (0.045 g, 21%) as a pink solid: mp 175-177 °C; ¹H NMR (500 MHz, CDCl₃) δ 8.22 (s, 1H), 7.95 (s, 1H), 7.75 (d, *J*= 8.7 Hz, 1H), 7.47-7.42 (m, 3H), 7.19 (dd, *J*= 8.3, 2.3 Hz, 1H), 4.91 (s, 2H), 4.10 (s, 3H), 3.91 (s, 3H); ESI MS *m*/*z* 294 [M + H]⁺.

### EXAMPLE 41

This example illustrates a preparation of 6-methoxy-2-(1-methyl-1*H-*pyrrolo[3,2-*b*]pyridin-5-yl)isoindolin-1-one in an embodiment of the invention.

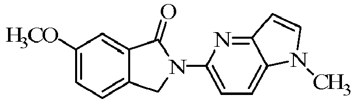

### Step A: Synthesis of (E)-N,N-dimethyl-N'-(1-methyl-1H-pyrrolo[3,2-b]pyridin-5-yl) formimidamide

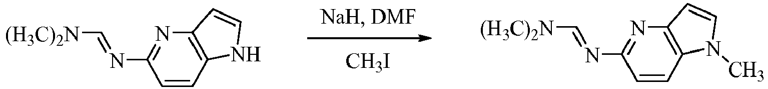

To a mixture of (*E*)-*N,N*-dimethyl-*N*'-(1*H*-pyrrolo[3,2-*b*]pyridin-5-yl)formimidamide (0.700 g, 3.72 mmol) and dimethylformamide (20 mL) was added sodium hydride (60% in mineral oil, 0.178 g, 4.46 mmol) at 0 °C. The mixture was stirred for 10 min and iodomethane (0.792 g, 5.58 mmol) was added at 0 °C and stirred for 2 h at room temperature. The mixture was poured into water and extracted with dichloromethane. The organic layer was dried over sodium sulfate, filtered, and concentrated. The residue was purified by chromatography (silica, 0-10% methanol in dichloromethane containing 0.5% ammonium hydroxide) to afford (*E*)-*N,N*-dimethyl-*N*'-(1-methyl-1*H*-pyrrolo[3,2-*b*]pyridin-5-yl) formimidamide (0.345 g, 46%) as a yellow solid: ¹H NMR (500 MHz, CDCl₃) δ 8.45 (s, 1H), 7.43 (dd, *J* = 8.6, 0.4 Hz, 1H), 7.15 (d, *J =* 3.1 Hz, 1H), 6.94 (d, *J* = 8.6 Hz, 1H), 6.51 (dd, J= 3.1, 0.4 Hz, 1H), 3.77 (s, 3H), 3.11 (s, 6H); ESI MS *m*/*z* 203 [M + H]⁺.

### Step B: Synthesis of 1-methyl-1H-pyrrolo[3,2-b]pyridin-5-amine

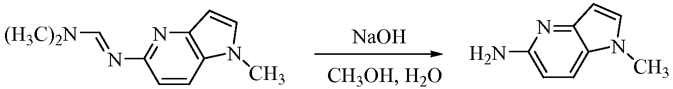

A mixture of (*E*)-*N,N*-dimethyl-*N*'-(1-methyl-1*H*-pyrrolo[3,2-*b*]pyridin-5-yl) formimidamide (0.298 g, 1.47 mmol), sodium hydroxide (2 N, 8 mL), and methanol (20 mL) was refluxed overnight, cooled to room temperature, and concentrated. The residue was poured into brine (30 mL), and the mixture was extracted with dichloromethane (2 × 30 mL). The organic layer was dried over sodium sulfate, filtered, and evaporated to give 1-methyl-1*H*-pyrrolo[3,2-*b*]pyridin-5-amine (0.194 g, 90%) as a yellow solid: ¹H NMR (500 MHz, CDCl₃) δ 7.43 (d, *J*= 8.7 Hz, 1H), 7.08 (d, *J*= 3.1 Hz, 1H), 6.44 (d, *J*= 8.7 Hz, 1H), 6.37 (d, *J*= 3.0 Hz, 1H), 4.27 (br s, 2H), 3.73 (s, 3H); ESI MS *m*/*z* 148 [M + H]⁺.

### Step C: Synthesis of 6-methoxy-2-(1-methyl-1H-pyrrolo[3,2-b]pyridin-5-yl) isoindolin-1-one

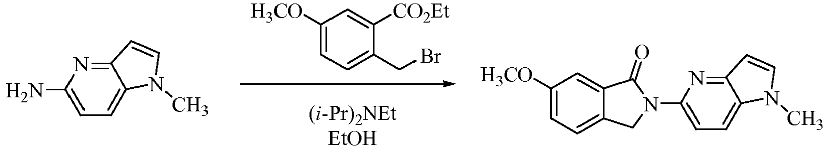

A mixture of 1-methyl-1*H*-pyrrolo[3,2-*b*]pyridin-5-amine (0.129 g, 0.878 mmol), ethyl 2-(bromomethyl)-5-methoxybenzoate (0.200 g, 0.732 mmol) and *N,N-*diisopropylethylamine (0.095 g, 0.732 mmol) in ethanol (8 mL) was heated in a sealed tube at 110 °C for 5 h. After cooling to room temperature, a solution of LiOH•H₂O (0.061 g, 1.46 mmol) in water (1mL) was added, and the mixture was stirred for 2 h. The solid was collected by filtration, washed with ethanol (5 mL), and dried under vacuum to give 6-methoxy-2-(1-methyl-1*H*-pyrrolo[3,2-*b*]pyridin-5-yl)isoindolin-1-one (0.046 g, 21%) as a gray solid: mp 230-234 °C; ¹H NMR (500 MHz, CDCl₃) δ 8.58 (d, *J*= 9.0 Hz, 1H), 7.71 (d, *J* = 9.0 Hz, 1H), 7.43 (m, 2H), 7.26 (d, *J* = 3.1 Hz, 1H), 7.17 (dd, *J* = 8.3, 2.4 Hz, 1H), 6.60 (d, *J*= 3.1 Hz, 1H), 5.17 (s, 2H), 3.90 (s, 3H), 3.84 (s, 3H); ESI MS *m*/*z* 294 [M + H]⁺.

### EXAMPLE 42

This example illustrates a preparation of 6-methoxy-2-(1-methyl-1*H-*pyrrolo[2,3-*c*]pyridin-5-yl)isoindolin-1-one in an embodiment of the invention.

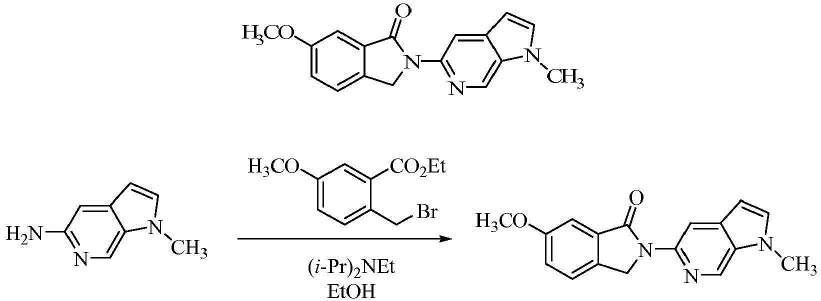

A mixture of 1-methyl-1*H-*pyrrolo[2,3-*c*]pyridin-5-amine (0.129 g, 0.878 mmol), ethyl 2-(bromomethyl)-5-methoxybenzoate (0.200 g, 0.732 mmol) and *N,N-*diisopropylethylamine (0.095 g, 0.732 mmol) in ethanol (8 mL) was heated in a sealed tube at 110 °C overnight. After cooling to room temperature, the mixture was concentrated. The residue was purified by chromatography (silica, 5:95 methanol/dichloromethane containing 0.5% ammonium hydroxide) to afford 6-methoxy-2-(1-methyl-1*H*-pyrrolo[2,3-*c*]pyridin-5-yl)isoindolin-1-one (0.051 g, 23%) as a yellow solid: mp 205-210 °C; ¹H NMR (500 MHz, CDCl₃) δ 8.80 (d, *J*= 0.9 Hz, 1H), 8.52 (s, 1H), 7.42 (m, 2H), 7.19 (d, *J*= 3.0 Hz, 1H), 7.17 (dd, *J*= 8.3, 2.4 Hz, 1H), 6.53 (d, *J*= 2.9 Hz, 1H), 5.11 (s, 2H), 3.90 (s, 3H), 3.89 (s, 3H); ESI MS *m*/*z* 294 [M + H]⁺.

### EXAMPLE 43

This example illustrates a preparation of 6-methoxy-2-(1-methyl-1*H-*pyrrolo[2,3-*b*]pyridin-5-yl)isoindolin-1-one in an embodiment of the invention.

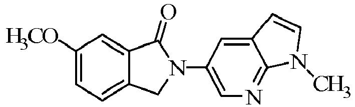

### Step A: Synthesis of 1-methyl-5-nitro-7-azaindole

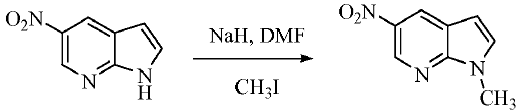

A solution of 5-nitro-7-azaindole (0.195 g, 1.2 mmol) in DMF (10 mL) was treated with NaH (60%, 0.057 g, 1.44 mmol), followed by iodomethane (0.204 g, 1.44 mmol). The reaction mixture was stirred at room temperature for 4.5 h, then poured into water (200 mL) and extracted with dichloromethane (3 × 100 mL). The combined organic layer was washed with brine (100 mL), dried over sodium sulfate, and concentrated. The crude product was purified by chromatography (silica, 50:50 ethyl acetate/hexanes) to yield 1-methyl-5-nitro-7-azaindole (0.152 g, 72%) as a yellow solid: ¹H NMR (500 MHz, CDCl₃) δ 9.23 (d, *J* = 2.4 Hz, 1H), 8.76 (d, *J* = 2.4 Hz, 1H), 7.36 (d, *J* = 3.6 Hz, 1H), 6.66 (d, *J* = 3.5 Hz, 1H), 3.96 (s, 3H).

### Step B: Synthesis of 5-amino-1-methyl-7-azaindole

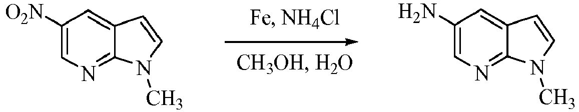

A mixture of 1-methyl-5-nitro-7-azaindole (0.152 g, 0.860 mmol), ammonium chloride (0.230 g, 4.30 mmol), iron powder (0.240 g, 4.30 mmol), methanol (10 mL), and water (10 mL) was heated at reflux for 1 h. After cooling to room temperature, the mixture was filtered and the filtrate extracted with ethyl acetate (3 × 50 mL). The organic layer was washed with brine (80 mL), dried over sodium sulfate, and concentrated. The crude product was purified by chromatography (silica, 50:50 ethyl acetate/hexanes) to give 5-amino-1-methyl-7-azaindole (0.105 g, 83%) as a brown oil: ¹H NMR (300 MHz, CDCl₃) δ 7.93 (d, *J*= 2.5 Hz, 1H), 7.24 (d, *J* = 2.5 Hz, 1H), 7.09 (d, *J* = 3.4 Hz, 1H), 6.26 (d, *J* = 3.3 Hz, 1H), 3.82 (s, 3H), 3.48 (br s, 2H).

### Step C: Synthesis of 6-methoxy-2-(1-methyl-1H-pyrrolo[2,3-b]pyridin-5-yl) isoindolin-1-one

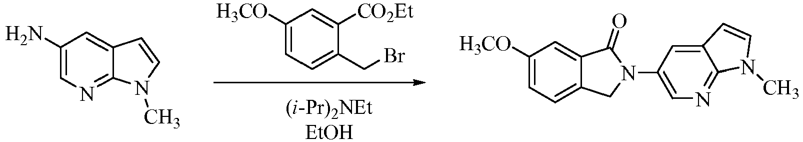

A mixture of ethyl 2-(bromomethyl)-5-methoxybenzoate (0.161 g, 0.590 mmol), 5-amino-1-methyl-7-azaindole (0.105 g, 0.710 mmol), *N,N*-diisopropylethylamine (0.076 g, 0.590 mmol) and ethanol (10 mL) was heated in a sealed tube at 110 °C overnight. After the reaction mixture was cooled to room temperature, lithium hydroxide (1 M, 2 mL) was added and the mixture stirred for 4 h. The solid was collected by filtration, washed with ethanol, and dried to give 6-methoxy-2-(1-methyl-1*H*-pyrrolo[2,3-*b*]pyridin-5-yl)isoindolin-1-one (0.051 g, 29%) as a white solid: mp 198-199 °C; ¹H NMR (300 MHz, CDCl₃) δ 8.64 (d, *J* = 2.3 Hz, 1H), 8.40 (d, *J* = 2.4 Hz, 1H), 7.43 (m, 2H), 7.23 (d, *J* = 3.5 Hz, 1H), 7.17 (dd, *J* = 8.3, 2.5 Hz, 1H), 6.48 (d, *J*= 3.4 Hz, 1H), 4.87 (s, 2H), 3.91 (s, 3H), 3.90 (s, 3H); ESI MS *m*/*z* 294 [M + H]⁺.

### EXAMPLE 44

This example illustrates a preparation of 6-methoxy-2-(1-methyl-1*H-*indazol-5-yl)isoindolin-1-one in an embodiment of the invention.

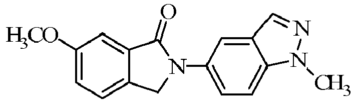

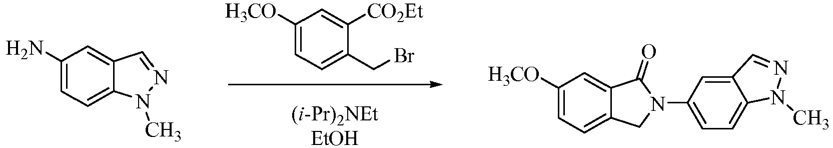

A mixture of ethyl 2-(bromomethyl)-5-methoxybenzoate (0.273 g, 1.0 mmol), 5-amino-1-methyl-indazole (0.177 g, 1.2 mmol) and *N,N*-diisopropylethylamine (0.129 mg, 1.0 mmol) in ethanol (15 mL) was heated in a sealed tube at 110 °C overnight. After the reaction mixture was cooled to room temperature, the solid was collected by filtration, washed with ethanol, and dried to give 6-methoxy-2-(1-methyl-1*H-*indazol-5-yl)isoindolin-1-one (0.109 g, 37%) as a pink solid: mp 213-214 °C; ¹H NMR (300 MHz, CDCl₃) δ 7.98 (m, 3H), 7.47-7.40 (m, 3H), 7.17 (dd, *J* = 8.2, 2.4 Hz, 1H), 4.86 (s, 2H), 4.10 (s, 3H), 3.90 (s, 3H); ESI MS *m*/*z* 294 [M + H]⁺.

### EXAMPLE 45

This example illustrates a preparation of 6-methoxy-2-(1-methyl-1,2,3,4-tetrahydroquinolin-6-yl)isoindolin-1-one in an embodiment of the invention.

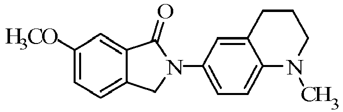

### Step A: Synthesis of 1-methyl-6-nitro-1,2,3,4-tetrahydroquinoline as an intermediate

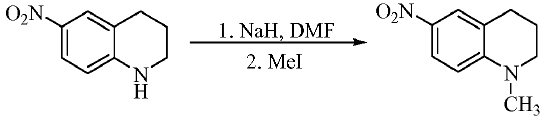

To a mixture of 6-nitro-1,2,3,4-tetrahydroquinoline (0.500 g, 2.81 mmol) in *N,N-*dimethylformamide (20.0 mL) was added sodium hydride (60% in mineral oil, 0.123 g, 3.09 mmol), and the mixture was stirred for 1 h at room temperature. Iodomethane (0.193 mL, 3.09 mmol) was added, and the mixture was stirred at room temperature overnight. Saturated aqueous ammonium chloride (50 mL) was added and the organics extracted with ethyl acetate (50 mL). The organics were washed with 5% aqueous lithium chloride (50 mL), water (50 mL) and brine (50 mL), dried over magnesium sulfate, and concentrated under reduced pressure to afford 1-methyl-6-nitro-1,2,3,4-tetrahydroquinoline (0.416 g, 100%) as an orange solid: ¹H NMR (300 MHz, CDCl₃) δ 7.99 (dd, *J* = 2.7, 9.3 Hz, 1H), 7.85 (m, *J* = 0.9, 1.2, 1.5 Hz, 1H), 6.45 (d, *J*= 9.3 Hz, 1H), 3.41 (t, *J*= 5.7 Hz, 2H), 3.03 (s, 3H), 2.78 (t, *J* = 6.3 Hz, 2H), 1.98 (m, 2H).

### Step B: Synthesis of 1-methyl-1,2,3,4-tetrahydroquinolin-6-amine as an intermediate

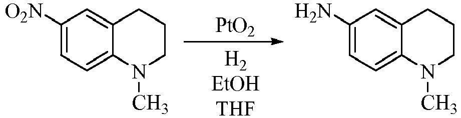

A mixture of 1-methyl-6-nitro-1,2,3,4-tetrahydroquinoline (0.416 g, 2.16 mmol) and 10% palladium on carbon (0.04 g, 10% by weight) in a mixture of ethanol (30 mL) and tetrahydrofuran (5 mL) was shaken under an atmosphere of hydrogen at 40 psi for 4 h. The mixture was filtered through diatomaceous earth and concentrated under reduced pressure. Purification by flash chromatography (silica, 1:3, ethyl acetate/hexanes) afforded 1-methyl-1,2,3,4-tetrahydroquinolin-6-amine (0.308 g, 77%) as a brown oil: ¹H NMR (500 MHz, CDCl₃) δ 6.50 (m, 2H), 6.41 (d, *J* = 1.5 Hz, 1H), 3.24 (bs, 2H), 3.07 (m, *J* = 2.5, 3.0 Hz, 2H), 2.80 (s, 3H), 2.70 (t, *J*= 6.5 Hz, 2H), 1.96 (m, 2H).

### Step C: Synthesis of 6-methoxy-2-(1-methyl-1,2,3,4-tetrahydroquinolin-6-yl)isoindolin-1-one

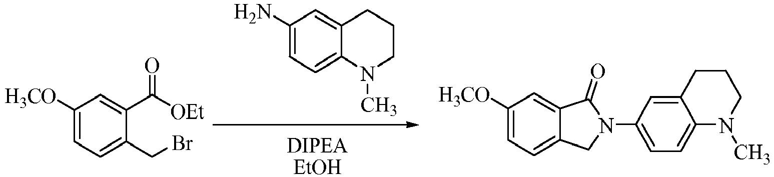

A mixture of 1-methyl-1,2,3,4-tetrahydroquinolin-6-amine (0.304 g, 1.87 mmol) and *N,N*-diisopropylethyl amine (0.350 mL, 1.87 mmol) in ethanol (30 mL) was stirred for 10 min in a sealed tube at room temperature. Ethyl 2-(bromomethyl)-5-methoxybenzoate (0.340 g, 0.1.24 mmol) was added portionwise over 3 h, and the mixture was heated to 100 °C overnight. The mixture was cooled to room temperature, LiOH•H₂Owas added as a solution in water (2 mL), and the mixture was stirred an additional night at room temperature. The mixture was concentrated under reduced pressure and purification by flash chromatography (silica, 1:5 ethyl acetate/hexanes) afforded 6-methoxy-2-(1-methyl-1,2,3,4-tetrahydroquinolin-6-yl)isoindolin-1-one (0.032 g, 8%) as a grey solid: mp 123-127 °C; ¹H NMR (500 MHz, CDCl₃) δ 7.40 (s, 1H), 7.38 (d, *J*= 2.5 Hz, 2H), 7.35 (d, *J*= 8.5 Hz, 1H), 7.11 (dd, *J* = 2.5, 8.5 Hz, 1H), 6.62 (d, *J* = 8.5 Hz, 1H), 4.71 (s, 2H), 3.88 (s, 3H), 3.22 (t, *J* = 5.5, 6.0 Hz, 2H), 2.90 (s, 3H), 2.81 (t, *J* = 6.5 Hz, 2H), 2.00 (m, 2H); APCI MS *m*/*z* 309 [M + H]⁺.

### EXAMPLE 46

This example illustrates a preparation of 2-(2,3-dihydrobenzo[*b*][1,4]dioxin-6-yl)isoindolin-1-one in an embodiment of the invention.

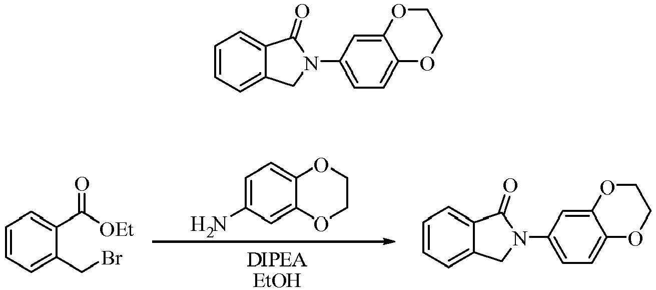

A mixture of 2,3-dihydrobenzo[*b*][1,4]dioxin-6-amine (0.149 g, 0.99 mmol) and *N,N-*diisopropylethyl amine (0.172 mL, 0.99 mmol) in ethanol (30 mL) was stirred for 10 min in a sealed tube at room temperature. Ethyl 2-(bromomethyl) benzoate (0.200 g, 0.82 mmol) was added portionwise over 3 h and the mixture was heated to 100 °C overnight. The mixture was cooled to room temperature, LiOH•H₂O was added as a solution in water (2 mL), and the mixture was stirred overnight at room temperature. The mixture was concentrated under reduced pressure and purification by flash chromatography (silica, 1:5 ethyl acetate/hexanes) afforded 2-(2,3-dihydrobenzo[b][1,4]dioxin-6-yl)isoindolin-1-one (0.111 g, 51%) as a brown solid: mp 170-176 °C; ¹H NMR (500 MHz, CDCl₃₎ δ 7.91 (d, *J* = 8.0 Hz, 1H), 7.58 (t, *J* = 7.5 Hz, 1H), 7.49 (m, 2H), 7.39 (d, *J* = 2.5 Hz, 1H), 7.31 (dd, *J* = 2.5, 8.5 Hz, 1H), 6.91 (d, *J* = 9.0 Hz, 1H), 4.79 (s, 2H), 4.28 (m, 4H); APCI MS *m*/*z* 268 [M +H]⁺.

### EXAMPLE 47

This example illustrates a preparation of 2-(benzo[d]thiazol-6-yl)-6-methoxyisoindolin-1-one in an embodiment of the invention.

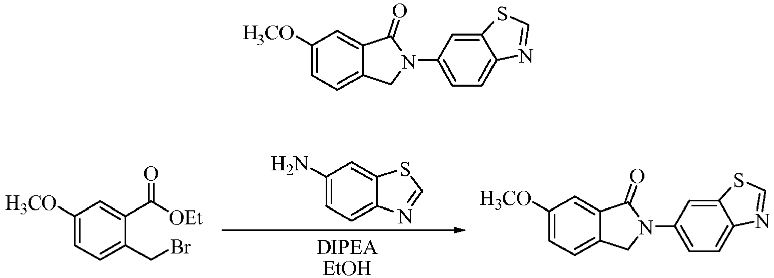

A mixture ofbenzo[d]thiazol-6-amine (0.275 g, 1.83 mmol), *N,N-*diisopropylethyl amine (0.318 mL, 1.83 mmol) in ethanol (30 mL) was stirred for 10 min in a sealed tube at room temperature. Ethyl 2-(bromomethyl)-5-methoxybenzoate (0.250 g, 0.91 mmol) was added portionwise over 3 h, and the mixture was heated to 100 °C overnight. The mixture was cooled to room temperature, LiOH•H₂O was added as a solution in water (2 mL), and the mixture was stirred overnight at room temperature. The solids were collected by filtration and washed with ethanol (10 mL) to afford 2-(benzo[d]thiazol-6-yl)-6-methoxyisoindolin-1-one (0.049 g, 18%) as an off-white solid: mp 204-207 °C; ¹H NMR (500 MHz, CDCl₃) δ 8.95 (s, 1H), 8.74 (d, *J*= 2.5 Hz, 1H), 8.16 (d, *J*= 9.0 Hz, 1H), 7.86 (dd, *J* = 2.5, 9.0 Hz, 1H), 7.43 (d, *J* = 6.5 Hz, 1H), 7.42 (s, 1H), 7.19 (dd, *J* = 2.5, 8.5 Hz, 1H), 4.90 (s, 2H), 3.91 (s, 3H); APCI MS *m*/*z* 297 [M + H]⁺.

### EXAMPLE 48

This example illustrates a preparation of 2-(benzofuran-6-yl)-6-methoxyisoindolin-1-one in an embodiment of the invention.

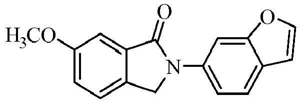

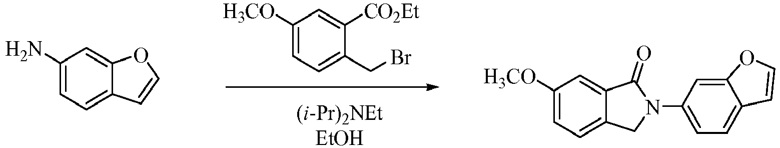

A mixture of ethyl 2-(bromomethyl)-5-methoxybenzoate (0.147 g, 0.538 mmol), 6-aminobenzofuran (0.086 g, 0.645 mmol) and *N,N-*diisopropylethylamine (0.070 g, 0.538 mmol) in ethanol (8 mL) was heated in a sealed tube at 110 °C overnight. After cooling to room temperature, a solution of LiOH•H₂O (0.068 g, 1.61 mmol) in water (1mL) was added, and the mixture was stirred overnight. The solid was collected by filtration, washed with ethanol, and dried to give 2-(benzofuran-6-yl)-6-methoxyisoindolin-1-one (0.046 g, 31%) as a yellow solid: mp 141-143 °C; ¹H NMR (500 MHz, CDCl₃) δ 8.14 (s, 1H), 7.70-7.61 (m, 3H), 7.42 (m, 2H), 7.16 (dd, *J*= 8.3, 2.5 Hz, 1H), 6.76 (m, 1H), 4.86 (s, 2H), 3.90 (s, 3H); ESI MS *m*/*z* 280 [M + H]⁺.

### EXAMPLE 49

This example illustrates a preparation of 6-methoxy-2-(2-methylquinazolin-6-yl)isoindolin-1-one in an embodiment of the invention.

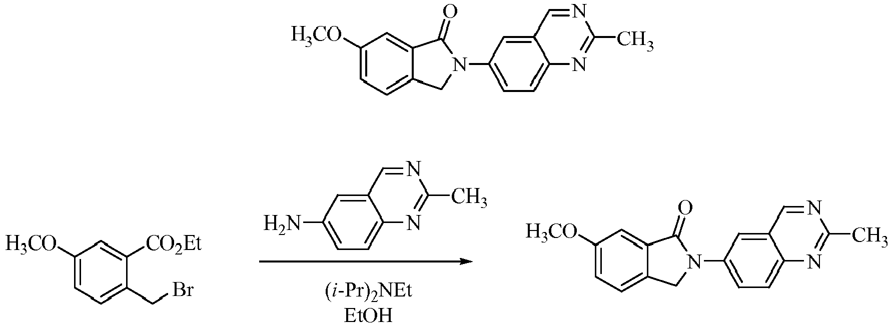

A mixture of ethyl 2-(bromomethyl)-5-methoxybenzoate (0.200 g, 0.732 mmol), 6-amino-2-methylquinazolin (0.140 g, 0.878 mmol), *N,N-*diisopropylethylamine (0.095 g, 0.732 mmol), and ethanol (8 mL) was heated in a sealed tube at 110 °C overnight. After cooling to room temperature, a solution of LiOH•H₂O (0.061 g, 1.46 mmol) in water (1mL) was added, and the mixture was stirred for 6 h. The solid was collected by filtration, washed with ethanol, and dried to give 6-methoxy-2-(2-methylquinazolin-6-yl)isoindolin-1-one (0.100 g, 45%) as an off-white solid: mp 245-247 °C; ¹H NMR (500 MHz, DMSO-*d₆)* δ 9.51 (s, 1H), 8.67 (dd, *J*= 9.2, 2.5 Hz, 1H), 8.47 (d, *J* = 2.5 Hz, 1H), 8.01 (d, *J* = 9.2 Hz, 1H), 7.62 (d, *J* = 8.3 Hz, 1H), 7.29 (m, 2H), 5.10 (s, 2H), 3.87 (s, 3H), 2.77 (s, 3H); ESI MS *m*/*z* 306 [M + H]⁺.

### EXAMPLE 50

This example illustrates a preparation of 6-(difluoromethoxy)-2-(2,3-dihydro-1H-inden-5-yl)isoindolin-1-one in an embodiment of the invention.

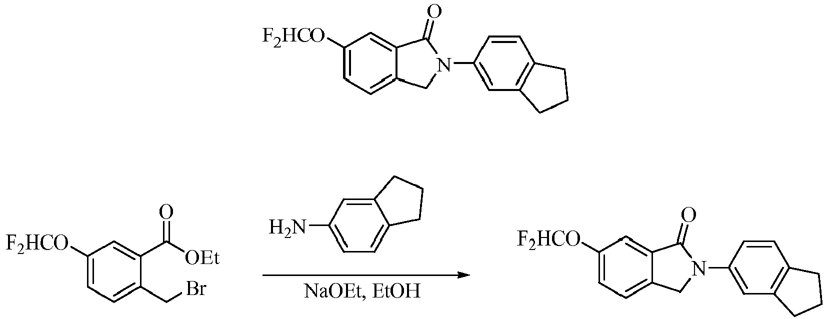

A mixture of ethyl 2-(bromomethyl)-5-(difluoromethoxy)benzoate (0.178 g, 0.58 mmol), 5-aminoindane (0.115 g, 0.86 mmol) and sodium ethoxide (0.118 g, 1.73 mmol) in ethanol (14 mL) was heated in a sealed tube to 120 °C overnight. The mixture was cooled to room temperature and concentrated under vacuum. The mixture was partitioned between 1 N HCl and ethyl acetate. The organic layer was separated and washed with 1 N HCl, then with 1:1 water/brine solution, dried over sodium sulfate, and filtered. The filtrate was concentrated to approximately one-third the original volume and the solid collected by filtration to afford 6-(difluoromethoxy)-2-(2,3-dihydro-1H-inden-5-yl)isoindolin-1-one (0.098 g, 54%) as an off-white solid: mp 137-139 °C; ¹H NMR (500 MHz, CDCl₃) δ 7.71 (s, 1H), 7.66 (d, *J* = 2.1 Hz, 1H), 7.50 (m, 2H), 7.30 (dd, *J* = 8.2, 2.2 Hz, 1H), 7.27 (m, 1H), 6.59 (t, *J* = 73.2 Hz, 1H), 4.83 (s, 2H), 2.96 (t, *J* = 7.4 Hz, 2H), 2.92 (t, *J* = 7.4 Hz, 2H) 2.11 (m, 2H); ESI MS *m*/*z* 316 [M+ H]⁺.

### EXAMPLE 51

This example illustrates a preparation of 6-methoxy-2-(quinolin-7-yl)isoindolin-1-one in an embodiment of the invention.

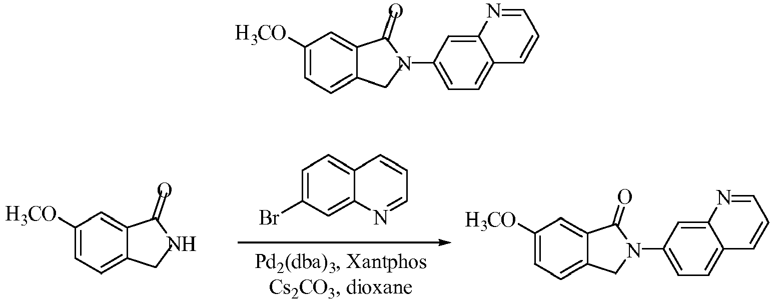

A mixture of 6-methoxyisoindolin-1-one (0.057g, 0.349 mmol), 7-bromoquinoline (0.087 g, 0.419 mmol), tris(dibenzylideneacetone)dipalladium (0.016 g, 0.0174 mmol), Xantphos (0.030 g, 0.0524 mmol), cesium carbonate (0.171 g, 0.524 mmol), and dioxane (4 mL) was heated under nitrogen in a sealed tube at 100 °C overnight. After cooling to room temperature, the mixture was diluted with dichloromethane (5 mL) and filtered. The filtrate was concentrated, and the residue was purified by chromatography (silica, 0-70% ethyl acetate in dichloromethane) to afford 6-methoxy-2-(quinolin-7-yl)isoindolin-1-one (0.086 g, 85%) as an off-white solid: mp 220-223 °C; ¹H NMR (500 MHz, CDCl₃) δ 8.90 (dd, *J*= 3.7, 1.7 Hz, 1H), 8.78 (dd, *J*= 9.0, 2.2 Hz, 1H), 8.15 (dd, *J*= 8.2, 1.2 Hz, 1H), 8.03 (d, *J*= 2.2 Hz, 1H), 7.88 (d, *J* = 9.0 Hz, 1H), 7.44 (m, 2H), 7.36 (dd, *J* = 8.2, 4.3 Hz, 1H), 7.20 (d, *J* = 8.3, 2.5 Hz, 1H), 4.96 (s, 2H), 3.91 (s, 3H); ESI MS *m*/*z* 291 [M + H]⁺.

### EXAMPLE 52

This example demonstrates the ability of compounds of Formaul I to increase SMN expression in cervical carcinoma cell lines in an embodiment of the invention.

As previously discussed, SMN1 is the gene deleted in SMA patients; the SMN2 gene remains intact in both SMA and normal patients. Therefore, SMN2 provides a potential means of increasing SMN production in SMA patients and is, thus, a target for developing SMA treatments. SMN2 is identical to SMN1 with the exception of a single base pair change that results in reduced expression of the gene. The reduction is based on the creation of an alternatively spliced RNA that produces an unstable protein carrying a deletion of exon 7. Both full length SMN and exon 7 deleted SMN are produced by the SMN2 gene via alternative RNA splicing, but the major product is the unstable deleted form.

Cervical carcinoma cell lines were transformed with a SMN2-linked luciferase-reporter gene construct. The reporter is designed to detect shifts in the alternative splicing of SMN2 sequences. In particular, the reporter is constructed as a fusion of the alternatively spliced sequences of the SMN2 gene and the luciferase gene. Luciferase sequences are in the correct translational reading frame only when the SMN2 sequences are spliced according to the normal SMN1 mechanism (e.g., splicing to include exon 7), which allows for translation of the stable SMN protein. Compounds were tested in the assay by administering the compound to the cell line and detecting an increase in the luciferase activity. The assay is described in greater detail in Zhang et al., Gene Ther., 8: 1532-8 (2001). Compounds were administered as described in Lunn et al, Chem. & Biol, 11: 1489-1493 (2004).

The compounds tested and the results are reported in Table 1. The results are reported as a fold-increase over a baseline reading of transformed cells without any test compound. The EC₅₀ value also is reported in Table 1 for some exemplary compounds of the invention.

**Table 1. SMN Splice Reporter Assay**

| **Structure** | **Identifier (ALB-#)** | **EC₅₀ (µM)** | **Fold Increase** |
|---|---|---|---|
| 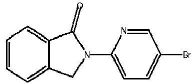 | 112648 | 0.456 | 1.264 |
| 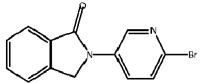 | 112649 | 18.243 | 1.373 |
| 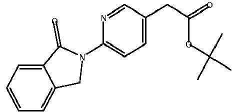 | 112719 | 6.577 | 1.324 |
| 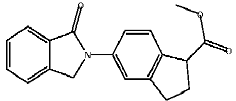 | 113487 | 0.143 | 1.259 |
| 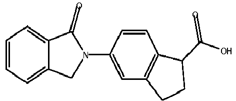 | 113488 | ND | 1.24 |
| 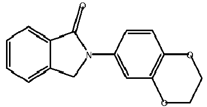 | 116069 | 0.095 | 1.348 |
| 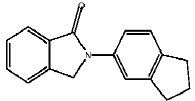 | 116070 | 0.067 | 1.445 |
| 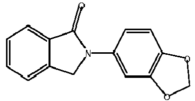 | 116238 | 2.449 | 1.235 |
| 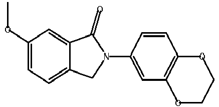 | 116239 | 0.02 | 1.228 |
| 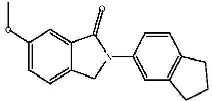 | 116241 | 0.098 | 1.512 |
| 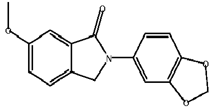 | 116360 | ND | 1.282 |
| 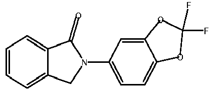 | 116485 | 2.239 | 1.202 |
| 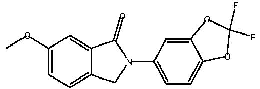 | 116565 | 0.519 | 1.3 |
| 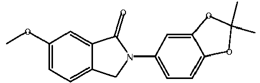 | 116566 | 0.009 | 1.308 |
| 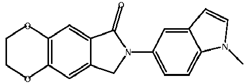 | 118275 | 0.014 | 1.484 |
| 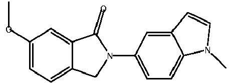 | 118417 | 0.045 | 1.485 |
| 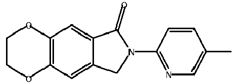 | 118418 | 0.063 | 1.496 |
| 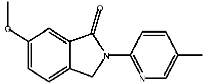 | 118553 | 0.166 | 1.308 |
| 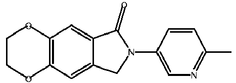 | 118554 | 0.708 | 1.364 |
| 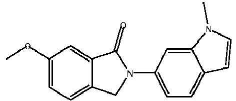 | 118561 | 0.143 | 1.74 |
| 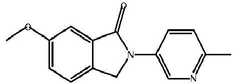 | 118735 | ND | 1.149 |
| 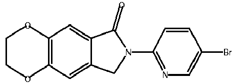 | 118736 | 0.137 | 1.413 |
| 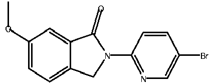 | 118737 | 0.415 | 1.358 |
| 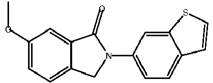 | 118739 | 0.102 | 1.617 |
| 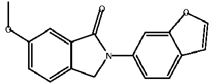 | 118740 | 0.022 | 1.388 |
| 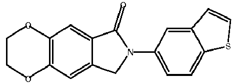 | 118900 | 0.066 | 1.399 |
| 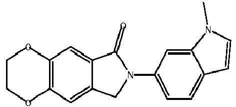 | 118902 | 0.029 | 1.774 |
| 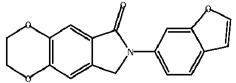 | 118903 | 0.006 | 1.458 |
| 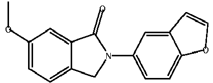 | 119047 | 0.059 | 1.456 |
| 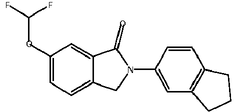 | 119337 | 0.157 | 1.66 |
| 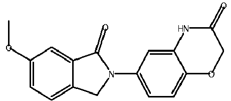 | 119516 | ND | 1.182 |
| 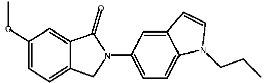 | 119517 | 0.439 | 1.59 |
| 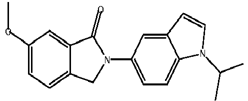 | 119518 | 0.071 | 1.617 |
| 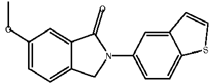 | 119519 | 0.031 | 1.666 |
| 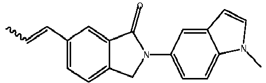 | 119520 | 0.045 | 1.54 |
| 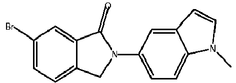 | 119521 | 0.096 | 1.491 |
| 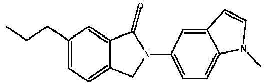 | 119522 | 0.068 | 1.557 |
| 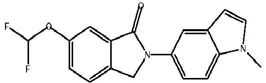 | 119537 | 0.112 | 1.604 |
| 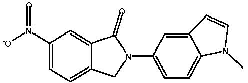 | 119538 | ND | 1.1 |
| 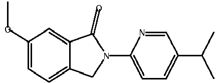 | 119654 | 0.235 | 1.44 |
| 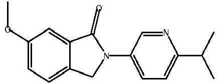 | 119655 | 0.491 | 1.372 |
| 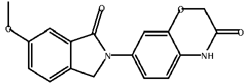 | 119656 | 0.078 | 1.375 |
| 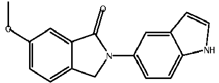 | 119657 | ND | 1.21 |
| 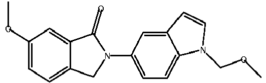 | 119658 | 0.059 | 1.42 |
| 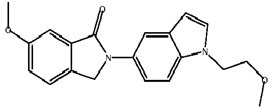 | 119659 | 0.068 | 1.405 |
| 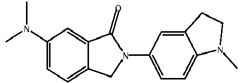 | 119660 | ND | 1.074 |
| 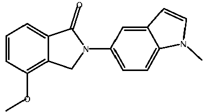 | 119865 | 0.842 | 1.444 |
| 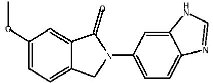 | 119866 | 0.421 | 1.313 |
| 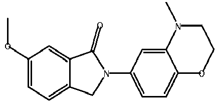 | 119867 | 0.117 | 1.495 |
| 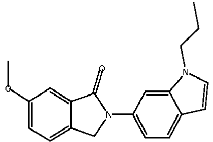 | 119868 | 2.28 | 1.582 |
| 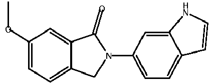 | 119869 | 0.106 | 1.716 |
| 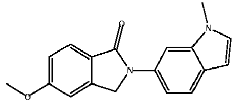 | 119870 | 0.242 | 1.83 |
| 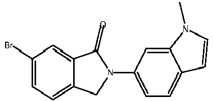 | 120117 | 0.829 | 1.519 |
| 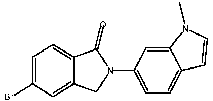 | 120118 | 0.453 | 1.893 |
| 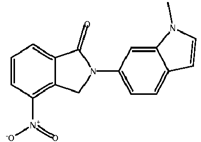 | 120119 | 0.398 | 1.212 |
| 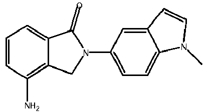 | 120120 | ND | 1.094 |
| 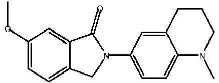 | 120121 | 0.02 | 1.702 |
| 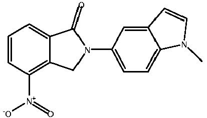 | 120122 | 0.235 | 1.385 |
| 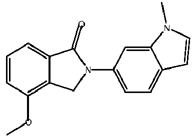 | 120123 | 1.096 | 1.966 |
| 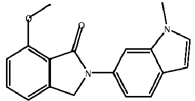 | 120124 | ND | 1.17 |
| 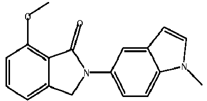 | 120125 | 0.54 | 1.332 |
| 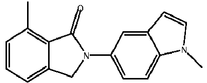 | 120278 | 0.161 | 1.502 |
| 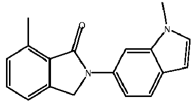 | 120279 | 0.242 | 1.784 |
| 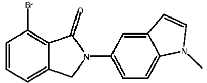 | 120280 | 0.455 | 1.527 |
| 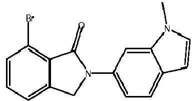 | 120281 | 0.397 | 1.788 |
| 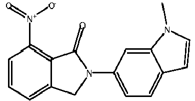 | 120282 | 0.737 | 1.291 |
| 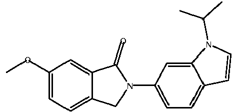 | 120283 | ND | 1.273 |
| 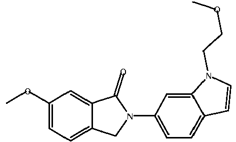 | 120284 | 7.87 | 1.19 |
| 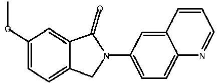 | 120286 | 0.03 | 1.713 |
| 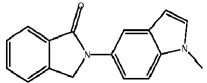 | 120287 | 0.158 | 1.433 |
| 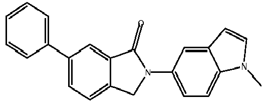 | 120288 | 0.408 | 1.364 |
| 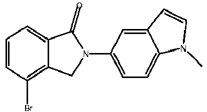 | 120289 | 1.027 | 1.39 |
| 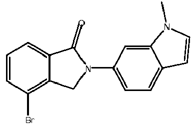 | 120290 | 0.609 | 1.386 |
| 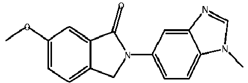 | 120291 | 0.111 | 1.362 |
| 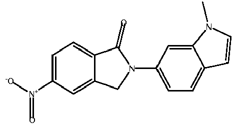 | 120292 | 0.209 | 1.551 |
| 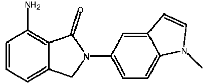 | 120427 | 0.294 | 1.606 |
| 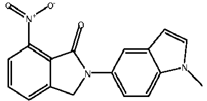 | 120428 | 0.338 | 1.262 |
| 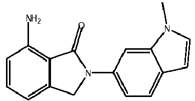 | 120429 | 0.152 | 1.745 |
| 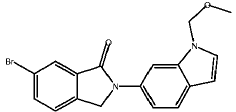 | 120430 | 0.306 | 1.374 |
| 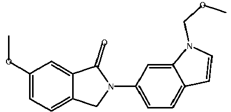 | 120431 | 0.12 | 1.311 |
| 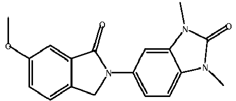 | 120432 | 0.83 | 1.234 |
| 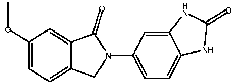 | 120433 | 0.977 | 1.296 |
| 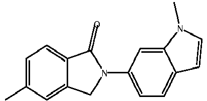 | 120434 | 0.054 | 1.708 |
| 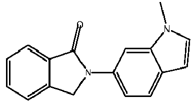 | 120435 | 0.64 | 1.585 |
| 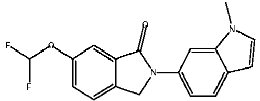 | 120591 | 0.509 | 1.709 |
| 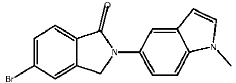 | 120592 | 0.112 | 1.51 |
| 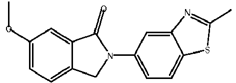 | 120593 | 0.014 | 1.322 |
| 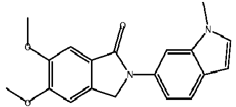 | 120594 | 2.062 | 1.561 |
| 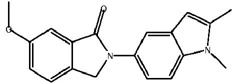 | 120595 | 0.114 | 1.531 |
| 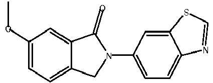 | 120596 | 0.126 | 1.591 |
| 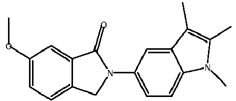 | 120597 | 0.777 | 1.434 |
| 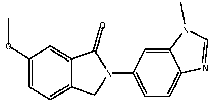 | 120598 | 0.183 | 1.552 |
| 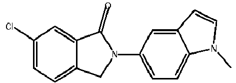 | 120599 | 0.061 | 1.407 |
| 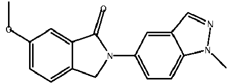 | 120600 | 0.059 | 1.312 |
| 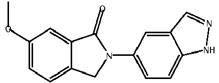 | 120601 | ND | 1.059 |
| 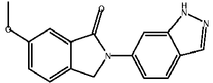 | 120602 | 0.154 | 1.412 |
| 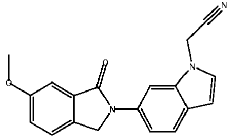 | 120603 | 2.767 | 1.199 |
| 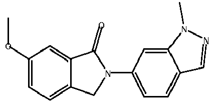 | 120604 | 0.415 | 1.441 |
| 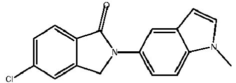 | 120778 | 0.131 | 1.492 |
| 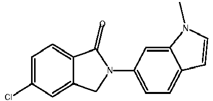 | 120779 | 0.317 | 1.704 |
| 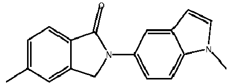 | 120780 | ND | 1.344 |
| 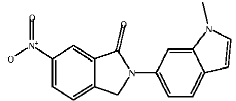 | 120781 | ND | 1.129 |
| 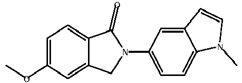 | 120782 | 0.184 | 1.49 |
| 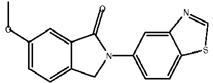 | 120785 | 0.059 | 1.353 |
| 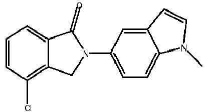 | 120786 | 0.679 | 1.376 |
| 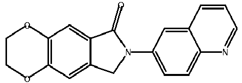 | 120787 | 0.015 | 1.807 |
| 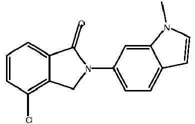 | 120788 | ND | 1.25 |
| 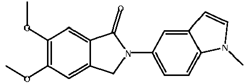 | 120789 | 9.79 | 1.427 |
| 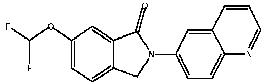 | 120790 | 0.142 | 1.926 |
| 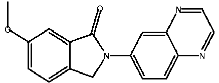 | 120791 | 0.07 | 1.372 |
| 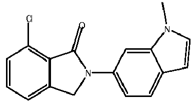 | 120792 | 0.238 | 1.642 |
| 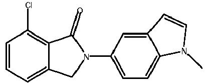 | 120793 | 0.361 | 1.395 |
| 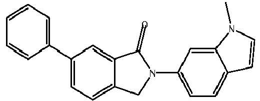 | 120949 | 0.373 | 1.816 |
| 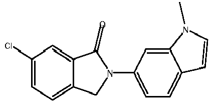 | 120950 | 0.984 | 1.482 |
| 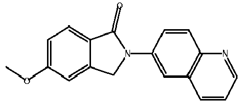 | 120951 | 0.135 | 1.769 |
| 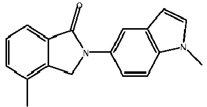 | 120952 | 0.414 | 1.374 |
| 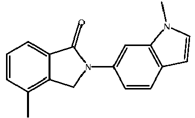 | 120953 | 1.164 | 1.495 |
| 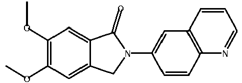 | 120954 | 2.043 | 1.356 |
| 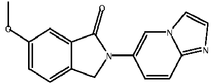 | 120955 | 1.591 | 1.265 |
| 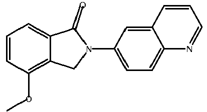 | 120956 | 1.252 | 1.329 |
| 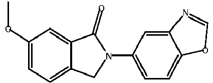 | 120957 | ND | 1.254 |
| 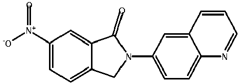 | 120958 | 2.661 | 1.381 |
| 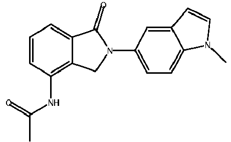 | 121074 | ND | 1.078 |
| 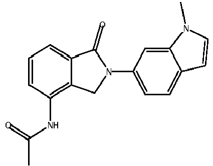 | 121075 | ND | 1.087 |
| 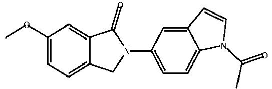 | 121076 | 0.049 | 1.295 |
| 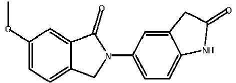 | 121077 | ND | 1.107 |
| 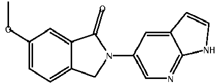 | 121078 | ND | 1.246 |
| 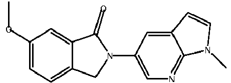 | 121079 | 0.042 | 1.385 |
| 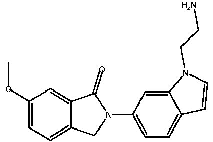 | 121080 | ND | 1.163 |
| 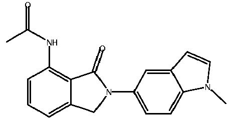 | 121081 | 0.177 | 1.581 |
| 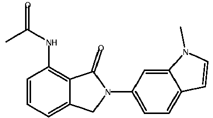 | 121082 | 0.825 | 1.496 |
| 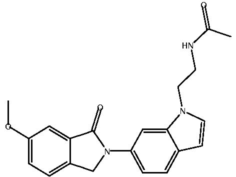 | 121083 | ND | 1.083 |
| 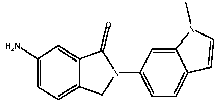 | 121256 | 1.591 | 1.638 |
| 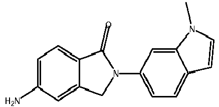 | 121257 | 0.175 | 1.856 |
| 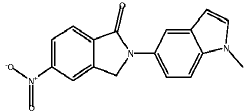 | 121258 | 0.125 | 1.336 |
| 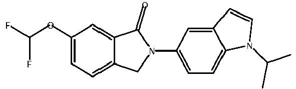 | 121259 | ND | 1.166 |
| 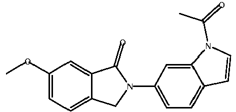 | 121260 | 0.035 | 1.414 |
| 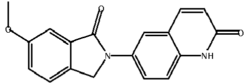 | 121261 | ND | 1.157 |
| 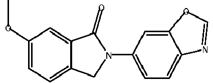 | 121262 | 0.336 | 1.317 |
| 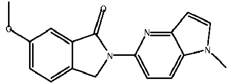 | 121263 | 0.161 | 1.39 |
| 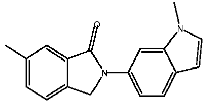 | 121504 | 0.422 | 1.742 |
| 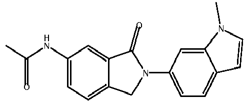 | 121505 | 0.203 | 1.817 |
| 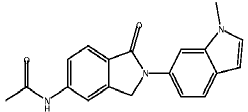 | 121506 | 1.045 | 1.488 |
| 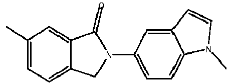 | 121507 | 0.034 | 1.411 |
| 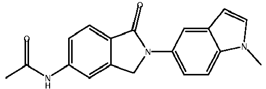 | 121508 | ND | 1.201 |
| 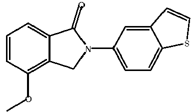 | 121509 | 3.711 | 1.564 |
| 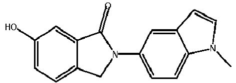 | 121510 | 0.159 | 1.542 |
| 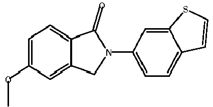 | 121511 | 0.113 | 1.808 |
| 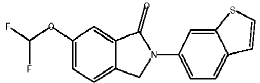 | 121512 | 0.226 | 1.766 |
| 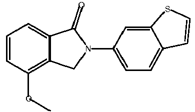 | 121513 | 0.749 | 1.612 |
| 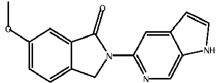 | 121514 | ND | 1.172 |
| 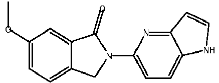 | 121515 | 0.222 | 1.24 |
| 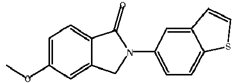 | 121643 | 0.045 | 1.778 |
| 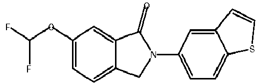 | 121644 | 0.115 | 1.801 |
| 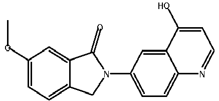 | 121645 | ND | 1.093 |
| 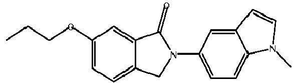 | 121785 | 0.08 | 1.29 |
| 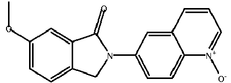 | 121786 | 0.188 | 1.498 |
| 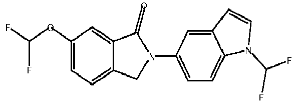 | 121787 | ND | 1.221 |
| 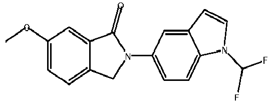 | 121788 | ND | 1.19 |
| 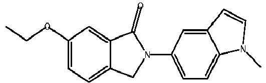 | 121789 | 0.069 | 1.461 |
| 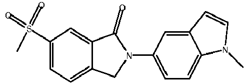 | 121790 | 0.556 | 1.336 |
| 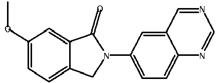 | 121791 | 1.062 | 1.448 |
| 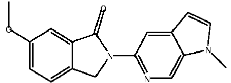 | 121792 | 0.101 | 1.384 |
| 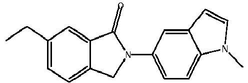 | 121793 | 0.085 | 1.529 |
| 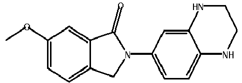 | 121921 | 0.217 | 1.365 |
| 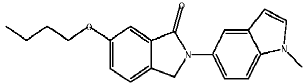 | 121922 | 0.225 | 1.301 |
| 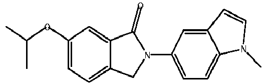 | 121923 | ND | 1.194 |
| 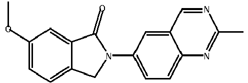 | 121924 | 1.355 | 1.489 |
| 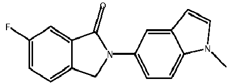 | 121925 | 0.193 | 1.347 |
| 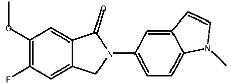 | 121926 | 0.199 | 1.533 |
| 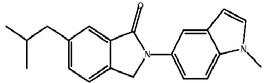 | 121927 | 0.036 | 1.323 |
| 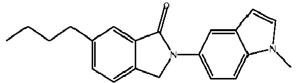 | 121928 | 0.148 | 1.384 |
| 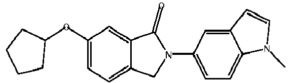 | 121929 | 0.083 | 1.316 |
| 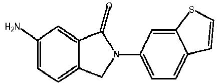 | 122036 | 0.347 | 1.827 |
| 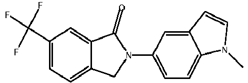 | 122037 | 0.862 | 1.406 |
| 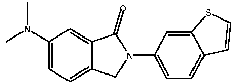 | 122038 | 0.038 | 1.592 |
| 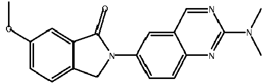 | 122039 | 7.116 | 1.459 |
| 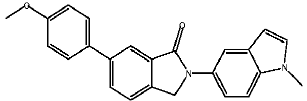 | 122040 | ND | 1.148 |
| 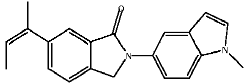 | 122041 | 0.076 | 1.606 |
| 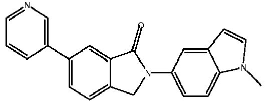 | 122042 | 0.728 | 1.422 |
| 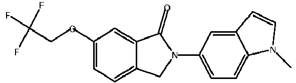 | 122043 | 0.54 | 1.341 |
| 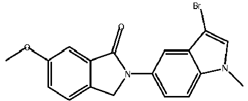 | 122044 | 0.16 | 1.383 |
| 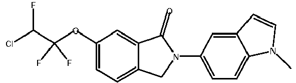 | 122180 | 0.092 | 1.64 |
| 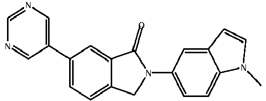 | 122181 | ND | 1.169 |
| 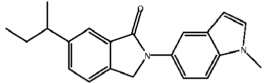 | 122182 | 0.101 | 1.539 |
| 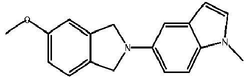 | 122183 | 0.593 | 1.518 |
| 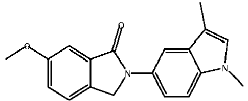 | 122184 | 0.409 | 1.639 |
| 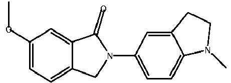 | 122314 | ND | 1.026 |
| 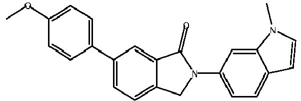 | 122315 | ND | 1.091 |
| 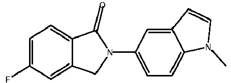 | 122316 | 0.065 | 1.401 |
| 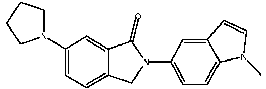 | 122317 | 0.187 | 1.47 |
| 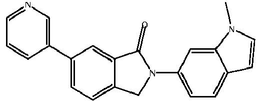 | 122318 | 0.459 | 1.94 |
| 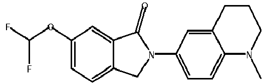 | 122319 | 0.203 | 1.97 |
| 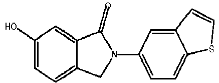 | 122430 | 0.086 | 1.769 |
| 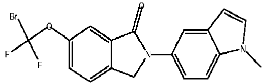 | 122431 | 0.22 | 1.552 |
| 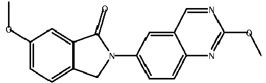 | 122432 | 1.143 | 1.32 |
| 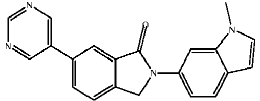 | 122433 | 1.067 | 1.333 |
| 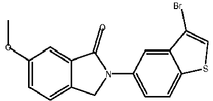 | 122434 | 0.378 | 1.718 |
| 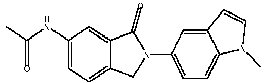 | 122435 | 0.143 | 1.412 |
| 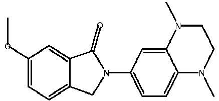 | 122436 | 5.951 | 1.354 |
| 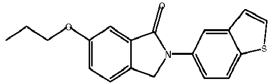 | 122551 | ND | 1.235 |
| 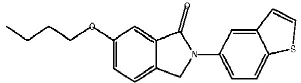 | 122552 | ND | 1.202 |
| 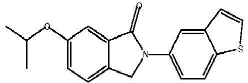 | 122553 | ND | 1.082 |
| 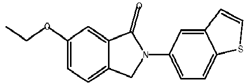 | 122554 | 0.027 | 1.475 |
| 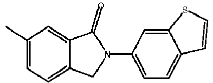 | 122555 | 0.087 | 1.509 |
| 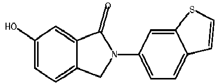 | 122556 | 0.139 | 1.671 |
| 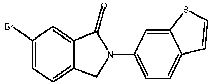 | 122557 | ND | 1.383 |
| 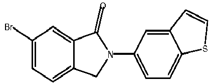 | 122558 | 0.094 | 1.469 |
| 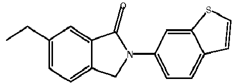 | 122683 | 0.125 | 1.582 |
| 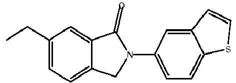 | 122684 | 0.057 | 1.665 |
| 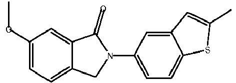 | 122685 | 0.024 | 1.251 |
| 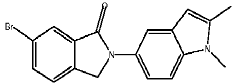 | 122818 | 0.207 | 1.551 |
| 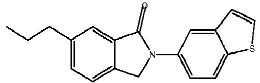 | 122819 | 0.035 | 1.547 |
| 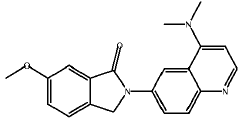 | 122820 | ND | 1.066 |
| 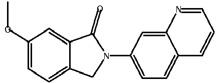 | 122821 | 0.037 | 1.401 |
| 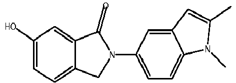 | 123010 | 0.302 | 1.336 |
| 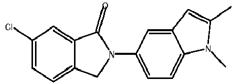 | 123011 | 0.088 | 1.481 |
| 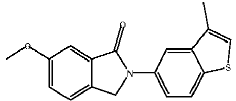 | 123012 | 0.272 | 1.773 |
| 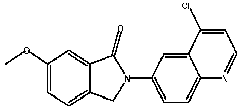 | 123013 | 0.902 | 1.413 |
| 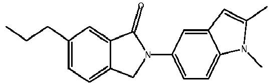 | 123014 | 0.323 | 1.407 |
| 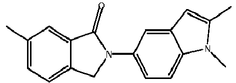 | 123015 | 0.053 | 1.43 |
| 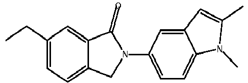 | 123016 | 0.197 | 1.481 |
| 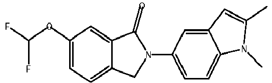 | 123204 | 0.374 | 1.361 |
| 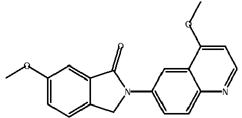 | 123205 | ND | 1.227 |
| 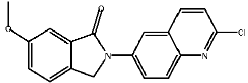 | 123206 | 0.119 | 1.531 |
| 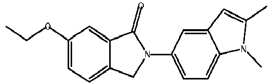 | 123328 | 0.444 | 1.316 |
| 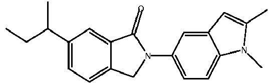 | 123329 | 0.569 | 1.327 |
| 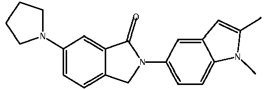 | 123330 | ND | 1.113 |
| 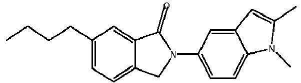 | 123331 | 0.66 | 1.31 |
| 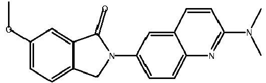 | 123332 | 0.207 | 1.272 |
| 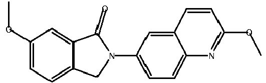 | 123333 | 0.02 | 1.376 |
| 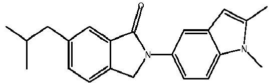 | 123439 | 0.359 | 1.357 |
| 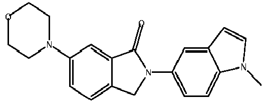 | 123587 | ND | 1.256 |
| 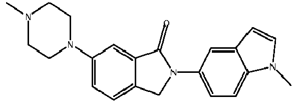 | 123588 | ND | 1.183 |
| 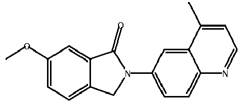 | 123589 | 0.078 | 1.541 |
| 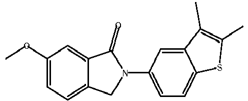 | 123590 | 0.128 | 1.405 |
| 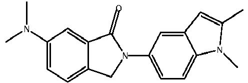 | 123707 | 0.099 | 1.635 |
| 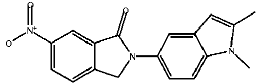 | 123708 | ND | 1.179 |
| 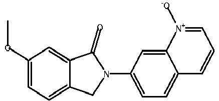 | 123709 | ND | 1.137 |
| 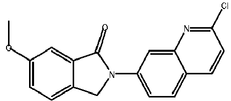 | 123710 | 0.047 | 1.327 |
| 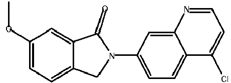 | 123711 | 1.585 | 1.262 |
| 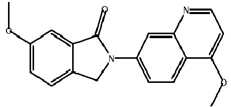 | 123712 | 0.147 | 1.38 |
| 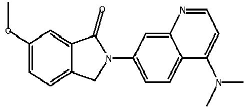 | 123713 | ND | 1.072 |
| 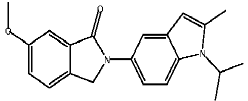 | 123714 | 2.634 | 1.863 |
| 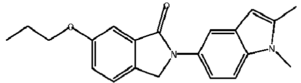 | 123715 | 0.145 | 1.216 |
| 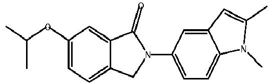 | 123934 | 0.063 | 1.554 |
| 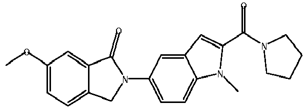 | 123935 | ND | 1.124 |
| 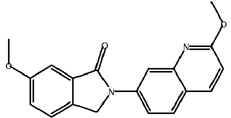 | 123936 | 0.784 | 1.429 |
| 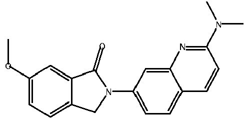 | 123937 | ND | 1.202 |
| 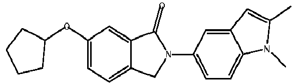 | 123938 | ND | 1.2 |
| 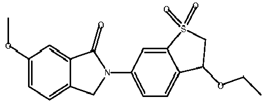 | 124031 | ND | 1.106 |
| 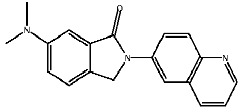 | 124032 | 0.009 | 1.659 |
| 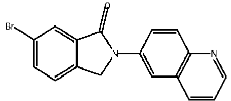 | 124033 | 0.225 | 1.795 |
| 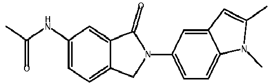 | 124034 | 0.44 | 1.494 |
| 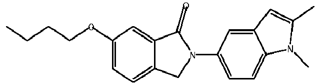 | 124035 | ND | 1.118 |
| 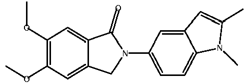 | 124036 | 1.637 | 1.34 |
| 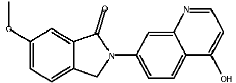 | 124037 | 5.97 | 1.377 |
| 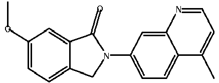 | 124038 | 0.026 | 1.509 |
| 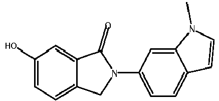 | 124039 | 0.52 | 1.893 |
| 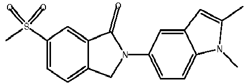 | 124166 | ND | 1.227 |
| 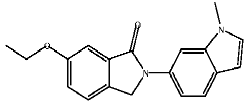 | 124167 | 0.069 | 1.759 |
| 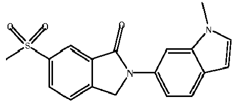 | 124168 | ND | 1.462 |
| 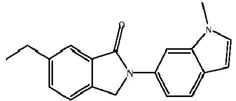 | 124169 | 0.152 | 1.673 |
| 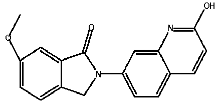 | 124170 | ND | 1.102 |
| 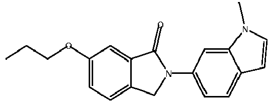 | 124325 | 0.145 | 1.69 |
| 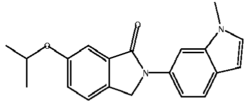 | 124326 | 0.17 | 1.666 |
| 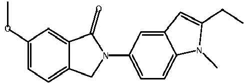 | 124327 | 0.56 | 1.361 |
| 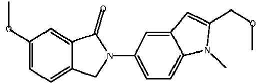 | 124328 | ND | 1.305 |
| 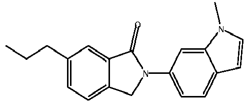 | 124428 | 0.151 | 1.692 |
| 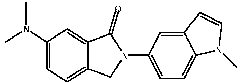 | 124429 | 0.024 | 1.594 |
| 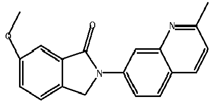 | 124430 | 0.046 | 1.411 |
| 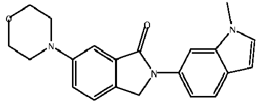 | 124539 | ND | 1.652 |
| 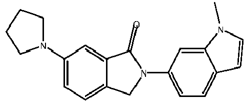 | 124596 | 0.047 | 1.681 |
| 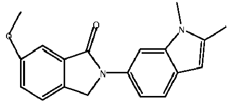 | 124597 | 0.347 | 1.358 |
| 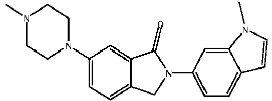 | 124649 | ND | 1.119 |
| 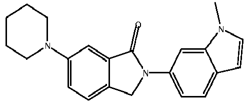 | 124650 | 0.096 | 1.684 |
| 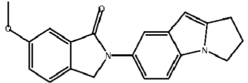 | 124651 | 0.116 | 1.452 |
| 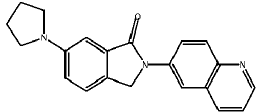 | 124770 | 0.024 | 1.948 |
| 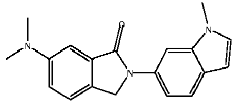 | 124771 | 0.043 | 1.689 |
| 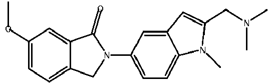 | 124772 | ND | 1.148 |
| 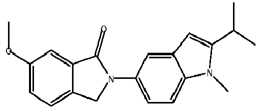 | 124773 | ND | 1.255 |
| 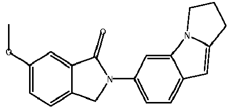 | 124774 | 0.327 | 1.369 |
| 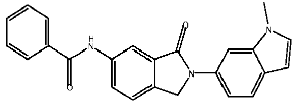 | 124775 | ND | 1.216 |
| 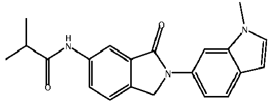 | 124776 | ND | 2.069 |
| 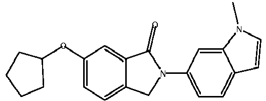 | 124860 | 0.135 | 1.582 |
| 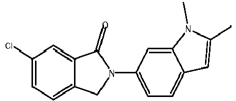 | 124861 | ND | 1.268 |
| 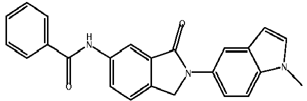 | 124862 | ND | 1.07 |
| 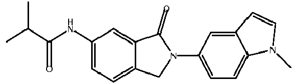 | 124863 | ND | 1.251 |
| 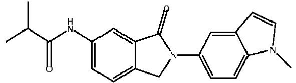 | 124864 | 0.277 | 1.519 |
| 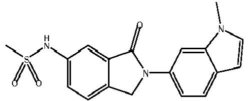 | 124977 | ND | 1.061 |
| 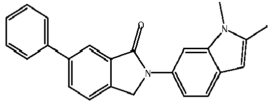 | 124978 | ND | 1.115 |
| 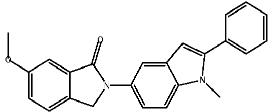 | 124979 | ND | 1.378 |
| 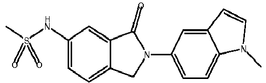 | 124980 | ND | 1.534 |
| 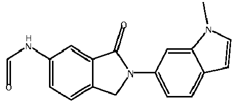 | 124981 | ND | 1.638 |
| 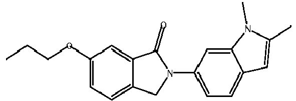 | 124982 | ND | 1.221 |
| 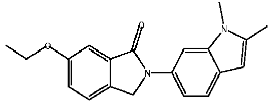 | 124983 | ND | 1.305 |
| 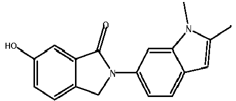 | 124984 | ND | 1.401 |
| 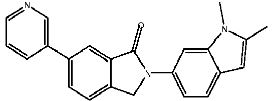 | 125093 | ND | 1.082 |
| 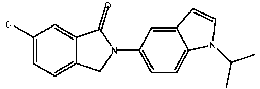 | 125094 | 0.009 | 1.256 |
| 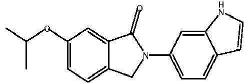 | 125095 | 0.003 | 1.134 |
| 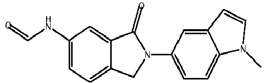 | 125096 | 0.044 | 1.371 |
| 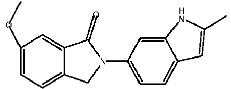 | 125097 | 0.05 | 1.347 |
| 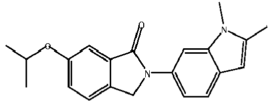 | 125098 | 0.134 | 1.251 |
| 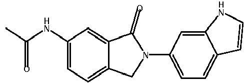 | 125269 | 0.015 | 1.416 |
| 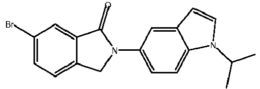 | 125270 | 0.016 | 1.446 |
| 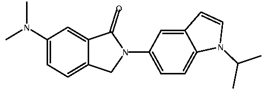 | 125271 | 0.022 | 1.285 |
| 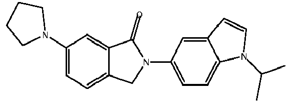 | 125384 | ND | 1.091 |
| 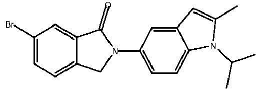 | 125385 | ND | 1.454 |
| 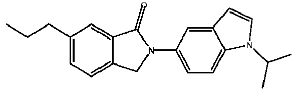 | 125386 | ND | 1.037 |
| 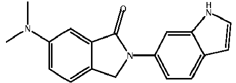 | 125600 | 0.004 | 1.405 |
| 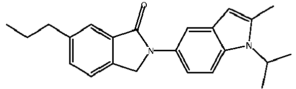 | 125601 | ND | 1.027 |
| 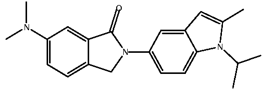 | 125602 | ND | 1.139 |
| 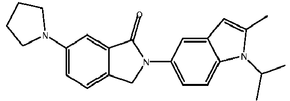 | 125603 | ND | 0.995 |
| 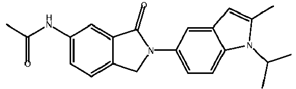 | 125822 | ND | 1.329 |
| 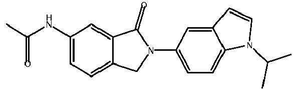 | 126111 | 0.063 | 1.34 |

Indoprofen increases luciferase activity in this assay. Although this assay is designed to detect correction of the SMN2 splicing defect, it is known that indoprofen does not increase luciferase expression in this assay via effects on splicing. Nonetheless, the effects of indoprofen detected in this assay have been shown to extend to an increase in production of SMN from the endogenous SMN2 gene in SMA patient fibroblasts.

Many of the tested compounds increased luciferase activity in the assay, indicating that the compounds of the invention can be used to increase expression of SMN from the endogenous SMN2 gene.

### EXAMPLE 53

This example demonstrates the ability of compounds of the invention to increase SMN-containing gem particles in fibroblasts from SMA patients in an embodiment of the invention.

SMN protein can be found in punctate nuclear particles called "gems" (Liu and Dreyfuss, EMBO J,. 15 (14): 3555-3565 (1996)). In fibroblast cells derived from SMA patients, the number of gems corresponds to disease severity as follows: severe type 1 patients have approximately 5 gems per 100 nuclei; mild type 3 patients have 20-50 gems per 100 nuclei; normal individuals have approximately 100-150 gems per 100 nuclei (Coovert et al., Hum Mol Genet, 6 (8): 1205-1214 (1997), Young et al., Exp Cell Res, 265 (2): 252-261 (2001)).

Gems are detectable by immunohistochemistry and gem counts in patient fibroblasts have been used to test the ability of compounds to increase SMN protein levels (Mattis et al., Hum Genet, 120: 589-601 (2006)). Following these methods, fibroblast cells from a type 1 SMA patient (3813 cells, Coriell Cell Repositories) were treated for 48 hours with a compound of the invention. The cells were then fixed and incubated with a monoclonal antibody against SMN (4B7, Wolstencroft et al., Hum Mol Genet, 14: 1199-1210 (2005)). A FITC-conjugated anti-mouse secondary antibody was used to visualize the labeled gems. A positive control in these experiments was provided by fibroblasts from the same patient treated with 1000 µM valproic acid. As shown in Table 2, treatment of the cells with compounds of the invention increased the number of SMN-containing gems in patient fibroblasts.

**Table 2.**

| **Compound Identifier** | **Concentration (µm)** | **Average number of gems per 100 nuclei** | **DMSO Control** | **VPA Positive Control** |
|---|---|---|---|---|
| LB-118417 | 3 | 53 | 5 | 39 |
| ALB-118561 | 10 | 139 | 6 | 14 |
| ALB-118739 | 1 | 76 | 5 | 39 |
| ALB-119047 | 0.1 | 32 | 7 | 40 |
| ALB-119522 | 1 | 6 | 5 | 40 |
| ALB-119537 | 0.1 | 31 | 5 | 39 |
| ALB-119869 | 1 | 49 | 7 | 31 |
| ALB-120121 | 0.1 | 16 | 5 | 39 |
| ALB-120286 | 0.1 | 132 | 6 | 14 |
| ALB-120286a | 0.1 | 53 | 9 | 41 |
| ALB-120595 | 1 | 32 | 5 | 40 |
| ALB-120596 | 0.1 | 148 | 6 | 14 |
| ALB-120602 | 0.1 | 68 | 7 | 40 |
| ALB-120790 | 1 | 27 | 5 | 40 |
| ALB-120790a | 1 | 85 | 9 | 41 |
| ALB-121505 | 1 | 47 | 7 | 31 |
| ALB-121789 | 1 | 10 | 5 | 40 |
| ALB-122182 | 1 | 18 | 5 | 40 |
| ALB-122317 | 1 | 45 | 5 | 40 |
| ALB-123011 | 1 | 37 | 5 | 40 |
| ALB-123707 | 0.1 | 53 | 7 | 31 |
| ALB-123714 | 1 | 84 | 9 | 41 |
| ALB-124032 | 1 | 65 | 9 | 35 |
| ALB-124326 | 1 | 98 | 9 | 35 |
| ALB-124429 | 1 | 44 | 7 | 31 |
| ALB-124596 | 1 | 43 | 7 | 31 |
| ALB-124597 | 1 | 42 | 9 | 35 |
| ALB-124771 | 1 | 50 | 7 | 31 |

### EXAMPLE 54

This example demonstrates the ability of compounds of the invention to increase total cellular SMN protein in fibroblasts from SMA patients.

Western blots were performed using standard methods. SMA type I fibroblasts (3813 cells; Coriell Cell Repositories) were treated with compounds for 48 hours. Monoclonal SMN anti-SMN antibody 4B7 (Wolstencroft et al., Hum Mil Genet, 14:1199-1210 (2005)) was used to identify SMN protein. Tubulin was used as an internal normalization standard. DMSO treatment serves as the negative control. The results are presented in Table 3.

**Table 3.**

| **Compound Identifier** | **Concentration (µM)** | **SMN:Tubulin ratio as % of Vehicle Control** |
|---|---|---|
| ALB-118561 | 10 | 151.976 |
| ALB-119047 | 0.1 | 125.19 |
| ALB-120596 | 1 | 124.41 |
| ALB-120602 | 1 | 123.67 |
| ALB-124326 | 3 | 147.687 |

### EXAMPLE 55

The following example demonstrates that compounds of the invention can increase activation of the glutamate transporter promoter in an embodiment of the invention.

The astroglial glutamate transporter, GLT-1/EAAT-2, is responsible for the majority of glutamate transport in the brain and spinal cord. To identify compounds capable of inducing EAAT-2 expression, a cell-based reporter assay was used that consisted of COS-7 cells that were stably transfected with a plasmid containing at 2.7 Kb fragment of the human GLT-1/EAAT-2 promoter driving expression of the luciferase reporter (Rothstein et al., Nature, 433: 73-77 (2005)). Cells were incubated with compound at various concentrations for 48 h prior to measuring luciferase reporter activity using the Bright-Glo Luciferase Assay System (Promega). For example, ALB-118561 was found to be more active than indoprofen in increasing glutamate transporter promoter activity.

### EXAMPLE 56

This example demonstrates the use of compounds of the invention to promote translational read-through of a stop codon in an embodiment of the invention.

This assay was similar in design to that described in WO 01/44516 A2. This was a cell-based luciferase reporter assay designed to detect translational read-through via insertion of a translational stop codon within the coding sequence that includes luciferase. In the presence of compounds that promote read-through of translational stop codons, active luciferase was produced and detected via chemiluminescence. Compounds of the invention typically were active in three versions of this assay: luciferase reporter containing a UGA stop codon, luciferase reporter containing a UAG stop codon, which is the first stop codon downstream of the improper splice junction between exons 6 and 8 in the human SMN2 transcript, and luciferase preceded by the open reading frame of the human SMN2 gene. The results of tests in the latter assay are reported in Table 4.

**Table 4.**

| **Compound Identifier** | **Max Fold** | **EC₅₀ (µM)** |
|---|---|---|
| ALB-116069 | 1.973 | 2.443 |
| ALB-116239 | 2.021 | 0.421 |
| ALB-116566 | 1.358 | 4.178 |
| ALB-118275 | 1.282 | 2.089 |
| ALB-118561 | 2.228 | 0.296 |
| ALB-118740 | 1.672 | 0.22 |
| ALB-119047 | 2.035 | 4.276 |
| ALB-119518 | 1.201 | 2.805 |
| ALB-119521 | 1.559 | 2.958 |
| ALB-119522 | 1.728 | 1.837 |
| ALB-119537 | 2.121 | 1.352 |
| ALB-119658 | 1.192 | 6.026 |
| ALB-119659 | 1.378 | 0.752 |
| ALB-119867 | 2.037 | 0.676 |
| ALB-120594 | 2.083 | 19.364 |
| ALB-120596 | 2.229 | 1.722 |
| ALB-120599 | 1.884 | 1.892 |
| ALB-120600 | 1.833 | 4.887 |
| ALB-120601 | 1.646 | 15.596 |
| ALB-120602 | 2.122 | 1.107 |
| ALB-120604 | 1.649 | 7.907 |
| ALB-120791 | 1.913 | 0.641 |
| ALB-120955 | 1.664 | 14.06 |
| ALB-121259 | 1.032 | 0.032 |
| ALB-121789 | 1.793 | 0.35 |
| ALB-121924 | 1.662 | 2.649 |
| ALB-122435 | 1.98 | 1.291 |
| ALB-122685 | 1.868 | 0.667 |
| ALB-122818 | 1.806 | 12.764 |
| ALB-123011 | 1.891 | 2.642 |
| ALB-123330 | 1.4 | NC |
| ALB-123707 | 1.438 | 0.091 |
| ALB-123714 | 1.181 | 1.905 |
| ALB-123934 | 1.301 | 97.724 |
| ALB-124032 | 1.022 | NC |
| ALB-124326 | 0.946 | 0.129 |
| ALB-124771 | 1.799 | 4.207 |
| ALB-125094 | 1.158 | NC |
| ALB-125270 | 1.194 | NC |

### EXAMPLE 57

This example demonstrates the plasma and brain pharmacokinetics of compounds of the invention.

FVB or CD-1 mice, approximately 5 weeks old, are given a single dose of compound at a prescribed concentration (e.g., 5 mg/kg). Compounds typically are administered either orally or intravenously. Three to five mice are euthanized at various times between 15 minutes and 24 hours after dosing and brain and plasma are harvested. Tissues are homogenized in acetonitrile and analyzed on an API 4000 LC/MS/MS system coupled with an Agilent 1100 series liquid chromatograph. All pharmacokinetic analyses are conducted using WinNonlin (Version 4.01; Pharsight, Palo Alto, CA) with the exception of brain/plasma ratios, which are calculated using Microsoft Excel 2000. AUC values are the area under the concentration-time curve from time zero to the last measured time point. Half life values are extrapolated from 3-4 time points taken at less than 4 hours after dosing. Some preferred compounds of the invention exhibit plasma and brain pharmacokinetic properties that suggest utility in treating CNS conditions.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Present invention furthermore is characterized by the following aspects:
1. A compound or pharmaceutically acceptable salt of Formula I: 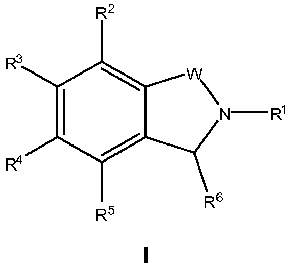 wherein
   W is selected from the group consisting of C(O), C(S), and CH₂;
   R¹ heterocycloalkylaryl, heteroaryl, or heterocycloalkyl, each of which is optionally substituted with 1 to 3 substituents individually selected from the group consisting of C₁-C₈ alkyl, C₃-C₆ cycloalkyl, C₁-C₈ alkoxy, C₁-C₈ haloalkoxy, C₁-C₈ haloalkyl, halogen, -CN, -C(O)OR⁷, -C(O)NR⁷(R⁸), -NO₂, -SO₂NR⁷(R⁸), -NR⁹(SO₂)R¹⁰, -NR⁷C(O)NR⁸R⁹, amino, C₁-C₄ alkylamino, C₃-C₆ cycloalkylamino, aryl, heteroaryl, and heterocycloalkyl;
   R², R³, R⁴, and R⁵ are independently selected from the group consisting of H, hydroxyl, C₁-C₈ alkyl, C₂-C₈ alkenyl, C₃-C₆ cycloalkyl, C₁-C₈ alkoxy, C₃-C₆ cycloalkyloxy, C₁-C₈ haloalkoxy, C₁-C₈ haloalkyl, halogen, alkylsulfonyl, -CN, -NO₂, -SO₂NR⁷(R⁸), -NR⁹(SO₂)R¹⁰, -NR⁷C(O)R⁸, -NR⁷C(O)NR⁸R⁹, amino, C₁-C₄ alkylamino, C₃-C₆ cycloalkylamino, aryl, heteroaryl, and heterocycloalkyl; or
   R² and R³, R³ and R⁴ or R⁴ and R⁵ can be taken together with the carbon atoms to which they are attached to form a 5- or 6-membered cycloalkyl or heterocycloalkyl; comprising 1 or 2 heteroatoms selected from the group consisting ofN, O, and S;
   R⁶ is selected from the group consisting of H and C₁-C₈ alkyl;
   R⁷, R⁸, and R⁹ are independently selected from the group consisting of H, C₁-C₈ alkyl, C₃-C₆ cycloalkyl, aryl, and heteroaryl; and
   R¹⁰ is selected from the group consisting of C₁-C₈ alkyl, C₃-C₆ cycloalkyl, aryl, and heteroaryl;
   wherein C₁-C₈ alkyl is optionally substituted with one or more subsitutents individually selected from the group consisting of -CN, C₁-C₈ alkoxy, C₁-C₈ haloalkoxy, C₁-C₈ haloalkyl, -C(O)OR⁶, -C(O)NR⁶(R⁷), amino, C₁-C₄ alkylamino, C₃-C₆ cycloalkylamino, and heterocycloalkyl, and
   wherein aryl is optionally substituted with one or more substituents individually selected from the group consisting of halogen, -NO₂, -CN, C₁-C₈ alkyl, C₁-C₈ haloalkyl, and C₁-C₈ alkoxy.
2. The compound or pharmaceutically acceptable salt of aspect 1, wherein R¹ is selected from the group consisting of
   a heteroaryl comprising one or two oxygen or sulfur atoms and/or from one to four nitrogen atoms provided that the total number of heteroatoms in each ring is four or less and each ring has at least one carbon atom,
   a heterocycloalkyl comprising a 5- or 6-membered monocyclic ring that contains one, two, or three heteroatoms selected from nitrogen, oxygen, and/or sulfur, and
   a heterocycloalkyl-fused phenyl, wherein the heterocycloalkyl consists of a 5- or 6-membered monocyclic ring that contains one, two, or three heteroatoms selected from nitrogen, oxygen, and/or sulfur,
   each of which is optionally substituted with 1 to 3 substituents individually selected from the group consisting of C₁-C₈ alkyl, C₃-C₆ cycloalkyl, C₁-C₈ alkoxy, C₁-C₈ haloalkoxy, C₁-C₈ haloalkyl, halogen, -CN, -C(O)OR⁷, -C(O)NR⁷(R⁸), -NO₂, -SO₂NR⁷(R⁸),-NR⁹(SO₂)R¹⁰, -NR⁷C(O)NR⁸R⁹, amino, C₁-C₄ alkylamino, C₃-C₆ cycloalkylamino, aryl, heteroaryl, and heterocycloalkyl.
3. The compound or pharmaceutically acceptable salt of aspect1, wherein R¹ is selected from the group consisting of pyrenyl, 1H-indolyl, benzo[b]thiophenyl, benzofuranyl, 1H-benzo[d]imidazolyl, 2H-indazolyl, benzo[d]thiazolyl, benzo[d]oxazolyl, isoquinolinyl, quinolinyl, quinoxalinyl, 3,4-dihydro-2H-benzo[b][1,4]oxazinyl, 2H-benzo[b][1,4]oxazin-3(4H)-onyl, 1H-benzo[d]-imidazol-2(3H)-onyl, quinoline-2(1H)-onyl, 2,3-dihydro-1H-indenyl, pyridinyl, pyridazinyl, pyrimidyl, pyrazinyl, triazinyl, pyrrolyl, pyrazolyl, imidazolyl, (1,2,3,)- and (1,2,4)-triazolyl, pyrazinyl, pyrimidinyl, tetrazolyl, furyl, thiophenyl, isothiazolyl, thiazolyl, isoxazolyl, oxadiazolyl, oxazolyl, pyrrolo[2,3-c]pyridinyl, pyrrolo[3,2-c]pyridinyl, pyrrolo[2,3-b]pyridinyl, pyrrolo[3,2-b]pyridinyl, pyrrolo[3,2-d]pyrimidinyl, pyrrolo[2,3-d]pyrimidinyl, quinazolinyl, phthalazinyl, imidazo[1,2-a]pyridinyl, pyrrolinyl, pyranyl, piperidyl, tetrahydrofuranyl, tetrahydrothiopheneyl, morpholinyl, 1,2,3,4-tetrahydronaphthalenyl, 2,3-dihydro-1H-indenyl, 1,2,3,4-tetrahydroquinolinyl, 1,2,3,4-tetrahydroquinoxalinyl, and 2,3-dihydrobenzo[b][1,4]dioxinyl.
4. The compound or pharmaceutically acceptable salt of aspect1, wherein R¹ is selected from the group consisting of 1H-indolyl, benzo[b]thiophenyl, benzofuranyl, 1H-benzo[d]imidazolyl, 2H-indazolyl, benzo[d]thiazolyl, benzo[d]oxazolyl, isoquinolinyl, quinolinyl, quinoxalinyl, 3,4-dihydro-2H-benzo[b][1,4]oxazinyl, 2H-benzo[b][1,4]oxazin-3(4H)-only, 1H-benzo[d]-imidazol-2(3H)-onyl, quinoline-2(1H)-onyl, pyridinyl, furyl, thiophenyl, pyrrolo[2,3-c]pyridinyl, pyrrolo[3,2-c]pyridinyl, pyrrolo[2,3-b]pyridinyl, pyrrolo[3,2-b]pyridinyl, pyrrolo[3,2-d]pyrimidinyl, pyrrolo[2,3-d]pyrimidinyl, quinazolinyl, phthalazinyl, imidazo[1,2-a]pyridinyl, 1,2,3,4-tetrahydronaphthalenyl, 2,3-dihydro-1H-indenyl, 1,2,3,4-tetrahydroquinolinyl, 1,2,3,4-tetrahydroquinoxalinyl, and 2,3-dihydrobenzo[b][1,4]dioxinyl.
5. The compound or pharmaceutically acceptable salt of aspect1, wherein R¹ is selected from the group consisting of 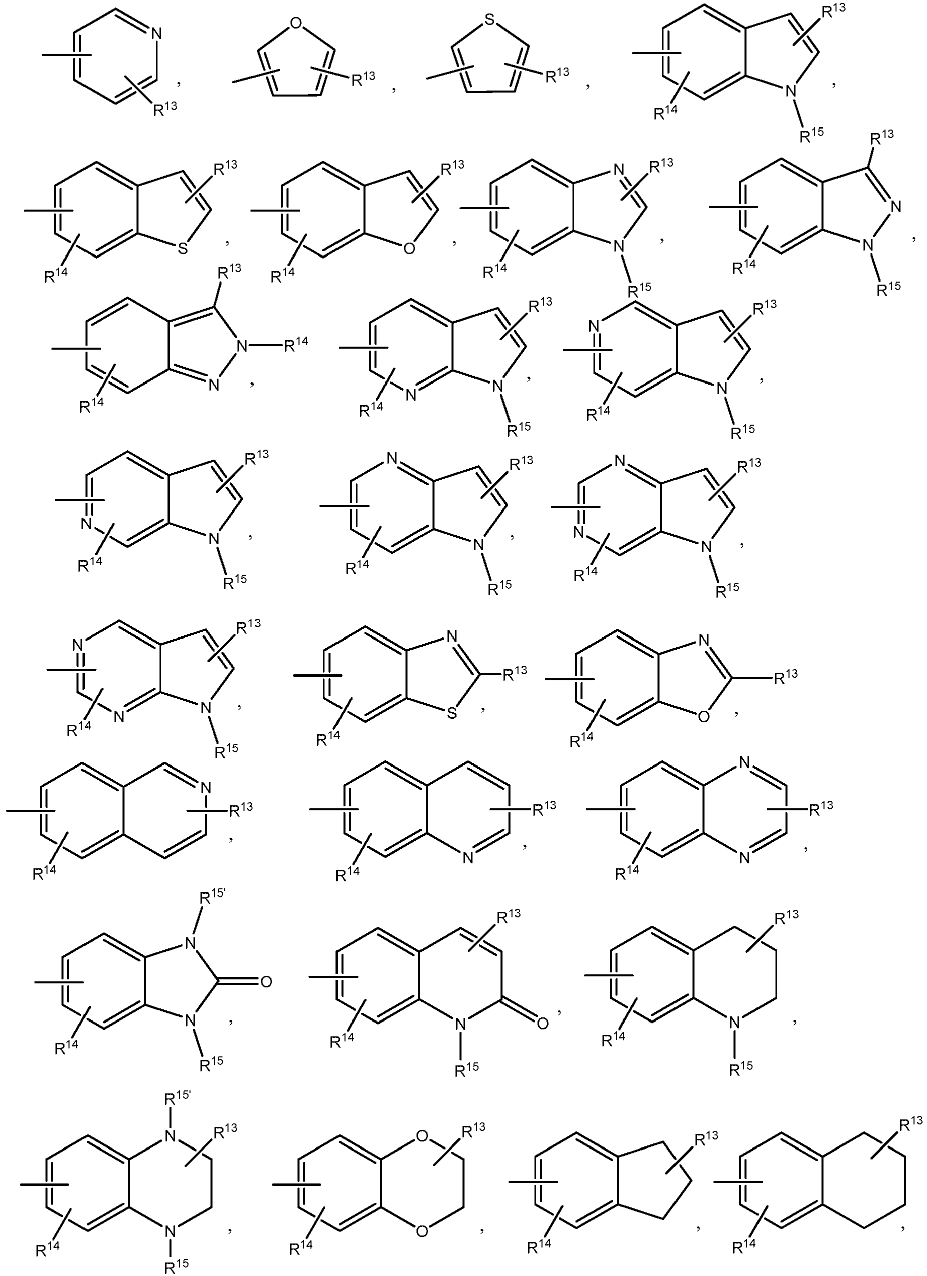 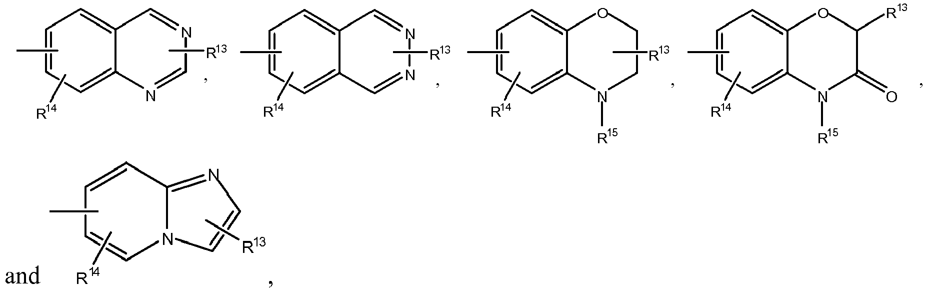 wherein
   R¹³ and R¹⁴ are the same or different and each is selected from the group consisting of H, C₁-C₈ alkyl, C₃-C₆ cycloalkyl, C₁-C₈ alkoxy, C₁-C₈ haloalkoxy, C₁-C₈ haloalkyl, halogen, -CN, -C(O)OR⁷, -C(O)NR⁷(R⁸), -NO₂, -SO₂NR⁷(R⁸), -NR⁹(SO₂)R¹⁰, -NR⁷C(O)NR⁸R⁹, amino, C₁-C₄ alkylamino, C₃-C₆ cycloalkylamino, aryl, heteroaryl, and heterocycloalkyl; and R¹⁵ and R¹⁵ are the same or different and each is hydrogen or C₁-C₈ alkyl,
   wherein C₁-C₈ alkyl is optionally substituted with one or more subsitutents individually selected from the group consisting of of -CN, C₁-C₈ alkoxy, C₁-C₈ haloalkoxy, C₁-C₈ haloalkyl, -C(O)OR⁶, -C(O)NR⁶(R⁷), amino, C₁-C₄ alkylamino, C₃-C₆ cycloalkylamino, and heterocycloalkyl.
6. The compound or pharmaceutically acceptable salt of aspect5, wherein R¹ is selected from the group consisting of 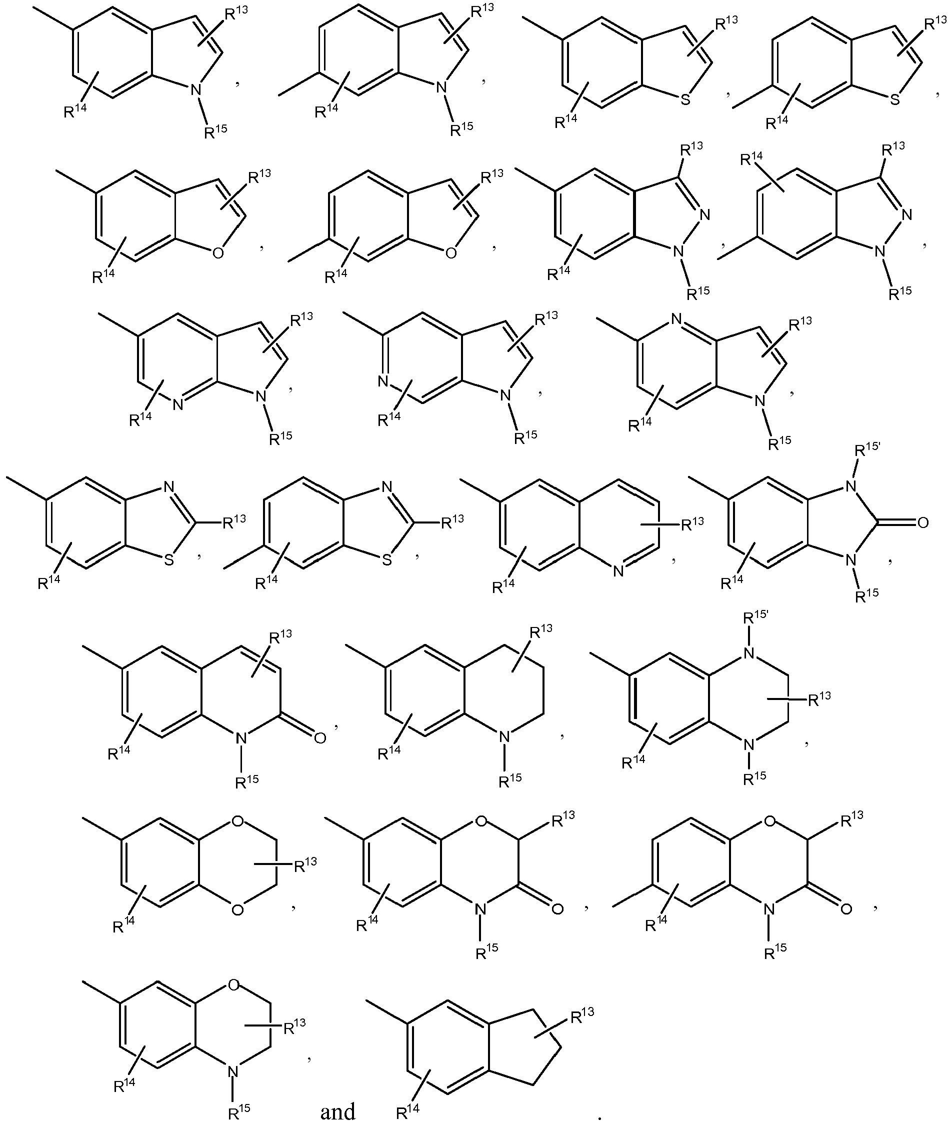
7. The compound or pharmaceutically acceptable salt of aspect5 or 6, wherein R¹³ is selected from the group consisting of H, halogen, -CN, C₁-C₈ alkyl, C₁-C₈ alkoxy, C₁-C₈ haloalkoxy, C₁-C₈ haloalkyl, amino, C₁-C₄ alkylamino, and phenyl, and wherein C₁-C₈ alkyl is optionally substituted with one or more subsitutents individually selected from the group consisting of C₁-C₈ alkoxy, C₁-C₈ haloalkoxy, C₁-C₈ haloalkyl, -C(O)OR⁶, -C(O)NR⁶(R⁷), -CN, amino, C₁-C₄ alkylamino, C₃-C₆ cycloalkylamino, and heterocycloalkyl.
8. The compound or pharmaceutically acceptable salt of any of aspect 5-7,
   wherein R¹³ is selected from the group consisting of H and C₁-C₈ alkyl optionally substituted with one or more subsitutents individually selected from the group consisting of C₁-C₈ alkoxy, C₁-C₈ haloalkoxy, C₁-C₈ haloalkyl, -C(O)OR⁶, -C(O)NR⁶(R⁷), -CN, amino, C₁-C₄ alkylamino, C₃-C₆ cycloalkylamino, and heterocycloalkyl.
9. The compound or pharmaceutically acceptable salt of any of aspect 5-8,
   wherein R¹³ is C₁-C₈ alkyl that is optionally substituted with one or more subsitutents individually selected from the group consisting of C₁-C₈ alkoxy, C₁-C₈ haloalkoxy, C₁-C₈ haloalkyl, -C(O)OR⁶, -C(O)NR⁶(R⁷), -CN, amino, C₁-C₄ alkylamino, C₃-C₆ cycloalkylamino, and heterocycloalkyl.
10. The compound or pharmaceutically acceptable salt of any of aspect 5-9,
   wherein R¹³ is methyl, ethyl, propyl, isopropyl, -(CH₂)ₙCN, or -(CH₂)ₙNH₂, and n is 1 to 8.
11. The compound or pharmaceutically acceptable salt of any of aspect 5-10,
   wherein R¹⁵ is hydrogen, methyl, or i-propyl.
12. The compound or pharmaceutically acceptable salt of any of aspect1-11,
   wherein W is C(O).
13. The compound or pharmaceutically acceptable salt of any of aspect 1-11,
   wherein W is CH₂.
14. The compound or pharmaceutically acceptable salt of any of aspect 1-13,
   wherein R² is selected from the group consisting of H, hydroxyl, halogen, C₁-C₈ alkyl, C₁-C₈ alkoxy, C₁-C₈ haloalkoxy, nitro, amino, C₁-C₄ alkylamino, aryl, and heterocycloalkyl.
15. The compound or pharmaceutically acceptable salt of any of aspect 1-13,
   wherein R² is selected from the group consisting of H, hydroxyl, nitro, amino, C₁-C₈ alkyl, C₁-C₈ alkoxy, C₁-C₈ haloalkoxy, -NR⁷C(O)R⁸, and phenyl.
16. The compound or pharmaceutically acceptable salt of any of aspect 1-15,
   wherein R³ is selected from the group consisting of H, hydroxyl, halogen, C₁-C₈ alkyl, C₂-C₈ alkenyl, C₁-C₈ alkoxy, C₃-C₆ cycloalkyloxy, C₁-C₈ haloalkoxy, alkylsulfonyl, nitro, amino, C₁-C₄ alkylamino, -NR⁷C(O)R⁸, heterocycloalkyl, and heteroaryl.
17. The compound or pharmaceutically acceptable salt of aspect1-15, wherein the haloalkoxy is -OCHF₂, -OCH₂CF₃, -OCBrF₂, or -OCF₂CHFCl.
18. The compound or pharmaceutically acceptable salt of any of aspect 1-17,
   wherein R⁴ is selected from the group consisting of H, hydroxyl, halogen, C₁-C₈ alkyl, C₁-C₈ alkoxy, C₁-C₈ haloalkoxy, nitro, amino, C₁-C₄ alkylamino, and heterocycloalkyl.
19. The compound or pharmaceutically acceptable salt of any of aspect 1-17,
   wherein R⁴ is H, nitro, C₁-C₈ alkoxy, or C₁-C₈ haloalkoxy.
20. The compound or pharmaceutically acceptable salt of any of aspect 1-19,
   wherein R⁵ is selected from the group consisting of H, hydroxyl, halogen, C₁-C₈ alkyl, C₁-C₈ alkoxy, C₁-C₈ haloalkoxy, amino, C₁-C₄ alkylamino, and -NR⁷C(O)R⁸.
21. The compound or pharmaceutically acceptable salt of any of aspect 1-19,
   wherein R⁵ is selected from the group consisting of H, C₁-C₈ alkyl, -NHC(O)R⁸, C₁-C₆ alkoxy, C₁-C₆ haloalkoxy, amino, and C₁-C₄ alkylamino.
22. A compound of Table 1, or pharmaceutically acceptable salt thereof.
23. A composition comprising at least one compound or pharmaceutically acceptable salt of any of aspect 1-22 and a pharmaceutically acceptable carrier.
24. A method of increasing survival motor neuron protein (SMN) expression in a cell comprising administering a compound or pharmaceutically acceptable salt of any of aspect 1-22 to a cell comprising a nucleic acid encoding SMN2, wherein the cell optionally is in a host, whereby SMN expression is increased.
25. The method of aspect24, wherein the cell is in a host, and the host is a mammal.
26. The method of aspect25, wherein the mammal is a human afflicted with a disease associated with under-expression of SMN.
27. The method of aspect26, wherein the disease is spinal muscular atrophy (SMA).
28. A method of treating spinal muscular atrophy (SMA) in a mammal comprising administering a therapeutically effective amount of at least one compound or pharmaceutically acceptable salt of any of aspect 1-22 to the mammal, whereupon SMA is treated.
29. A method of increasing in a cell the expression of a nucleic acid that encodes a translational stop codon, the method comprising administering a compound or pharmaceutically acceptable salt of any of aspect1-22 to a cell comprising a nucleic acid that encodes a translational stop codon, wherein the cell optionally is in a host, whereby expression of the nucleic acid is increased.
30. The method of aspect29, wherein the stop codon has been introduced directly or indirectly by mutation or frameshift.
31. The method of aspect29, wherein the host is a mammal.
32. The method of aspect31, wherein the mammal is a human afflicted with a disease associated with the translational stop codon introduced directly or indirectly by mutation or frameshift.
33. The method of aspect32, wherein the disease is cancer, diabetes, cystic fibrosis, Rett syndrome, ataxia-telangiecstasia, or muscular dystrophy.
34. A method of increasing the expression of excitatory amino acid transporter (EAAT2) in a cell comprising administering to a cell comprising a nucleic acid that encodes EAAT2 a compound or pharmaceutically acceptable salt of any of aspect 1-22, wherein the cell optionally is in a host, whereby the expression of EAAT2 is increased.
35. The method of aspect34, wherein the cell is in a host, and the host is a mammal.
36. The method of aspect35, wherein the mammal is a human that has an elevated level of glutamate in the central nervous system.
37. The method of aspect36, wherein the mammal is a human that has suffered stroke or trauma to an area of the central nervous system.
38. The method of aspect36, wherein the mammal is a human that is afflicted with a disease, optionally, a neurodegenerative disease.
39. The method of aspect38, wherein the disease is SMA, stroke, Parkinson's disease, Alzheimer's disease, amyotrophic lateral sclerosis (ALS), multiple sclerosis (MS), Huntington's disease, or epilepsy.
40. A method of treating or preventing a neurological disorder selected from the group consisting of SMA, stroke, Parkinson's disease, Alzheimer's disease, ALS, MS, Huntington's disease, and epilepsy in a mammal comprising administering a therapeutically effective amount of at least one compound or pharmaceutically acceptable salt of any of aspect 1-22 to the mammal, whereupon the neurological disorder is treated or prevented.
41. A method of treating or preventing an infectious disease in a mammal comprising administering a therapeutically effective amount of at least one compound or pharmaceutically acceptable salt of any of aspect 1-22 to the mammal, whereupon the infectious disease is treated or prevented.
42. The method of aspect41, wherein the infectious disease is viral.
43. The method of aspect41, wherein the infectious disease is Human Immunodeficiency Virus infection (HIV/AIDS), viral hepatitis, Human Herpes virus (HHV) infection, or Human Papilloma Virus (HPV) infection.
44. The method of aspect41, wherein the infectious disease is severe acute respiratory syndrome (SARS), herpes zoster, or Pseduomonas aeruginosa infection.

## Claims

1. A compound of 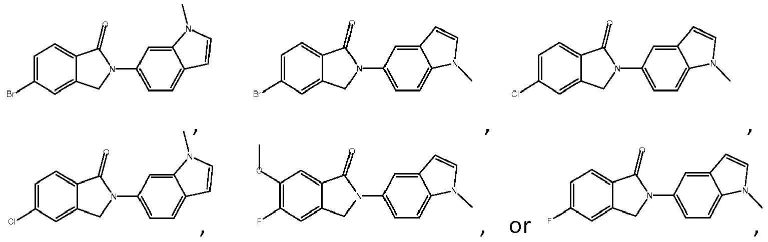 or pharmaceutically acceptable salt thereof.

2. A composition comprising at least one compound or pharmaceutically acceptable salt of claim 1 and a pharmaceutically acceptable carrier.

3. A compound or pharmaceutically acceptable salt of claim 1 for use in increasing survival motor neuron protein (SMN) expression in a cell comprising a nucleic acid encoding SMN2, wherein the cell optionally is in a host.

4. A compound or pharmaceutically acceptable salt of claim 1 for use in treating spinal muscular atrophy (SMA).

5. A compound or pharmaceutically acceptable salt of claim 1 for use in increasing the expression of a nucleic acid that encodes a translational stop codon in a cell, wherein the cell optionally is in a host.

6. The compound of claim 5, wherein the host is a human afflicted with a disease selected from the group consisting of cancer, diabetes, cystic fibrosis, Rett syndrome, ataxia-telangiecstasia, and muscular dystrophy.

7. A compound or pharmaceutically acceptable salt of claim 1 for use in increasing the expression of excitatory amino acid transporter (EAAT2) in a cell, wherein the cell optionally is in a host.

8. The compound of claim 7, wherein the host is a human that is afflicted with a disease selected from the group consisting of SMA, stroke, Parkinson's disease, Alzheimer's disease, amyotrophic lateral sclerosis (ALS), multiple sclerosis (MS), Huntington's disease, and epilepsy.

9. A compound or pharmaceutically acceptable salt of claim 1 for use in treating or preventing a neurological disorder selected from the group consisting of SMA, stroke, Parkinson's disease, Alzheimer's disease, ALS, MS, Huntington's disease, and epilepsy.

10. A compound or pharmaceutically acceptable salt of claim 1 for use in treating or preventing an infectious disease.

11. The compound of claim 10, wherein the infectious disease is viral.

12. The compound of claim 10, wherein the infectious disease is Human Immunodeficiency Virus infection (HIV/AIDS), viral hepatitis, Human Herpes virus (HHV) infection, or Human Papilloma Virus (HPV) infection, severe acute respiratory syndrome (SARS), herpes zoster, or Pseduomonas aeruginosa infection.
